# EUROPEAN PATENT SPECIFICATION

(11) **EP 2 405 270 B1**
(45) Date of publication and mention of the grant of the patent: **17.07.2013**
(21) Application number: 11162598.4
(22) Date of filing: 29.06.2007
(51) Int. Cl.: G01N 33/574, C07K 16/28, G01N 33/68

(54) **IGFBP2-Biomarker**
IGFBP2-Biomarker
Biomarqueur IGFBP2

(30) Priority: 30.06.2006 US 818004 P
(43) Date of publication of application: 11.01.2012
(62) Divisional of application: 07810179.7
(73) Proprietor: Merck Sharp & Dohme Corp., Rahway, NJ 07065-0907 (US)
(72) Inventor: Wang, Yan, Warren, NJ 07059 (US)
(74) Representative: Horgan, James Michael Frederic

(56) References cited:
- WO-A-2004/111603
- WO-A-2006/060419

## Description

### Field of the Invention

The present invention relates to methods for determining if an IGF1R inhibitor is efficacious in a patient receiving the inhibitor, for example, to treat cancer.

### Background of the Invention

The insulin-like growth factors, also known as somatomedins, include insulin-like growth factor-I (IGF-I) and insulin-like growth factor-II (IGF-II) (Klapper, *et al.,* (1983) Endocrinol. 112:2215 and Rinderknecht, *et al.,* (1978) Febs.Lett. 89:283). These growth factors exert mitogenic activity on various cell types, including tumor cells (Macaulay, (1992) Br. J. Cancer 65:311), by binding to a common receptor named the insulin-like growth factor receptor-1 (IGF1R) (Sepp-Lorenzino, (1998) Breast Cancer Research and Treatment 47:235). Interaction of IGFs with IGF1R activates the receptor by triggering autophosphorylation of the receptor on tyrosine residues (Butler, *et al.,* (1998) Comparative Biochemistry and Physiology 121:19). Once activated, IGF1R, can bind to intracellular substrates such as IRS-1 and Sch. Phosphorylated IRS-1 can activate the p85 regulatory subunit of PI3 kinase, leading to activation of several downstream substrates, including the p70 S6 kinase and protein kinase B (Akt). Akt phosphorylation in turn, enhances protein synthesis through mTOR activation and triggers anti-apoptotic effects of IGF1R through phosphorylation and inactivation of Bad. In parallel to PI3 kinase-driven signaling, recruitment of Grb2/SOS by phosphorylated IRS-1 or Shc leads to recruitment of Ras and activation of the Raf1/MEK/ERK pathway and downstream nuclear factors, resulting in the induction of cellular proliferation. Clearly, inhibition of the activity in this pathway would be a valuable means by which to treat diseases mediated by any member of this pathway (*e.g*., IGF1R). Inhibition of IGF1R activity has proven to be a valuable method to treat or prevent growth of human cancers and other proliferative diseases. For example, overexpression of insulin-like growth factor receptor-I has been demonstrated in several cancer cell lines and tumor tissues. Likewise, monitoring the activity of this pathway is a valuable marker for the effect of an IGF1R inhibitor on the pathway's downstream effects, *e.g*., malignant cell growth.

Another modulator of cellular growth is IGFBP2. IGFBP2 has been identified as a possible growth promoter of malignant cells. IGFBP2 expression depends, at least in part, on IGF1-mediated IGF1R activation (Martin et al., Endocrinology (2007) 148(5): 2532-2541). Activation of the PI3 kinase part of the IGF1R signaling pathway has been linked to IGFBP2 expression (Martin *et al.* (2007)).

Currently, there are several known anti-cancer therapies that target IGF1R; for example, anti-IGF1R antibodies (See *e.g*., WO2003/100008) Assessing the proper dosage to provide to a subject receiving IGF1R inhibitor therapy can be difficult using current technology. For example, a clinician may need to resort to measuring tumor size or cancer progression after several weeks or months of therapy so as to determine if the dosage is proper. Such a process can be time consuming and, thus, dangerous in view of the fact that certain cancers must be treated rapidly and effectively in order to reach a positive therapeutic outcome (*e.g*., survival). There is, thus, a need in the art for rapidly and conveniently determining whether a given dosage is proper.

WO 2006/060419 discloses IRS-1 phosphorylation as biomarker for selection of patients for anti-IGF1R therapy. WO 2004/111603 discloses biomarkers including IGFBP-1 for predicting the response to chemotherapy using doxorubicin and docetaxel.

### Summary of the Invention

The present invention addresses this need by providing the methods of the present invention. As is discussed herein, the present invention provides a simple and convenient method for monitoring the magnitude or inhibition of the IGF1R system or cascade in the body of a subject receiving IGF1R inhibitor therapy by monitoring IGFBP2 levels in the subject's body over the course of inhibitor treatment. It has been demonstrated that IGF1R treatment causes IGFBP2 levels to depress over time in the body of a subject receiving the inhibitor. The link between IGFBP2 levels and the level of activity in the IGF1R signaling cascade make blood levels of IGFBP2 a convenient indicator for the magnitude of effect an inhibitor therapy is having on the cascade. It has also been demonstrated that IGFBP2 levels decrease by a maximum amount and that this amount represents a convenient pharmacokinetic target. A clinician or other practitioner administering an IGF1R inhibitor to a subject with a medical condition mediated by IGF1R can, in turn, follow blood IGFBP2 levels over time in the subject's body and, based on this observation, decide if treatment should be altered in some way, *e.g*., if dosage should be increased, decreased, maintained or discontinued.

For example, described herein is a method for monitoring the effect of an IGF1R inhibitor on IGFBP2 concentration in the body of a subject administered said inhibitor comprising measuring IGFBP2 levels in the body of the subject over time during a course of treatment of said inhibitor. Such clinical/pharmacokinetic data is valuable in the evaluation of both the efficacy and the dosage (*e.g*., amount and/or frequency) of an given IGF1R inhibitor therapeutic regimen. In a more specific example the method comprises (i) measuring an IGFBP2 concentration in the body of said subject before treatment with said inhibitor (*e.g*., in a treatment-naïve subject never exposed to the inhibitor or in a subject who is in the midst of an ongoing therapeutic regimen); (ii) administering one or more doses of said inhibitor to said subject; (iii) measuring an IGFBP2 concentration in the body of said subject following said administration; (iv) comparing the level of IGFBP2 measured in step (i) with the level of IGFBP2 measured in step (iii). For example, inhibitor is determined to lower the IGFBP2 concentration if the level measured in step (i) is higher than the concentration measured in step (iii); and wherein the inhibitor is determined not to lower the IGFBP2 concentration if the level measured in step (i) is not higher than the concentration measured in step (iii).

The present invention provides a method for monitoring the effect of an IGF1R inhibitor on the IGF1 receptor in the body of a subject administered said inhibitor comprising evaluating IGFBP2 levels in the body of the subject over time as defined by the claims;
*e.g*., wherein the inhibitor is determined to inhibit the receptor if IGFBP2 levels are observed to decrease over time following said administration; or wherein the inhibitor is determined not to inhibit the receptor if IGFBP2 levels are not observed to decrease over time following said administration. In an embodiment of the invention, the inhibitor is determined to inhibit the receptor if IGFBP2 levels are observed to decrease by at least 51% over time following a first administration of said inhibitor; or wherein the inhibitor is determined not to inhibit the receptor if IGFBP2 levels are not observed to decrease by at least 51 % over time following a first administration of said inhibitor. In an embodiment of the invention, the method comprises (i) measuring an IGFBP2 level in the body of said subject before treatment with said inhibitor; (ii)
measuring an IGFBP2 level in the body of said subject following administration of one or more doses of said inhibitors; (iii) comparing the level of IGFBP2
measured in step (i) with the level of IGFBP2 measured in step (ii); wherein the inhibitor is determined to inhibit the receptor if IGFBP2 levels are observed to decrease over time following said administration; or wherein the inhibitor is determined not to inhibit the receptor if IGFBP2 levels are not observed to decrease over time following said administration. In an embodiment of the invention, the IGF1R inhibitor is an antibody or antigen-binding fragment thereof that binds specifically to IGF1 R.

The present invention also provides a method for evaluating dosage of an IGF1R inhibitor administered to a subject as defined in the claims comprising evaluating IGFBP2 levels in the body of the subject over time; wherein said dosage is determined to be insufficient if IGFBP2 levels are not observed to decrease by at least 51 % over time following said administration; or wherein said dosage is determined to be sufficient if IGFBP2 levels are observed to decrease by at least 51% over time following said administration. In an embodiment of the invention, the method comprises (i) measuring an IGFBP2 level in the body of said subject before treatment with said inhibitor; (ii) measuring an IGFBP2 level in the body of said subject following administration of one or more doses of said inhibitor; (iii)

comparing the level of IGFBP2 measured in step (i) with the level of IGFBP2 measured in step (ii); wherein said dosage is determined to be insufficient if IGFBP2 levels are not observed to decrease by at least 51 % over time following said administration; or wherein said dosage is determined to be sufficient if IGFBP2 levels are observed to decrease by at least 51% over time following said administration. For example, in an embodiment of the invention, if the dosage is determined to be acceptable, the subject is continued on a therapeutic regiment comprising administering the evaluated dose.

The present invention further comprises a method for determining if a subject that suffers from cancer has a cancer that is responsive to an IGF1R inhibitor as defined in the claims, comprising administering said inhibitor to said subject and evaluating IGFBP2 levels in the body of the subject over time; wherein said condition is determined to be unresponsive to said inhibitor if the IGFBP2 levels are not observed to decrease over time following said administration. In an embodiment of the invention, the method comprises: (i) measuring an IGFBP2 level in the body of said subject before treatment with said inhibitor; (ii) measuring an IGFBP2 level in the body of said subject following administration of one or more doses of said inhibitor; (iii) comparing the level of IGFBP2 measured in step (i) with the level of IGFBP2 measured in step (ii); wherein said cancer is determined to be unresponsive to said inhibitor if the IGFBP2 levels are not observed to decrease over time following said administration.

For example, the subject is undergoing a therapeutic regimen comprising administering the IGF1R inhibitor, *e.g*., at a dosage of 0.3, 1, 3, 10 or 20 mg/kg once a week.

Disclosed is further a method for treating a medical condition, in a subject, mediated by IGF1R expression or activity comprising: (i) measuring an IGFBP2 level in the body of said subject prior to any administration of an IGF1R inhibitor; (ii) administering one or more doses of an IGF1R inhibitor to said subject; (iii) measuring an IGFBP2 level in the body of said subject following said administration; (iv) comparing the level of IGFBP2 measured in step (i) with the level of IGFBP2 measured in step (iii); and (v) increasing dosage of said inhibitor if the IGFBP2 level does not decrease by at least 51 % following said administration. Dosage may be maintained if the 51% target is reached. Dosage may be decreased if IGFBP2 levels are reduced significantly and unacceptably below 51%.

Disclosed is also a method for selecting a dose of an IGF1R inhibitor comprising administering a dose of said inhibitor to a subject with a medical condition mediated by IGF1R expression or activity and evaluating IGFBP2 levels in the body of the subject; wherein said dosage is selected if IGFBP2 levels are observed to decrease by at least 51 % of an IGFBP2 level measured prior to first administration of said inhibitor following said administration. For example, the method comprises (i) measuring an IGFBP2 level in the body of said subject before treatment with said inhibitor; (ii) administering one or more doses of said inhibitor to said subject (*e.g*., wherein the doses are of a common amount and frequency); (iii) measuring an IGFBP2 level in the body of said subject following said administration; and (iv) comparing the level of IGFBP2 measured in step (i) with the level of IGFBP2 measured in step (iii); wherein said dose is selected if IGFBP2 levels are observed to decrease by at least 51% of an IGFBP2 level measured prior to first administration of said. inhibitor following said administration For example, a therapeutic regimen comprising administration of the dose is continued if the dose is selected.

In an embodiment of any of the inventions discussed herein, the IGF1R inhibitor is an antibody or antigen-binding fragment thereof that binds specifically to IGF1R, *e.g*., wherein the antibody or fragment comprises one or more complementarity determining regions (CDRs) selected from the group consisting of:
RASQSIGSSLH (SEQ ID NO:) *e.g*., which is CDR-L1;
YASQSLS (SEQ ID NO:) *e.g*., which is CDR-L2;
HQSSRLPHT (SEQ ID NO:) *e.g*., which is CDR-L3;
SFAMH (SEQ ID NO:) *e.g*., which is CDR-H1
GFTFSSFAMH (SEQ ID NO:) *e.g*., which is CDR-H1;
VIDTRGATYYADSVKG (SEQ ID NO:) *e.g*., which is CDR-H2;
LGNFYYGMDV (SEQ ID NO:) *e.g*., which is CDR-H3;
or a mature fragment of a light chain immunoglobulin which comprises the amino acid sequence of SEQ ID NO: 2, 4, 6 or 8; or a mature fragment of a heavy chain immunoglobulin which comprises the amino acid sequence of SEQ ID NO: 10 or 12; or a pharmaceutical composition thereof which comprises a pharmabeutically acceptable carrier.

In an embodiment of any of the inventions discussed herein the subject suffers from *e.g*., osteosarcoma, rhabdomyosarcoma, neuroblastoma, any pediatric cancer, kidney cancer, leukemia, renal transitional cell cancer, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, vasoactive intestinal peptide secreting tumors,

Head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, hairy cell leukemia, mast cell leukemia, mast cell neoplasm, follicular lymphoma, diffuse large cell lymphoma, mantle cell lymphoma, Burkitt Lymphoma, mycosis fungoides, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and a myeloproliferative disorder, soft tissue sarcoma, thyroid cancer, endometrial cancer, carcinoid cancer, germ cell tumors, liver cancer.

In an embodiment of any of the inventions discussed herein, the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an IGFR-TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 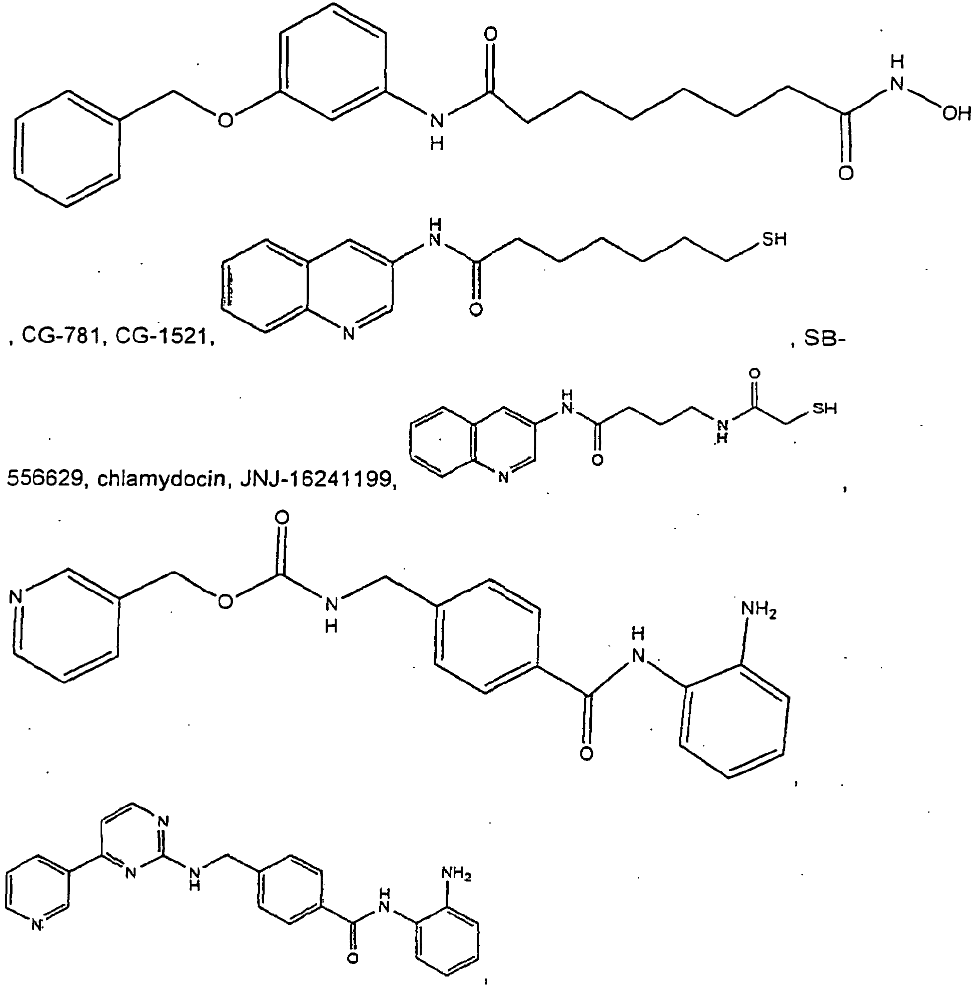 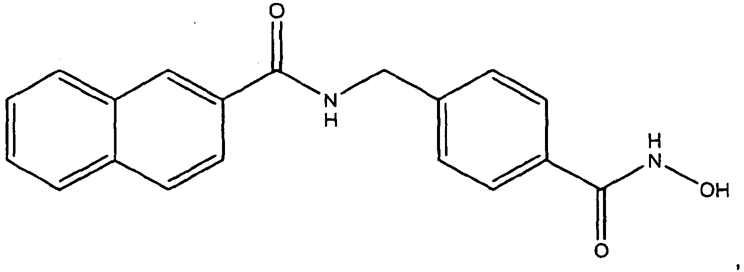 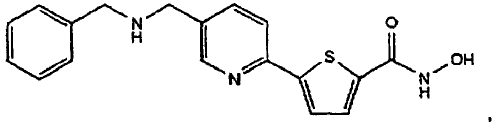 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N-[4-(2-(2-amino-4,7-dihydro-4-oxo-1H-pyrrolo[2,3- d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 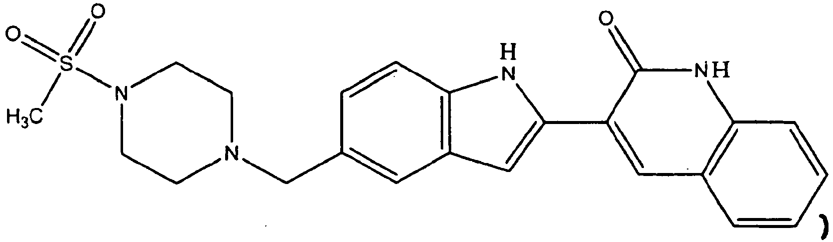 3-[5-(methylsulfonylpiperadinemethyl)-indolyll-quinolone, vatalanib, AG-013736, AVE-0005, the acetate salt of [D-Ser(Bu t) 6 ,Azgly 10] (pyro-Glu-His-Trp-Ser-Tyr-D-Ser(Bu t)-Leu-Arg-Pro-Azgly-NH₂ acetate [C₅₉H₈₄N₁₈O₁₄ ·(C₂H₄O₂)ₓ where x = 1 to 2.4], goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, lonafarnib, 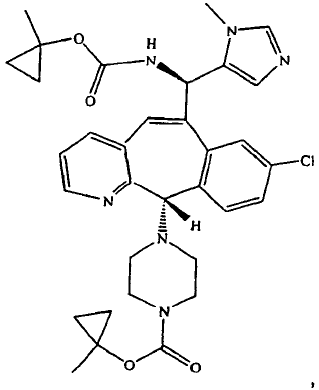 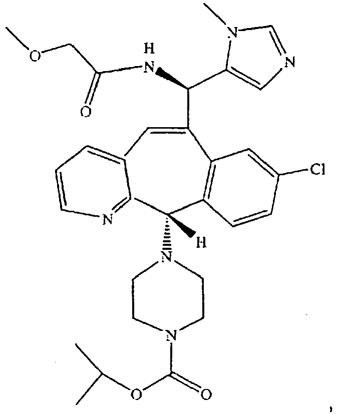 BMS-214662, tipifarnib; amifostine, NVP-LAQ824, suberoyl analide hydroxamic acid, valproic acid, trichostatin A, FK-228, SU 11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, , diphenhydramine, hydroxyzine, metoclopramide,, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa. In an embodiment of any of the inventions discussed herein the IGFBP2 level is determined using a radioimmunoassay (RIA), a western blot or an enzyme linked immunosorbent assay (ELISA) of a sample from the patient. Also, in an embodiment of any of the inventions discussed herein the sample which is evaluated for IGFBP2 concentration is blood or plasma from the patient.

### Detailed Description of the Invention

As is discussed herein, IGFBP2 levels have demonstrated to be a very useful pharmacokinetic marker for the magnitude by which an IGF1R inhibitor is inhibiting the IGF1R pathway in cells (*e.g*., malignant cells) in a subject's body. This data provides information which is valuable to a clinician in evaluating the appropriateness of a given dosage of the inhibitor. If, in view of the IGFBP2 levels observed, the IGF1R pathway is deemed, in the clinician's expert judgment, not to be sufficiently inhibited, then the dosage may be increased. If dosage and inhibition of the pathway is deemed sufficient, then dosage may be maintained. If dosage in pathway inhibition is deemed to be too high, dosage may be reduced.

It has been determined that the point at which IGFBP2 levels decrease in the blood of a subject by at least 51% (*e.g*., at least 52%, at least 53%, at least 54%, at least 55%, at least 56%, at least 57%, at least 58%, at least 59%, at least 60%, at least 61 %, at least 62%, at least 63%, at least 64%, at least 65%, at least 66%, at least 67%, at least 68%, at least 69%, at least 70%, at least 71 %, at least 72%, at least 73%, at least 74%, at least 75%, at least 76%, at least 77%, at least 78%, at least 79%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, at least 98%, at least 99%, or 100%) during course of an IGF1R inhibitor (*e.g*., an anti-IGF1R antibody as discussed herein) treatment regimen, receptors in the body of the subject are essentially saturated with the inhibitor. This point makes 51 % a very useful pharmacokinetic target in the treatment of any disease mediated by IGF1R expressioin or activity with an IGF1R inhibitor. The 51 % target or any of the target percentages discussed above (*e.g*., at least 60%, at least 70%, *etc*.) may be used at the target in connection with any of the methods discussed herein. Any such embodiment forms part of the present invention.

Furthermore, an aspect of the invention includes determining whether a patient exhibits elevated IGFBP2 levels. As discussed herein, the IGFBP2 levels in a patient has been correlated to tumor size: higher levels of IGFBP2 correlate to a large tumor size and *vice versa.*

The terms insulin-like growth factor-binding protein 2, IGFBP-2, IBP- 2 or IGF-binding protein 2 are well known in the art. In an embodiment of the invention, an IGFBP2 is human, *e.g*., which comprises the following amino acid sequence: See also UniProtKB/Swiss-Prot accession no. P18065, Genbank accession no. NP_000588: accession nos. IPI00297284.1 and M35410 and EMBL accession nos. A09809.

The term "IGF1 R" or "insulin-like growth factor-1 receptor", or the like, includes any species of IGF1R, *e.g*., human IGF1R.

In an embodiment, an antibody or antigen-binding fragment thereof that binds "specifically" to IGF1R (*e.g*., human IGF1R) binds with a Kd of about 10⁻⁸ M or 10⁻⁷ M or a lower number; or, in an embodiment of the invention, with a Kd of about 1.28X10⁻¹⁰ M or a lower number by Biacore measurement or with a Kd of about 2.05X10⁻¹² or a lower number by KinExA measurement. In another embodiment, an antibody that binds "specifically" to human IGF1R binds exclusively to human IGF1R and to no other protein (*e.g*., non-human IGF1 R).

### IGF1R inhibitors

The terms "IGF1 R inhibitor" or "IGF1 R antagonist" or the like include any substance that decreases the expression, ligand binding (*e.g*., binding to IGF-1 and/or IGF-2), kinase activity (*e.g*., autophosphorylation activity) or any other biological activity of IGF1R (*e.g*., mediation of anchorage independent cellular growth) that will elicit a biological or medical response of a tissue, system, subject or patient that is being sought by the administrator (such as a researcher, doctor or veterinarian) which includes any measurable alleviation of the signs, symptoms and/or clinical indicia of cancer (*e.g*., tumor growth) and/or the prevention, slowing or halting of progression or metastasis of cancer (*e.g*., neuroblastoma) to any degree.

An IGF1R inhibitor that is administered to a patient in a method described herein is any isolated antibody or antigen-binding fragment thereof that binds specifically to insulin-like growth factor-1 receptor (*e.g*., human IGF1R) or any soluble fragment thereof (*e.g*., monoclonal antibodies (*e.g*., fully human monoclonal antibodies), polyclonal antibodies, bispecific antibodies, Fab antibody fragments, F(ab)₂ antibody fragments, Fv antibody fragments (*e.g*., VH or VL), single chain Fv antibody fragments, dsFv antibody fragments, humanized antibodies, chimeric antibodies or anti-idiotypic antibodies) such as any of those disclosed in any of Burtrum et. al Cancer Research 63:8912-8921(2003); in French Patent Applications FR2834990, FR2834991 and FR2834900 and in PCT Application Publication Nos. WO 03/100008; WO 03/59951; WO 04/71529; WO 03/106621; WO 04/83248; WO 04/87756, WO 05/16970; and WO 02/53596.

An IGF1R inhibitor that is administered to a patient in a method described herein is an isolated anti-insulin-like growth factor-1 receptor (IGF1R) antibody comprising a mature 19D12/15H12 Light Chain (LC)-C, D, E or F and a mature 19D12/15H12 heavy chain (HC)-A or B (*e.g*., mature LCF/mature HCA). An IGF1R inhibitor that is administered to a patient in a method described herein is an isolated antibody that specifically binds to IGF1R that comprises one or more complementarity determining regions (CDRs) of 19D12/15H12 Light Chain-C, D, E or F and/or 19D12/15H12 heavy chain-A or B (*e.g*., all 3 light chain CDRs and all 3 heavy chain CDRs).

The amino acid and nucleotide sequences of the some antibody chains of the invention are shown below. Dotted, underscored type indicates the signal peptide. Solid underscored type indicates the CDRs. Plain type indicates the framework regions. Mature or processed fragments lack the signal peptide (such mature fragments and antibodies or antigen-binding fragments thereof including such mature fragments form part of the present invention along with their uses).

### Modified 19D12/15H12 Light Chain-C (SEQ ID NO: 1)

### (SEQ ID NO: 2)

### Modified 19D12/15H12 Light Chain-D (SEQ ID NO: 3)

### (SEQ ID NO: 4)

### Modified 19D12/15H12 Light Chain-E (SEQ ID NO: 5)

### (SEQ ID NO: 6)

### Modified 19D12/15H12 Light Chain-F (SEQ ID NO: 7)

### (SEQ ID NO: 8)

### Modified 19D12/15H12 heavy chain-A (SEQ ID NO: 9)

### (SEQ ID NO: 10)

### Modified 19D12/15H12 heavy chain-B (SEQ ID NO: 11)

### (SEQ ID NO: 12)

Cell lines containing plasmids comprising a CMV promoter operably linked to the 15H12/19D12 light chains and heavy chains have been deposited at the American Type Culture Collection (ATCC); 10801 University Boulevard; Manassas, Virginia 20110-2209 on May 21, 2003. The deposit name and the ATCC accession numbers for the cell lines are set forth below:
CMV promoter-15H12/19D12 LCC (κ)-
   Deposit name: "15H12/19D12 LCC (κ)";
   ATCC accession No.: PTA-5217
CMV promoter-15H12/19D12 LCD (κ)-
   Deposit name: "15H12/19D12 LCD (κ)";
   ATCC accession No.: PTA-5218
CMV promoter-15H12/19D12 LCE (κ)-
   Deposit name: "15H12/19D12 LCE (κ)";
   ATCC accession No.: PTA-5219
CMV promoter-15H12/19D12 LCF (κ)-
   Deposit name: "15H12/19D12 LCF (κ)";
   ATCC accession No.: PTA-5220
CMV promoter-15H12/19D12 HCA (γ4)-
   Deposit name: "15H12/19D12 HCA (γ4)"
   ATCC accession No.: PTA-5214
CMV promoter-15H12/19D12 HCB (y4)-
   Deposit name: "15H12/19D12 HCB (γ4)"
   ATCC accession No.: PTA-5215
CMV promoter-15H12/19D12 HCA (γ1)-
   Deposit name: "15H12/19D12 HCA (γ1)";
   ATCC accession No.: PTA-5216

All restrictions on access to the cell lines deposited in ATCC will be removed upon grant of a patent. The present invention includes methods and compositions (*e.g*., any disclosed herein) comprising anti-IGF1R antibodies and antigen-binding fragments thereof comprising any of the light and/or heavy immunoglobulin chains or mature fragments thereof located in any of the foregoing plasmids deposited at the ATCC.

In an embodiment of the invention, the IGF1R inhibitor is an isolated antibody or antigen-binding fragment thereof comprising one or more (*e.g*., 3) of the following CDR sequences:
RASQSIGSSLH (SEQ ID NO: 99);
YASQSLS (SEQ ID NO: 100);
HQSSRLPHT (SEQ ID NO: 101);
SFAMH (SEQ ID NO: 102);
VIDTRGATYYADSVKG (SEQ ID NO: 103);
LGNFYYGMDV (SEQ ID NO: 104).

For example, in an embodiment of the invention, a light chain immunoglobulin comprises 3 CDRs and/or a heavy chain immunoglobulin comprises 3 CDRs.

In an embodiment, an antibody that binds "specifically" to human IGF1R binds with a Kd of about 10⁻⁸ M or 10⁻⁷ M or a lower number; or, in an embodiment of the invention, with a Kd of about 1.28X10⁻¹⁰ M or a lower number by Biacore measurement or with a Kd of about 2.05X10⁻² or a lower number by KinExA measurement. In another embodiment, an antibody that binds "specifically" to human IGF1R binds exclusively to human IGF1R and to no other protein at significant or at detectable levels.

An IGF1R inhibitor that is administered to a patient in a method described herein comprises any light chain immunoglobulin and/or a heavy chain immunoglobulin as set forth in Published International Application No. WO 2002/53596.

For example, in an embodiment, the antibody comprises a light chain variable region comprising an amino acid sequence selected from the group consisting of SEQ ID NOs: 2, 6, 10, 14, 18, 22, 47 and 51 as set forth in WO 2002/53596 and/or a heavy chain variable region comprising an amino acid sequence selected from the group consisting of SEQ ID NOs: 4, 8, 12, 16, 20, 24, 45 and 49 as set forth in WO 2002/53596. In an embodiment, the antibody comprises a heavy and/or light chain selected from that of antibody 2.12.1; 2.13.2; 2.14.3; 3.1.1; 4.9.2; and 4.17.3 in WO 2002/53596.

An IGF1R inhibitor that can be administered to a patient comprises any light chain immunoglobulin and/or a heavy chain immunoglobulin as set forth in Published International Application No. WO 2003/59951.

For example, in an embodiment, the antibody comprises a light chain variable region comprising an amino acid sequence selected from the group consisting of SEQ ID NOs: 54, 61 and 65 as set forth in WO 2003/59951 and/or a heavy chain variable region comprising an amino acids sequence selected from the group consisting of SEQ ID NOs: 69, 75, 79 and 83 as set forth in WO 2003/59951.

An IGF1R inhibitor that can be administered to a patient comprises any light chain immunoglobulin and/or a heavy chain immunoglobulin as set forth in Published International Application No. WO 2004/83248.

For example, in an embodiment, the antibody comprises a light chain variable region comprising an amino acid sequence selected from the group consisting of SEQ ID NOs: 109, 111, 113, 115, 117, 119, 121, 123, 125, 127, 129, 131, 133, 135, 137, 139, 141 and 143 as set forth in WO 2004/83248 and/or a heavy chain variable region comprising an amino acids sequence selected from the group consisting of SEQ ID NOs: 108, 110, 112, 114, 116, 118, 120, 122, 124, 126, 128, 130, 132, 134, 136, 138, 140 and 142 as set forth in WO 2004/83248. In an embodiment, the antibody comprises a light and/or heavy chain selected from that of PINT-6A1; PINT-7A2; PINT-7A4; PINT-7A5; PINT-7A6; PINT-8A1; PINT-9A2; PINT-11A1; RINT-11A2; PINT-11A3; PINT-11A4; PINT-11A5; PINT-11A7; PINT-12A1; PINT-12A2; PINT-12A3; PINT-12A4 and PINT-12A5 in WO 2004/83248.

An IGF1R inhibitor that can be administered to a patient comprises any light chain immunoglobulin and/or a heavy chain immunoglobulin as set forth in Published International Application No. WO 2003/106621.

For example, in an embodiment, the antibody comprises a light chain variable region comprising an amino acid sequence selected from the group consisting of SEQ ID NOs: 8-12, 58-69, 82-86, 90, 94, 96, 98, as set forth in WO 2003/106621 and/or a heavy chain variable region comprising an amino acids sequence selected from the group consisting of SEQ ID NOs: 7, 13, 70-81, 87, 88, 92 as set forth in WO 2003/106621.

An IGF1R inhibitor that can be administered to a patient comprises any light chain immunoglobulin and/or a heavy chain immunoglobulin as set forth in Published International Application No. WO 2004/87756.

For example, in an embodiment, the antibody comprises a light chain variable region comprising an amino acid sequence of SEQ ID NO: 2 as set forth in WO 2004/87756 and/or a heavy chain variable region comprising an amino acid sequence of SEQ ID NO: 1 as set forth in WO 2004/87756.

An IGF1R inhibitor that can be administered to a patient comprises any light chain immunoglobulin and/or a heavy chain immunoglobulin as set forth in Published International Application No. WO 2005/16970.

For example, in an embodiment, the antibody comprises a light chain variable region comprising an amino acid sequence of SEQ ID NO: 6 or 10 as set forth in WO 2005/16970 and/or a heavy chain variable region comprising an amino acid sequence of SEQ ID NO: 2 as set forth in WO 2005/16970.

In an embodiment of the invention, an anti-IGF1R antibody or antigen-binding fragment thereof of the invention comprises an immunoglobulin heavy chain variable region comprising an amino acid sequence selected from the group consisting of:

In an embodiment of the invention, an anti-IGF1R antibody or antigen-binding fragment thereof of the invention comprises an immunoglobulin light chain variable region comprising an amino acid sequence selected from the group consisting of:

In an embodiment of the invention, the anti-IGF1R antibody comprises a light chain immunoglobulin, or a mature fragment thereof (*i.e.,* lacking signal sequence), or variable region thereof, comprising the amino acid sequence of: or (SEQ ID NO: 28). In an embodiment of the invention, the signal sequence is amino acids 1-22 of SEQ ID NOs: 25-28. In an embodiment of the invention, the mature variable region is underscored. In an embodiment of the invention, the CDRs are in bold/italicized font. In an embodiment of the invention, the anti-IGF1 R antibody or antigen-binding fragment thereof of the invention comprises one or more CDRs (*e.g*., 3 light chain CDRS) as set forth above.

In an embodiment of the invention, the anti-IGF1R antibody comprises a heavy chain immunoglobulin or a mature fragment thereof (i.e., lacking signal sequence), or a variable region thereof, comprising the amino acid sequence of: or (SEQ ID NO: 32). In an embodiment of the invention, tne signal sequence is amino acids 1-19 of SEQ ID NOs: 29-32. In an embodiment of the invention, the mature variable region is underscored. In an embodiment of the invention, the anti-IGF1R antibody or antigen-binding fragment thereof of the invention comprises one or more CDRs (*e.g*., 3 light chain CDRS) as set forth above.

In an embodiment of the invention, the anti-IGF1R antibody comprises a light chain variable region comprising the amino acid sequence of any of SEQ ID NOs: 19-24 paired with a heavy chain variable region comprising an amino acid sequence of any of SEQ ID NOs: 13-18, respectively. In an embodiment of the invention, the anti-IGF1R antibody comprises a mature light chain variable region comprising an amino acid sequence of any of SEQ ID NOs: 25 or 26 paired with a heavy chain variable region comprising an amino acid sequence of any of SEQ ID NOs: 29 or 30. In an embodiment of the invention, the anti-IGF1R antibody comprises a mature light chain variable region comprising an amino acid sequence of any of SEQ ID NOs: 27 or 28 paired with a heavy chain variable region comprising an amino acid sequence of any of SEQ ID NOs: 31 or 32.

In an embodiment of the invention, an anti-IGF1R antibody or antigen-binding fragment thereof of the invention comprises an immunoglobulin heavy chain or mature fragment or variable region of 2.12.1 fx (SEQ ID NO: 33) (in an embodiment of the invention, the leader sequence is underscored; in an embodiment of the invention, the CDRs are in bold/italicized font):

In an embodiment of the invention, the anti-IGF1R antibody or antigen-binding fragment thereof of the invention comprises amino acids 20-470 of 2.12.1 fx (SEQ ID NO: 33).

In an embodiment of the invention, an anti-IGF1R antibody or antigen-binding fragment thereof of the invention comprises mature immunoglobulin heavy chain variable region 2.12.1 fx (amino acids 20-144 or SEQ ID NO: 33; SEQ ID NO: 34):
q vqlvesgggl vkpggslrls caasgftfsd yymswirqap gkglewvsyi sssgstrdya dsvkgrftis rdnaknslyl qmnslraedt avyycardgv ettfyyyyyg mdvwgqgttv tvss

In an embodiment of the invention, an anti-IGF1R antibody or antigen-binding fragment thereof of the invention comprises an immunoglobulin light chain or mature fragment or variable region 2.12.1 fx (SEQ ID NO: 35) (in an embodiment of the invention, the leader sequence is underscored; in an embodiment of the invention, the CDRs are in bold/italicized font):

In an embodiment of the invention, an anti-IGF1R antibody or antigen-binding fragment thereof of the invention comprises amino acids 23-236 of 2.12.1 fx (SEQ ID NO: 35).

In an embodiment of the invention, an anti-IGF1R antibody or antigen-binding fragment thereof of the invention comprises mature immunoglobulin light chain variable region 2.12.1 fx (amino acids 23-130 of SEQ ID NO: 35; SEQ ID NO: 36):

In an embodiment of the invention, an anti-IGF1R antibody or antigen-binding fragment thereof comprises or consists of a light chain immunoglobulin chain comprising or consisting of amino acids 23-236 of 2.12.1 fx (SEQ ID NO: 35) and a heavy chain immunoglobulin chain comprising or consisting of amino acids 20-470 of 2.12.1 fx (SEQ ID NO: 33).

In an embodiment of the invention, the anti-IGF1R antibody or antigen-binding fragment thereof comprises one or more 2.12.1 fx CDRs (*e.g*., 3 light chain CDRs and/or 3 heavy chain CDRs) as set forth above.

In an embodiment of the invention, an anti-IGF1R antibody or antigen-binding fragment thereof of the invention or antigen-binding fragment thereof comprises a humanized 7C10 immunoglobulin light chain variable region; version 1 (SEQ ID NO: 37):

In an embodiment of the invention, an anti-IGF1R antibody or antigen-binding fragment thereof of the invention comprises humanized 7C10 immunoglobulin light chain variable region; version 2 (SEQ ID NO: 38):

In an embodiment of the invention, an anti-IGF1R antibody or antigen-binding fragment thereof of the invention comprises a humanized 7C10 immunoglobulin heavy chain variable region; version 1 (SEQ ID NO: 39):

In an embodiment of the invention, an anti-IGF1R antibody or antigen-binding fragment thereof of the invention comprises the humanized 7C10 immunoglobulin heavy chain variable region; version 2 (SEQ ID NO: 40):

In an embodiment of the invention, an anti-IGF1R antibody or antigen-binding fragment thereof of the invention comprises the humanized 7C10 immunoglobulin heavy chain variable region; version 3 (SEQ ID NO: 41):

In an embodiment of the invention, an anti-IGF1R antibody or antigen-binding fragment thereof of the invention comprises A12 immunoglobulin heavy chain variable region (SEQ ID NO: 42):

In an embodiment of the invention, an anti-IGF1R antibody or antigen-binding fragment thereof of the invention comprises A12 immunoglobulin light chain variable region (SEQ ID NO: 43): or
(SEQ ID NO: 106):

In an embodiment of the invention, an anti-IGF1R antibody or antigen-binding fragment thereof of the invention comprises 1A immunoglobulin heavy chain variable region (SEQ ID NO: 44): ;optionally including one or more of the following mutations: R30, S30, N31, S31, Y94, H94, D104, E104.

In an embodiment of the invention, an anti-IGF1R antibody or antigen-binding fragment thereof of the invention comprises 1A immunoglobulin light chain variable region (SEQ ID NO: 45): ;optionally including one or more of the following mutations: P96, I96, P100, Q100, R103, K103, V104, L104, .D105, E105

In an embodiment of the invention, an anti-IGF1R antibody or antigen-binding fragment thereof of the invention comprises single chain antibody (fv) 8A1 (SEQ ID NO: 46):

In an embodiment of the invention, an anti-IGF1R antibody or antigen-binding fragment thereof of the invention comprises single chain antibody (fv) 9A2 (SEQ ID NO: 47):

In an embodiment of the invention, an anti-IGF1R antibody or antigen-binding fragment thereof of the invention comprises single chain antibody (fv) 11A4 (SEQ ID NO: 48):

In an embodiment of the invention, an anti-IGF1R antibody or antigen-binding fragment thereof of the invention comprises single chain antibody (fv) 7A4 (SEQ ID NO: 49):

In an embodiment of the invention, an anti-IGF1R antibody or antigen-binding fragment thereof of the invention comprises single chain antibody (fv) 11A1 (SEQ ID NO: 50):

In an embodiment of the invention, an anti-IGF1R antibody or antigen-binding fragment thereof of the invention comprises single chain antibody (fv) 7A6 (SEQ ID NO: 51)

In an embodiment of the invention, an anti-IGF1R antibody or an antigen-binding fragment thereof (*e.g*., a heavy chain or light chain immunoglobulin) of the invention comprises one or more complementarity determing regions (CDR) selected from the group consisting of:
sywmh (SEQ ID NO: 52);
einpsngrtnynekfkr (SEQ ID NO: 53);
grpdyygsskwyfdv (SEQ ID NO: 54);
rssqsivhsnvntyle (SEQ ID NO: 55);
kvsnrfs (SEQ ID NO: 56); and
fqgshvppt (SEQ ID NO: 57).

In an embodiment of the invention, an anti-IGF1R antibody or an antigen-binding fragment thereof of the invention comprises a heavy chain immunoglobulin variable region selected from the group consisting of : and/or a light chain immunoglobulin variable region selected from the group consisting of:

Disclosed are methods wherein a patient is administered an anti-insulin-like growth factor receptor-1 (IGF1R) antibody wherein the variable region of the antibody is linked to any immunoglobulin constant region. In an embodiment, the light chain variable region is linked to a κ chain constant region. In an embodiment, the heavy chain variable region is linked to a γ1, γ2, γ3 or γ4 chain constant region. Any of the immunoglobulin variable regions set forth herein, in embodiments of the invention, can be linked to any of the foregoing constant regions.

Furthermore, disclosed herein is any antibody or antibody fragment comprising one or more CDRs (3 light chain CDRs and/or 3 heavy chain CDRs) and/or framework regions of any of the light chain immunoglobulin or heavy chain immunoglobulins set forth herein as identified by any of the methods set forth in Chothia et al., J. Mol. Biol. 186:651-663 (1985); Novotny and Haber, Proc. Natl. Acad. Sci. USA 82:4592-4596 (1985) or Kabat, E. A. et al., Sequences of Proteins of Immunological Interest, National Institutes of Health, Bethesda, Md., (1987)).

In an embodiment of the invention, the term "monoclonal antibody," as used herein, refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Monoclonal antibodies are advantageous in that they may be synthesized by a hybridoma culture, essentially uncontaminated by other immunoglobulins. The modifier "monoclonal" indicates the character of the antibody as being amongst a substantially homogeneous population of antibodies, and is not to be construed as requiring production of the antibody by any particular method. As mentioned above, the monoclonal antibodies to be used in accordance with the present invention may be made by the hybridoma method described by Kohler, et al., (1975) Nature 256: 495.

In an embodiment of the invention, a polyclonal antibody is an antibody which was produced among or in the presence of one or more other, non-identical antibodies. In general, polyclonal antibodies are produced from a B-lymphocyte in the presence of several other B-lymphocytes which produced non-identical antibodies. Usually, polyclonal antibodies are obtained directly from an immunized animal.

In an embodiment of the invention, a bispecific or bifunctional antibody is an artificial hybrid antibody having two different heavy/light chain pairs and two different binding sites. Bispecific antibodies can be produced by a variety of methods including fusion of hybridomas or linking of Fab' fragments. See, *e.g*., Songsivilai, et al., (1990) Clin. Exp. Immunol. 79: 315-321, Kostelny, et al., (1992) J Immunol. 148:1547- 1553. In addition, bispecific antibodies may be formed as "diabodies" (Holliger, et al., (1993) Proc. Nat. Acad. Sci. USA 90:6444-6448) or as "Janusins" (Traunecker, et al., (1991) EMBO J. 10:3655-3659 and Traunecker, et al., (1992) Int. J. Cancer Suppl. 7:51-52).

In an embodiment of the invention, the term "fully human antibody" refers to an antibody which comprises human immunoglobulin protein sequences only. A fully human antibody may contain murine carbohydrate chains if produced in a mouse, in a mouse cell or in a hybridoma derived from a mouse cell. Similarly, "mouse antibody" refers to an antibody which comprises mouse immunoglobulin protein sequences only.

The present invention includes "chimeric antibodies"- in an embodiment of the invention, an antibody which comprises a variable region of the present invention fused or chimerized with an antibody region (*e.g*., constant region) from another, human or non-human species (*e.g*., mouse, horse, rabbit, dog, cow, chicken). These antibodies may be used to modulate the expression or activity of IGF1R in a non-human species.

"Single-chain Fv" or "sFv" antibody fragments have, in an embodiment of the invention, the V_{H} and V_{L} domains of an antibody, wherein these domains are present in a single polypeptide chain. Generally, the sFv polypeptide further comprises a polypeptide linker between the V_{H} and V_{L} domains which enables the sFv to form the desired structure for antigen binding. Techniques described for the production of single chain antibodies (U.S. Patent Nos. 5,476,786; 5,132,405 and 4,946,778) can be adapted to produce anti-IGF1R-specific single chain antibodies. For a review of sFv see Pluckthun in The Pharmacology of Monoclonal Antibodies, vol. 113, Rosenburg and Moore eds. Springer-Verlag, N.Y., pp. 269-315 (1994).

In an embodiment of the invention, "disulfide stabilized Fv fragments" and "dsFv" refer to immunoglobulins comprising a variable heavy chain (V_{H}) and a variable light chain (V_{L}) which are linked by a disulfide bridge.

Antigen-binding fragments of antibodies within the scope of the present invention also include F(ab)₂ fragments which may, in an embodiment of the invention, be produced by enzymatic cleavage of an IgG by, for example, pepsin. Fab fragments may be produced by, for example, reduction of F(ab)₂ with dithiothreitol or mercaptoethylamine. A Fab fragment is, in an embodiment of the invention, a V_{L}-C_{L} chain appended to a V_{H}-C_{H1} chain by a disulfide bridge. A F(ab)₂ fragment is, in an embodiment of the invention, two Fab fragments which, in turn, are appended by two disulfide bridges. The Fab portion of an F(ab)₂ molecule includes, in an embodiment of the invention, a portion of the F_{c} region between which disulfide bridges are located.

In an embodiment of the invention, an F_{V} fragment is a V_{L} or V_{H} region.

Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), *e.g*. IgG-1, IgG-2, IgG-3 and IgG-4; IgA-1 and IgA-2. As discussed herein, any such antibody or antigen-binding fragment thereof is within the scope of the present invention.

The anti-IGF1R antibodies may, in an embodiment of the invention, be conjugated to a chemical moiety. The chemical moiety may be, *inter alia,* a polymer, a radionuclide or a cytotoxic factor. In an embodiment of the invention, the chemical moiety is a polymer which increases the half-life of the antibody or antigen-binding fragment thereof in the body of a subject. Suitable polymers include, but are not limited to, polyethylene glycol (PEG) (*e.g*., PEG with a molecular weight of 2kDa, 5 kDa, 10 kDa, 12kDa, 20 kDa, 30kDa or 40kDa), dextran and monomethoxypolyethylene glycol (mPEG). Lee, et al., (1999) (Bioconj. Chem. 10:973-981) discloses PEG conjugated single-chain antibodies. Wen, et al., (2001) (Bioconj. Chem. 12:545-553) disclose conjugating antibodies with PEG which is attached to a radiometal chelator (diethylenetriaminpentaacetic acid (DTPA)).

The antibodies and antibody fragments may, in an embodiment of the invention, be conjugated with labels such as ⁹⁹Tc,⁹⁹Y, ¹¹¹In, ³²P, ¹⁴C_{,} ¹²⁵I, ³H, ¹³¹I, ¹¹C, ¹⁵O, ¹³N, ¹⁸F, ³⁵S, ⁵¹Cr, ⁵⁷To, ²²⁶Ra, ⁶⁰Co, ⁵⁹Fe, ⁵⁷Se, ¹⁵²Eu, ⁶⁷CU, ²¹⁷Ci, ²¹¹At, ²¹²Pb, ⁴⁷Sc, ¹⁰⁹Pd, ²³⁴Th, and ⁴⁰K, ¹⁵⁷Gd, ⁵⁵Mn, ⁵²Tr and ⁵⁶Fe.

The antibodies and antibody fragments may also be, in an embodiment of the invention, conjugated with fluorescent or chemilluminescent labels, including fluorophores such as rare earth chelates, fluorescein and its derivatives, rhodamine and its derivatives, isothiocyanate, phycoerythrin, phycocyanin, allophycocyanin, o-phthaladehyde, fluorescamine, ¹⁵²Eu, dansyl, umbelliferone, luciferin, luminal label, isoluminal label, an aromatic acridinium ester label, an imidazole label, an acridimium salt label, an oxalate ester label, an aequorin label, 2,3-dihydrophthalazinediones, biotin/avidin, spin labels and stable free radicals.

The antibodies and antibody fragments may also be, in an embodiment of the invention, conjugated to a cytotoxic factor such as diptheria toxin, *Pseudomonas aeruginosa* exotoxin A chain , ricin A chain, abrin A chain, modeccin A chain, alpha-sarcin, *Aleurites fordii* proteins and compounds (e.g., fatty acids), dianthin proteins, *Phytoiacca americana* proteins PAPI, PAPII, and PAP-S, *momordica charantia* inhibitor, curcin, crotin, *saponaria officinalis* inhibitor, mitogellin, restrictocin, phenomycin, and enomycin.

Any method known in the art for conjugating the antibodies or antigen-binding fragments thereof to the various moieties may be employed, including those methods described by Hunter, et al., (1962) Nature 144:945; David, et al., (1974) Biochemistry 13:1014; Pain, et al., (1981) J. Immunol. Meth. 40:219; and Nygren, J., (1982) Histochem. and Cytochem. 30:407. Methods for conjugating antibodies are conventional and very well known in the art.

In an embodiment of the invention, an IGF1R inhibitor is BMS-577098 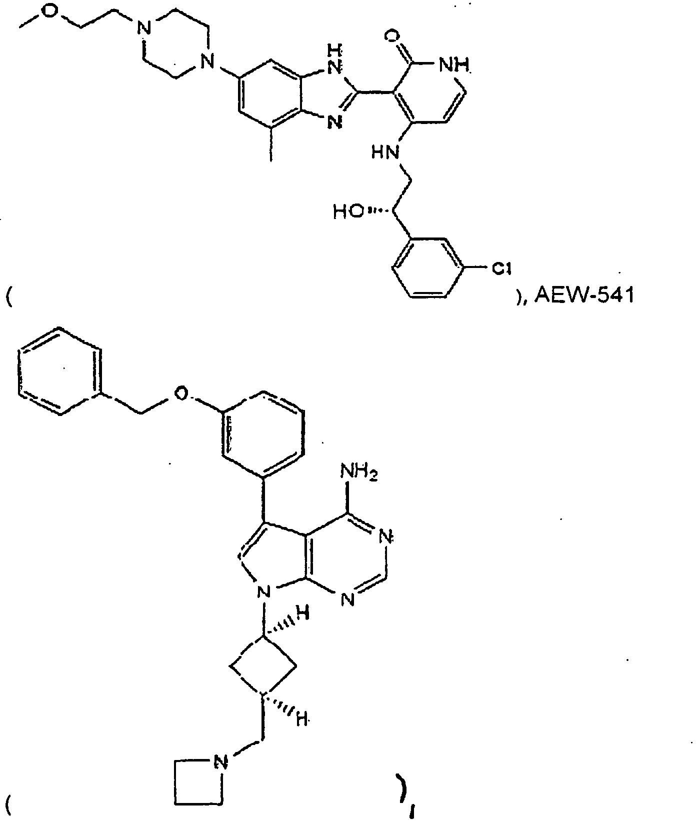 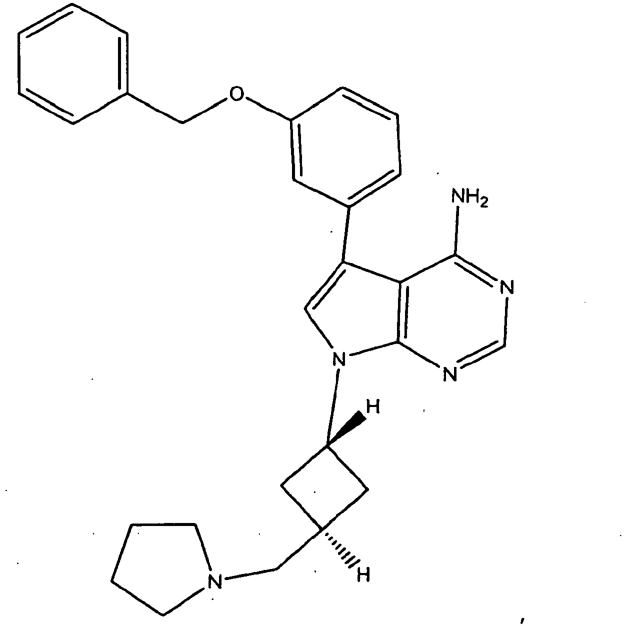 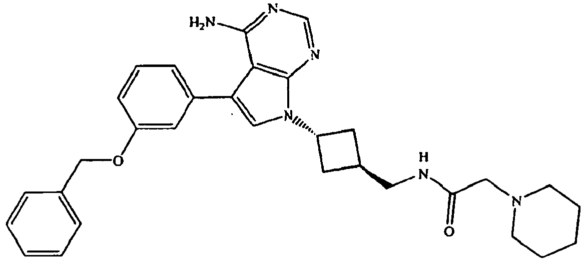 or

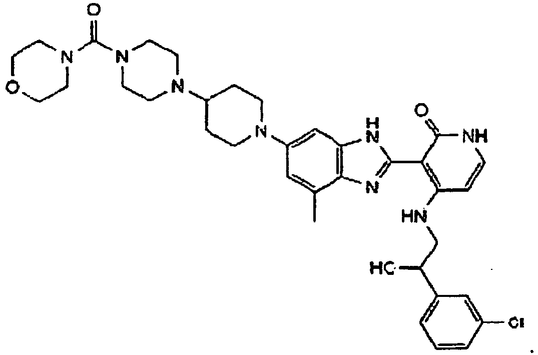

### Generation of Antibodies

Any suitable method can be used to elicit an antibody with the desired biologic properties to inhibit IGF1R. It may be desirable to prepare monoclonal antibodies (mAbs) from various mammalian hosts, such as mice, rodents, primates, humans, etc. Description of techniques for preparing such monoclonal antibodies may be found in, *e.g*., Stites, et al. (eds.) BASIC AND CLINICAL IMMUNOLOGY (4th ed.) Lange Medical Publications, Los Altos, CA, and references cited therein; Harlow and Lane (1988) ANTIBODIES: A LABORATORY MANUAL CSH Press; Goding (1986) MONOCLONAL ANTIBODIES: PRINCIPLES AND PRACTICE (2d ed.) Academic Press, New York, NY. Thus, monoclonal antibodies may be obtained by a variety of techniques familiar to researchers skilled in the art. Typically, spleen cells from an animal immunized with a desired antigen are immortalized, commonly by fusion with a myeloma cell. See Kohler and Milstein (1976) Eur. J. Immunol. 6:511-519. Alternative methods of immortalization include transformation with Epstein Barr Virus, oncogenes, or retroviruses, or other methods known in the art. See, e.g., Doyle, et al. (eds. 1994 and periodic supplements) CELL AND TISSUE CULTURE: LABORATORY PROCEDURES, John Wiley and Sons, New York, NY. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according, *e.g*., to the general protocol outlined by Huse, et al. (1989) Science 246:1275-1281.

Also, recombinant immunoglobulins may be produced, see Cabilly U.S. Patent No. 4,816,567; and Queen et al. (1989) Proc. Nat'l Acad. Sci. USA 86:10029-10033; or made in transgenic mice, see Mendez et al. (1997) Nature Genetics 15:146-156. Further methods for producing chimeric, humanized and human antibodies are well known in the art. See, *e.g.*, U.S. Pat. No. 5,530,101, issued to Queen et al, U.S. Pat. No. 5,225,539, issued to Winter et al*,* U. S. Pat. Nos. 4,816,397 issued to Boss et al.

Mammalian cell lines available as hosts for expression of antibodies of the invention are well known in the art and include many immortalized cell lines available from the American Type Culture Collection (ATCC). These include, *inter alia,* Chinese hamster ovary (CHO) cells, NSO, SP2 cells, HeLa cells, baby hamster kidney (BHK) cells, monkey kidney cells (COS), human hepatocellular carcinoma cells (*e.g*., Hep G2), A549 cells, 3T3 cells, HEK-293 cells and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. When recombinant expression vectors encoding the heavy chain or antigen-binding portion thereof, the light chain and/or antigen-binding portion thereof are introduced into mammalian host cells, the antibodies are produced by culturing the host cells for a period of time sufficient to allow for expression of the antibody in the host cells or, in an embodiment of the invention, secretion of the antibody into the culture medium in which the host cells are grown.

Antibodies can be recovered from the culture medium using standard protein purification methods. Further, expression of antibodies of the invention (or other moieties therefrom) from production cell lines can be enhanced using a number of known techniques. For example, the glutamine synthetase gene expression system (the GS system) is a common approach for enhancing expression under certain conditions. The GS system is discussed in connection with European Patent Nos. 0 216 846, 0 256 055, and 0 323 997 and European Patent Application No. 89303964.4.

It is likely that antibodies expressed by different cell lines or in transgenic animals will have different glycosylation from each other. However, all antibodies encoded by the nucleic acid molecules provided herein, or comprising the amino acid sequences provided herein are part of the instant invention, regardless of the glycosylation of the antibodies.

A convenient plasmid system useful for producing an anti-IGF1R antibody or antigen-binding fragment thereof is set forth in published U.S. application no. US2005/0176099 (see also WO2005/47512).

### Further Chemotherapeutics

The scope of the present disclosure comprises methods for treating a tumor which expresses IGF1R by administering an IGF1R inhibitor, e.g., as discussed herein, in association with a further chemotherapeutic agent or procedure. A further chemotherapeutic agent comprises any agent that elicits a beneficial physiological response in an individual to which it is administered; for example, wherein the agent alleviates or eliminates disease symptoms or causes within the subject to which it is administered. A further chemotherapeutic agent includes any anti-cancer chemotherapeutic agent. An anti-cancer therapeutic agent is any agent that, for example, alleviates or eliminates symptoms or causes of cancer in the subject to which it is administered.

In an embodiment, the further chemotherapeutic agent is one or more of etoposide (VP-16; 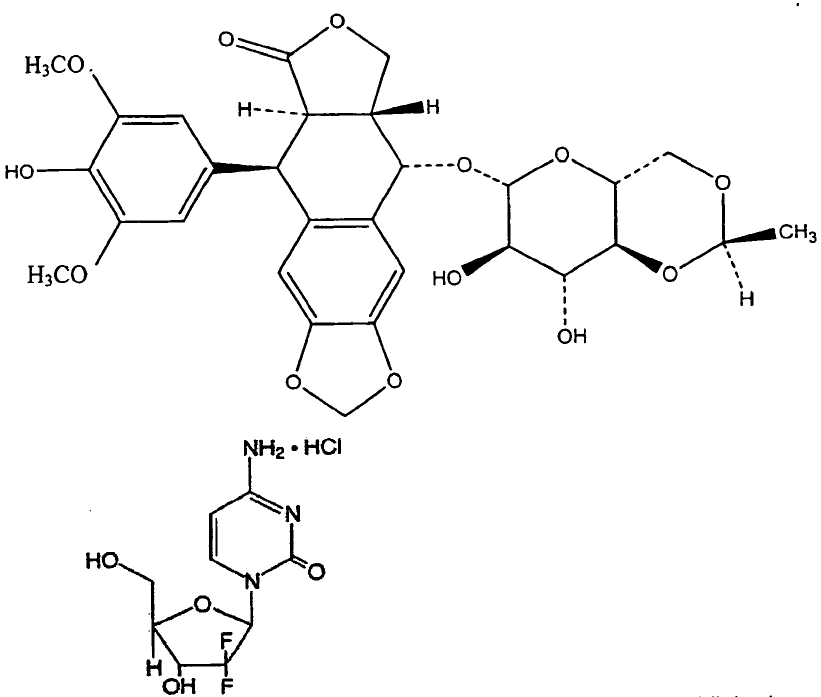 ;gemcitabine ( );any compound disclosed in published U.S. patent application no. U.S. 2004/0209878A1 (e.g., comprising a core structure represented by 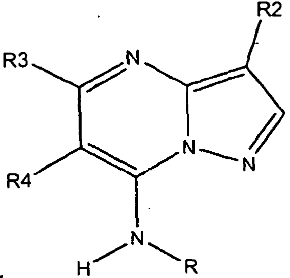 ) or doxorubicin ( 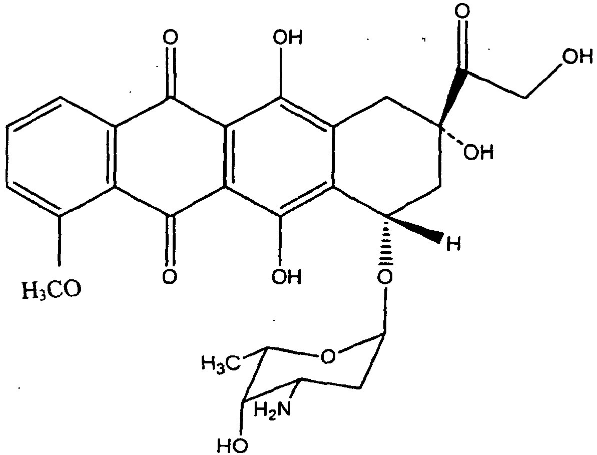 ) including Caelyx or Doxil® (doxorubicin HCl liposome injection; Ortho Biotech Products L.P; Raritan, NJ). Doxil® comprises doxorubicin in STEALTH® liposome carriers which are composed of N-(carbonyl-methoxypolyethylene glycol 2000)-1,2-distearoyl-sn-glycero-3-phosphoethanolamine sodium salt (MPEG-DSPE); fully hydrogenated soy phosphatidylcholine (HSPC), and cholesterol.

For example, the further chemotherapeutic agent is one or more of 5'-deoxy-5-fluorouridine ( 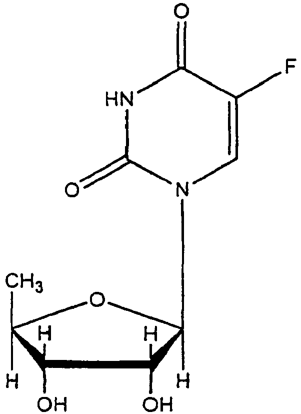 );vincristine ( 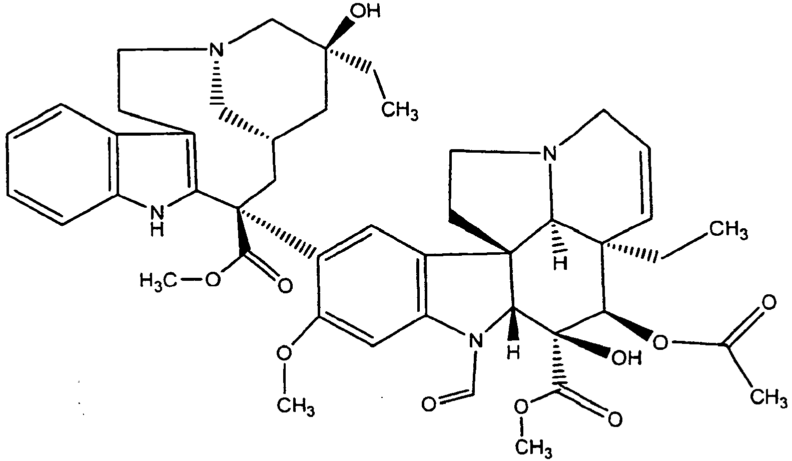 );
or temozolomide ( 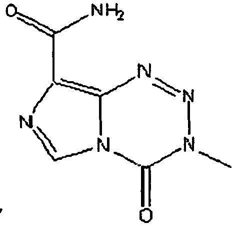 ).

In a further example, the further chemotherapeutic agent is one or more of any CDK inhibitor such as ZK-304709, Seliciclib (R-roscovitine) 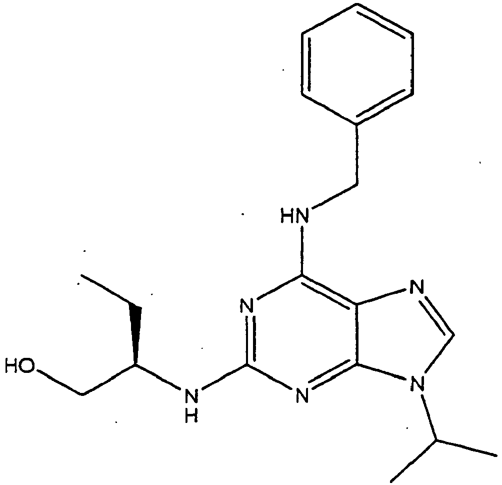 );or any MEK inhibitor such as PD0325901 ( 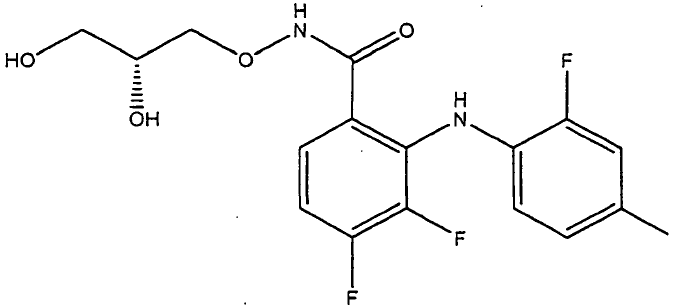 ), AZD-6244; capecitabine (5'-deoxy-5-fluoro-N-[(pentyloxy) carbonyl]-cytidine); or L-Glutamic acid, N -[4-[2-(2-amino-4,7-dihydro-4-oxo-1 H -pyrrolo[2,3- d ]pyrimidin-5-yl)ethyl]benzoyl]-, disodium salt, heptahydrate ( 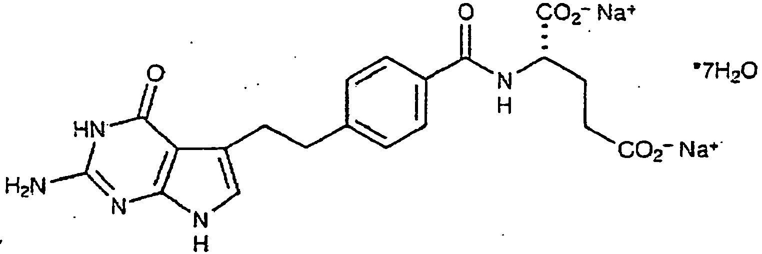 ;Pemetrexed disodium heptahydrate).

The further chemotherapeutic agent mav be one or more of camptothecin ( 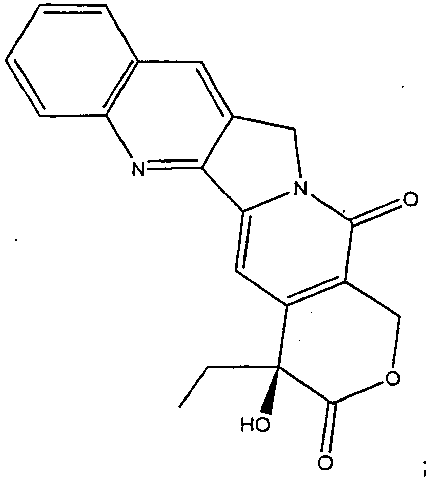 Stork et al., J. Am. Chem. Soc. 93(16): 4074-4075 (1971 ); Beisler et al., J. Med. Chem. 14(11): 1116-1117 (1962)); or irinotecan ( 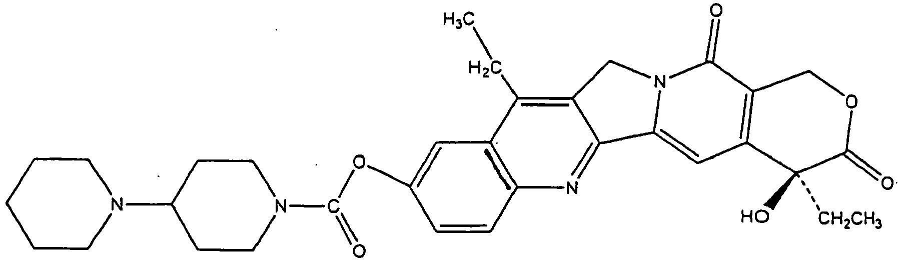 ; sold as Camptosar®; Pharmacia & Upjohn Co.; Kalamazoo, MI).

The further chemotherapeutic agent may be the FOLFOX regimen (oxaliplatin ( 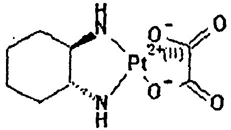 ), together with infusional fluorouracil ( 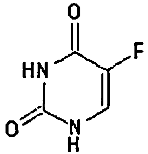 ) and folinic acid ( 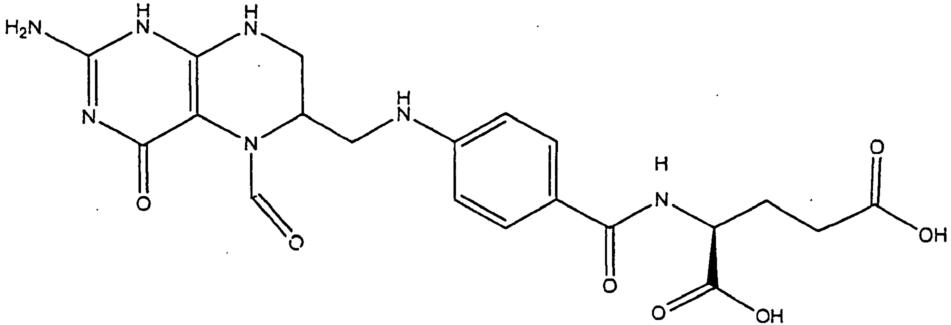 )) (Chaouche et al., Am. J. Clin. Oncol. 23(3):288-289 (2000); : de Gramont et al., J. Clin. Oncol. 18(16):2938-2947 (2000)).

The further chemotherapeutic agent may be one or more of any antiestrogen such as 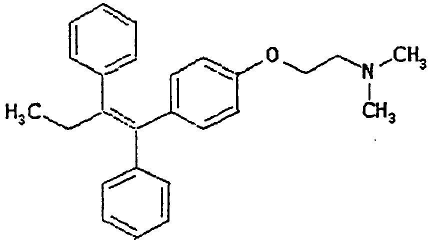 (tamoxifen; sold as Nolvadex® by AstraZeneca Pharmaceuticals LP; Wilmington, DE) or 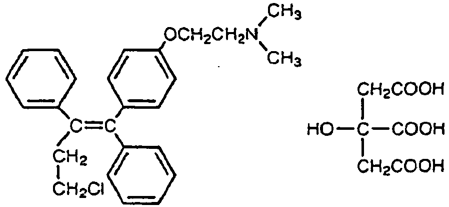 (toremifene citrate; sold as Fareston® by Shire US, Inc.; Florence, KY).

The further chemotherapeutic agent may be one or more of any aromatase inhibitor such as 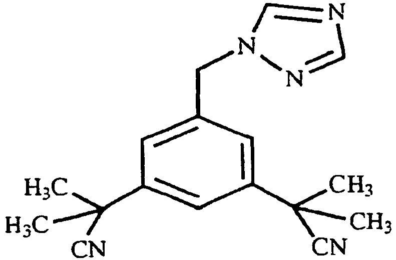 (anastrazole; sold as Arimidex® by AstraZeneca Pharmaceuticals LP; Wilmington, DE), 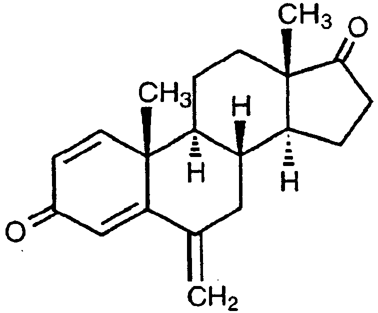 (exemestane; sold as Aromasin® by Pharmacia Corporation; Kalamazoo, MI) or 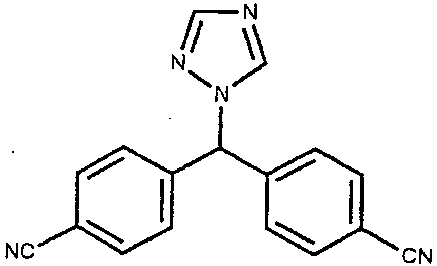 (letrozole; sold as Femara® by Novartis Pharmaceuticals Corporation; East Hanover, NJ).

The further chemotherapeutic agent may be one or more of any estrogen such as DES(diethylstilbestrol), 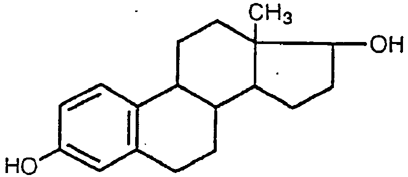 (estradiol; sold as Estrol® by Warner Chilcott, Inc.; Rockaway, NJ) or conjugated estrogens (sold as Premarin® by Wyeth Pharmaceuticals Inc.; Philadelphia, PA).

The further chemotherapeutic agent may be one or more of any anti-angiogenesis agent including bevacizumab (Avastin™; Genentech; San Francisco, CA), the anti-VEGFR-2 antibody IMC-1C11, other VEGFR inhibitors such as: CHIR-258 ( 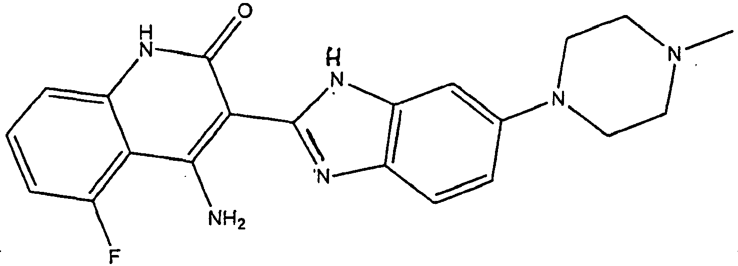 ), any of the inhibitors set forth in WO2004/13145 (*e.g.*, comprising the core structural formula: 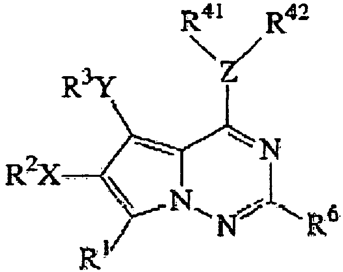 ), in WO2004/09542 (*e.g*.,.comprising the core structural formula: 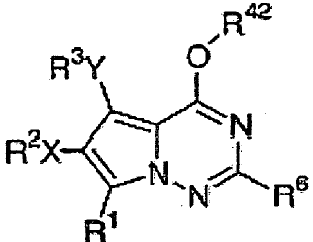 ), in WO 00/71129 (*e.g*., comprising the core structural formula: 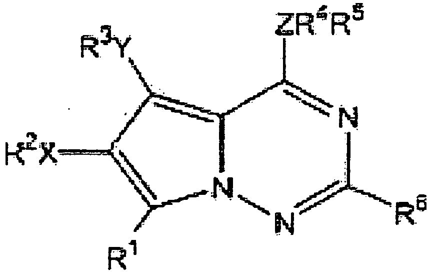 ),in WO2004/09601 (*e.g*., comprising the formula: 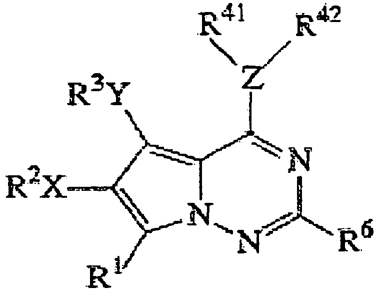 ), in WO2004/01059 (*e.g*., comprising the core structural formula: 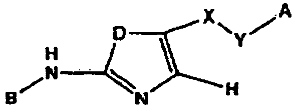 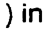 WO01/29025 (*e.g*., comprising the core structural formula: 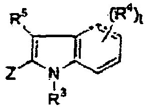 ), in WO02/32861 (*e.g*., comprising the core structural formula: 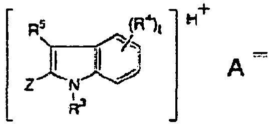 ) or set forth in WO03/88900 (*e.g*., comprising the core structural formula 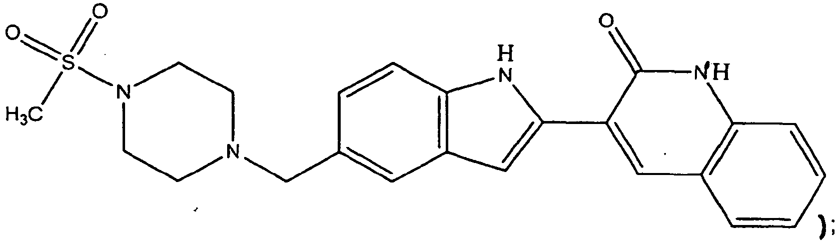 3-[5-(methylsulfonylpiperadinemethyl)-indolyl]-quinolone; Vatalanib 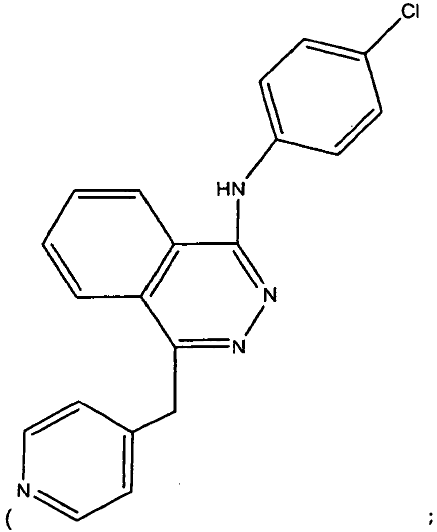 PTK/ZK; CPG-79787; ZK-222584), AG-013736 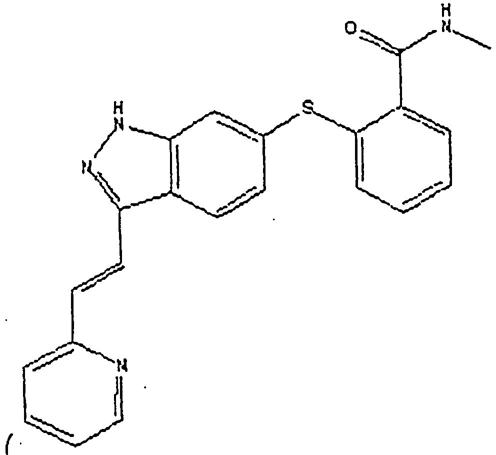 ); or the VEGF trap (AVE-0005), a soluble decoy receptor comprising portions of VEGF receptors 1 and 2.

The further chemotherapeutic agent is one or more of any LHRH (Lutenizing hormone-releasing hormone) agonist such as the acetate salt of [D-Ser(Bu t) 6 ,Azgly 10] (pyro-Glu-His-Trp-Ser-Tyr-D-Ser(Bu t)-Leu-Arg-Pro-Azgly-NH₂ acetate [C₅₉H₈₄N₁₈O₁₄·(C₂H₄O₂)ₓ where x = 1 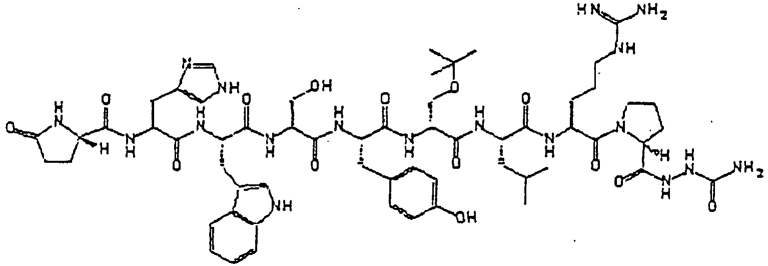 to 2.4]; (goserelin acetate; sold as Zoladex® by AstraZeneca UK Limited; Macclesfield, England), 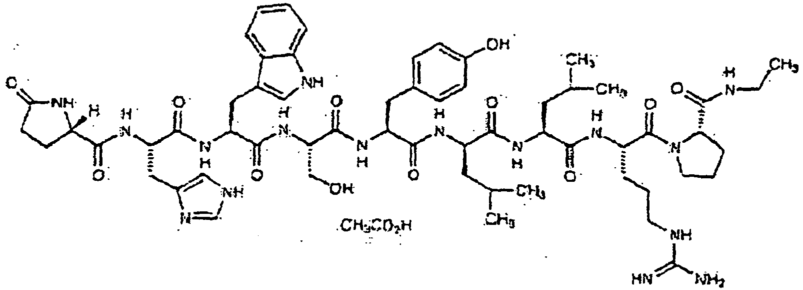 (leuprolide acetate; sold as Eligard® by Sanota-Synthelabo Inc.; New York, NY) or 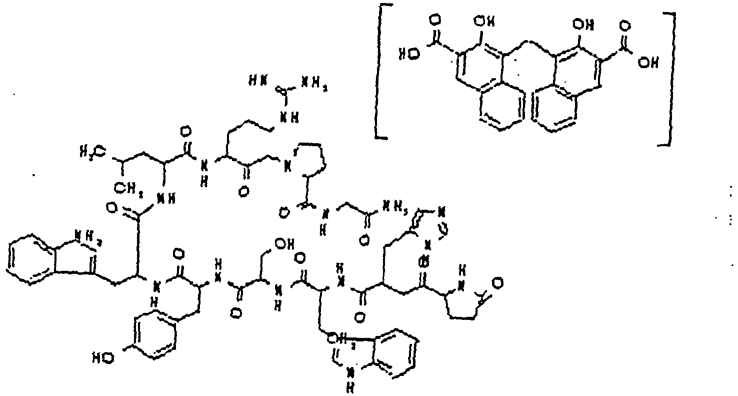 (triptorelin pamoate; sold as Trelstar® by Pharmacia Company, Kalamazoo, MI).

The further chemotherapeutic agent may be one or more of any progestational agent such as 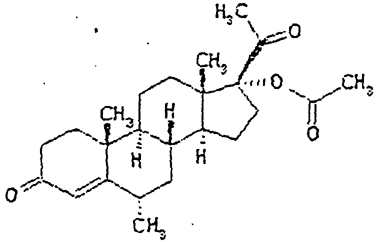 (medroxyprogesterone acetate; sold as Provera® by Pharmacia & Upjohn Co.; Kalamazoo, MI), 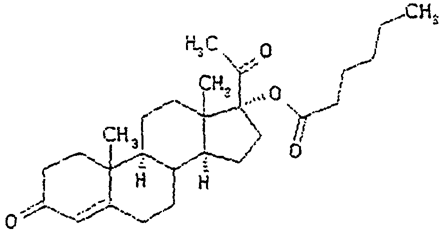 (hydroxyprogesterone caproate; 17-((1-Oxohexyl)oxy)pregn-4-ene-3,20-dione;), megestrol acetate or progestins.

The further chemotherapeutic agent may be one or more selective estrogen receptor modulators (SERM) such as 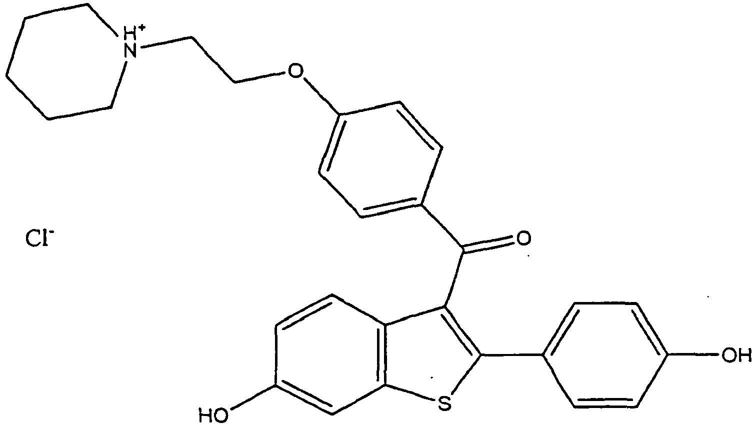 (raloxifene; sold as Evista® by Eli Lilly and Company; Indianapolis, IN).

The further chemotherapeutic agent may be one or more of any anti-androgen including, but not limited to: 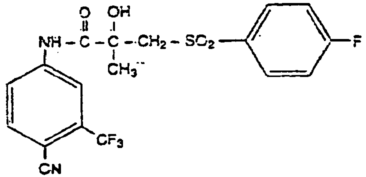 (bicalutamide; sold at CASODEX® by AstraZeneca Pharmaceuticals LP; Wilmington, DE); 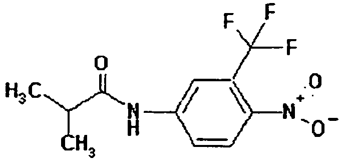 (flutamide; 2-methyl-N-[4-nitro-3 (trifluoromethyl) phenyl] propanamide; sold as Eulexin® by Schering Corporation; Kenilworth, NJ); 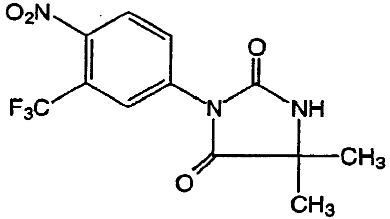 (nilutamide; sold as Nilandron® by Aventis Pharmaceuticals Inc.; Kansas City, MO) and 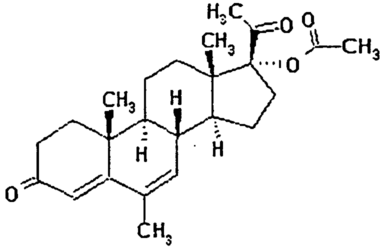 (Megestrol acetate; sold as Megace® by Bristol-Myers Squibb).

The further chemotherapeutic agent may be one or more of any EGF Receptor or HER2 antagonist, including, but not limited to, CP-724714 ( 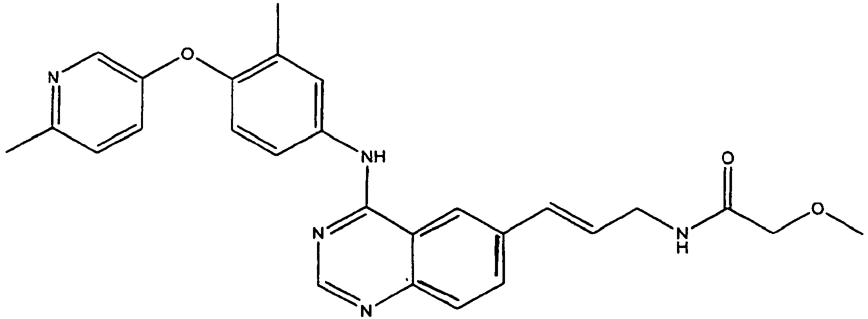 ); TAK-165 ( 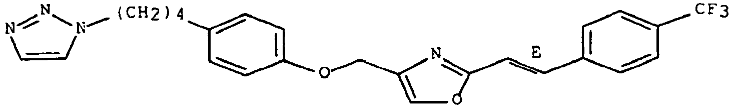 ); HKI-272 ( 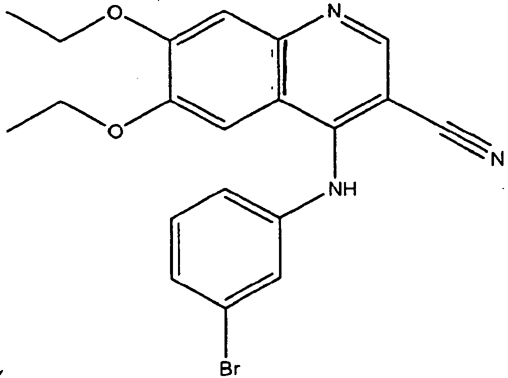 ); OSI-774 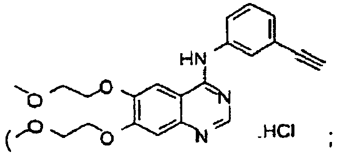 erlotinib, Hidalgo et al., J. Clin. Oncol. 19(13): 3267-3279 (2001)), Lapatanib ( 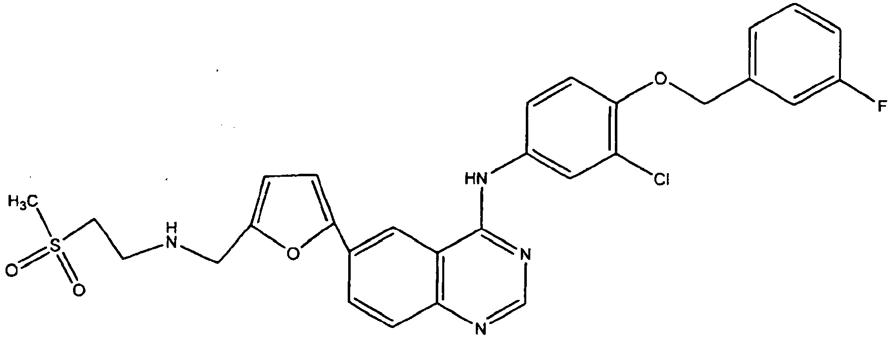 ; GW2016; Rusnak et al., Molecular Cancer Therapeutics 1:85-94 (2001); N-{3-Chloro-4-[(3-fluorobenzyl)oxy]phenyl}-6-[5-({[2-(methylsulfonyl)ethyl]amino}methyl)-2-furyl]-4-quinazolinamine; PCT Application No. WO99/35146), Canertinib (CI-1033; 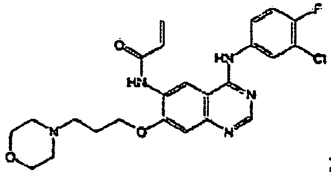 Erlichman et al., Cancer Res. 61(2):739-48 (2001 ); Smaill et al., J. Med. Chem. 43(7):1380-97 (2000)), ABX-EGF antibody (Abgenix, Inc.; Freemont, CA; Yang et al., Cancer Res. 59(6):1236-43 (1999); Yang et al., Crit Rev Oncol Hematol. 38(1):17-23 (2001)), erbitux (U.S. Patent No. 6,217,866; IMC-C225, cetuximab; Imclone; New York, NY), EKB-569 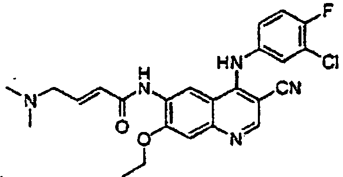 ( ; Wissner et al., J. Med. Chem. 46(1): 49-63 (2003)), PKI-166 ( 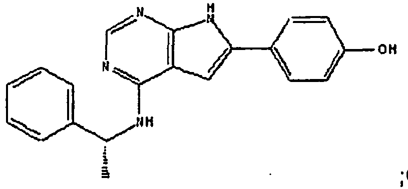 ;GP-75166), GW-572016, any anti-EGFR antibody and any anti-HER2 antibody.

The further chemotherapeutic agent may be one or more of any farnesyl protein transferase inhibitor including 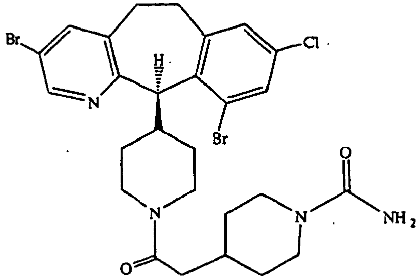 (Ionafarnib; Sarasar™; Schering-Plough; Kenilworth, NJ), 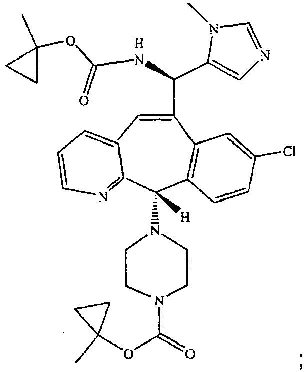 or 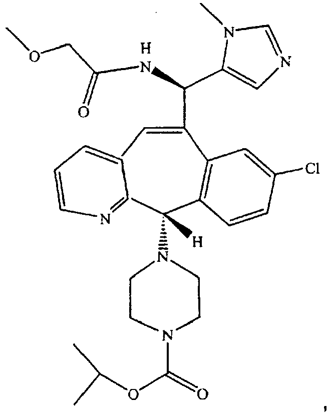 BMS-214662 ( 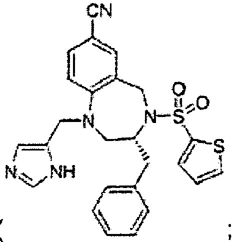 Hunt et al., J. Med. Chem. 43(20):3587-95 (2000 ); Dancey et al., Curr. Pharm. Des. 8:2259-2267 (2002); (R)-7-cyano-2,3,4,5-tetrahydro-1-(1H-imidazol-4-ylmethyl)-3-(phenylmethyl)-4-(2-thienylsulfonyl)-1H-1,4-benzodiazepine)) and R155777 (tipifarnib; Garner et al., Drug Metab. Dispos. 30(7):823-30 (2002); Dancey et al., Curr. Pharm. Des. 8:2259-2267 (2002); (B)-6-[amino(4-chlorophenyl)(1-methyl-1H-imidazol-5-yl)-methyl]-4-(3-chlorophenyl)-1-methyl-2(1H)-quinolinone]; 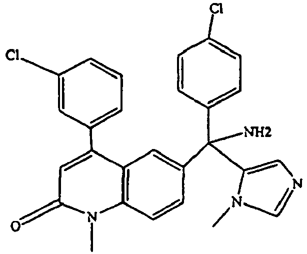 sold as Zarnestra™; Johnson & Johnson; New Brunswick, NJ).

The further chemotherapeutic agent may be one or more of any of 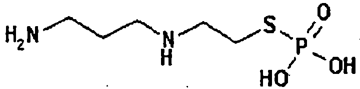 (Amifostine); 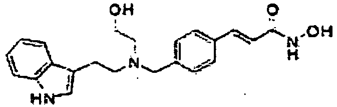 (NVP-LAQ824; Atadja et al., Cancer Research 64: 689-695 (2004)), 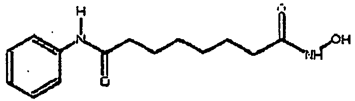 (suberoyl analide hydroxamic acid), 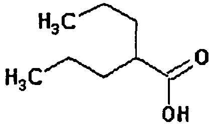 (Valproic acid; Michaelis et al., Mol. Pharmacol. 65:520-527 (2004)), 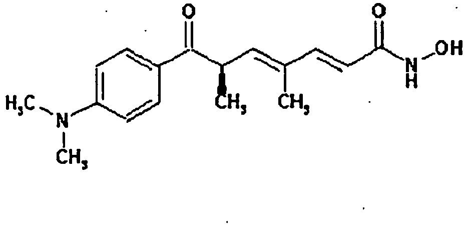 (trichostatin A), 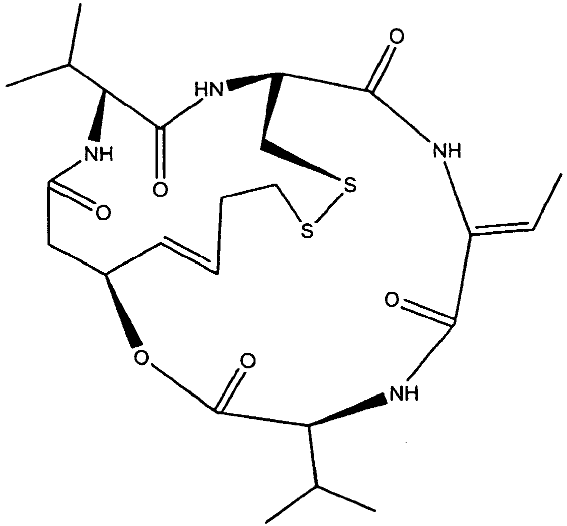 (FK-228; Furumai et al., Cancer Research 62: 4916-4921 (2002)), 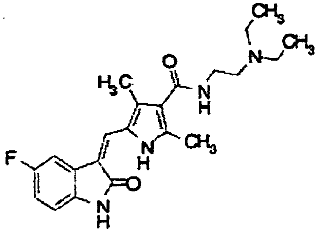 (SU11248; Mendel et al., Clin. Cancer Res. 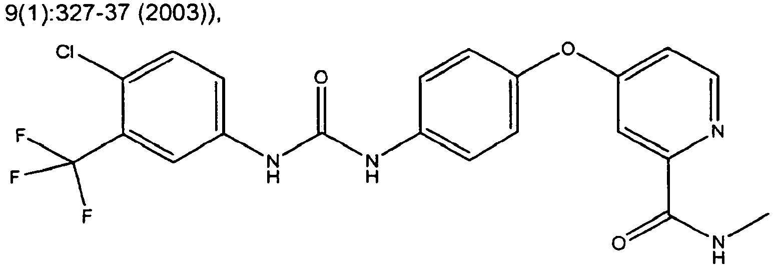 (BAY43-9006), 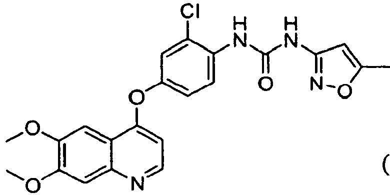 (KRN951), 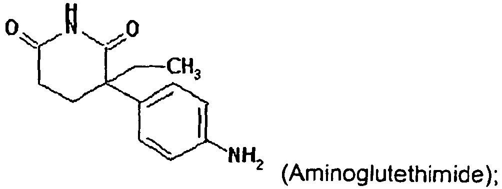 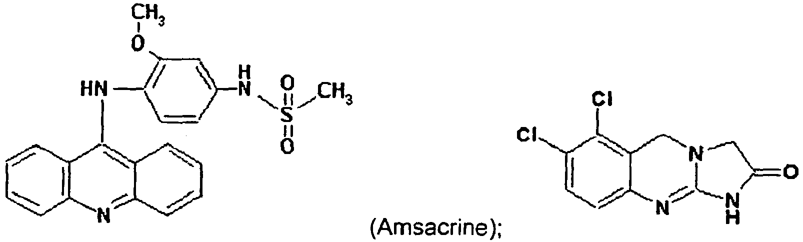 (Anagrelide); 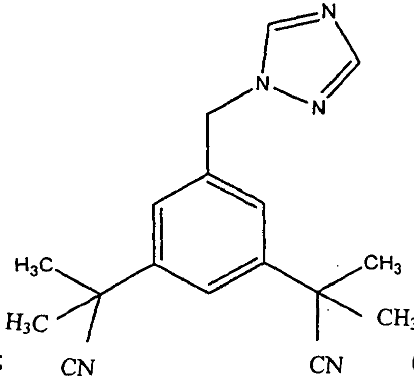 (Anastrozole; sold as Arimidex by AstraZeneca Pharmaceuticals LP; Wilmington, DE); Asparaginase; Bacillus Calmette-Guerin (BCG) vaccine (Garrido et al., Cytobios. 90(360):47-65 (1997)); 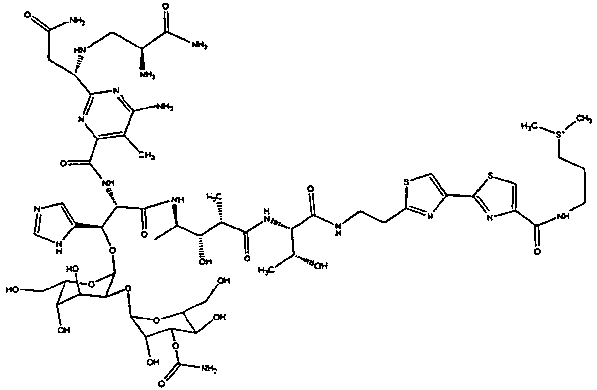 (Bleomycin); 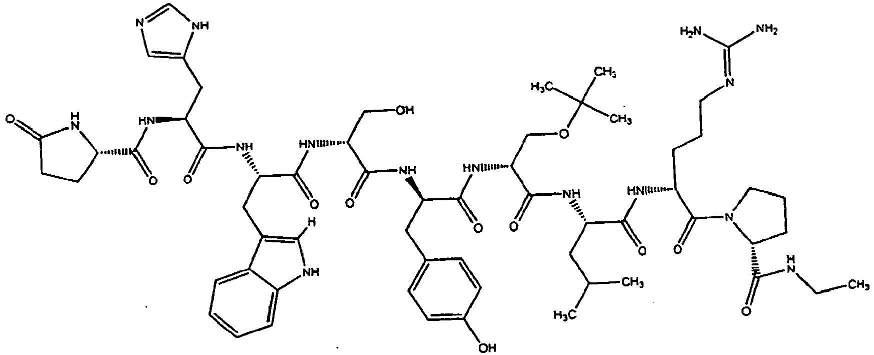 Buserelin); 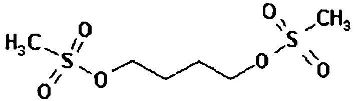 (Busulfan; 1,4-butanediol, dimethanesulfonate; sold as Busulfex® by ESP Pharma, Inc.; Edison, New 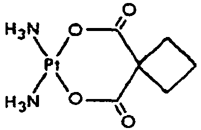 Jersey); (Carboplatin; sold as Paraplatin® by Bristol-Myers Squibb; Princeton, NJ); 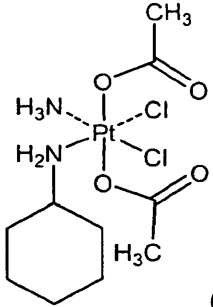 (satraplatin), 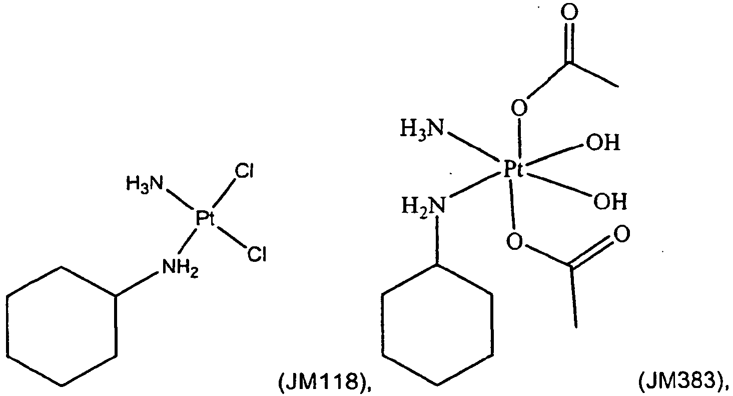 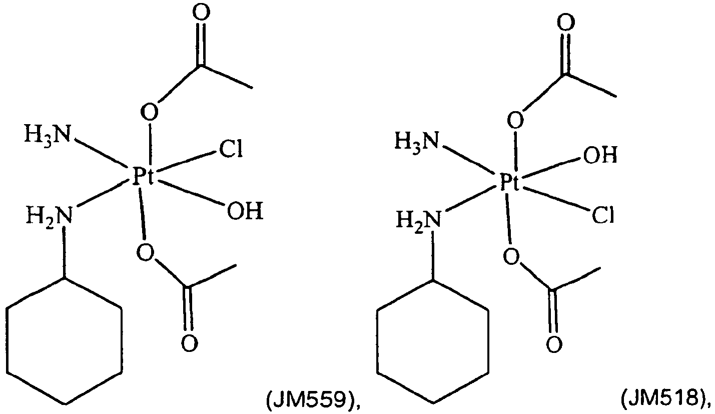 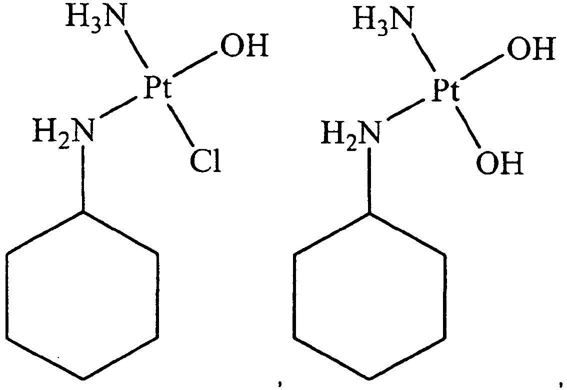 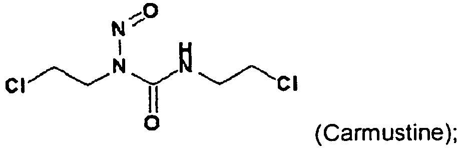 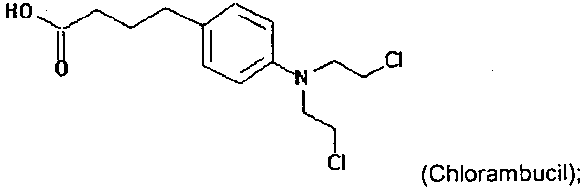 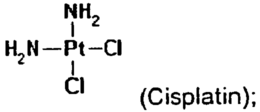 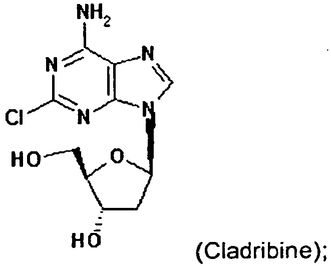 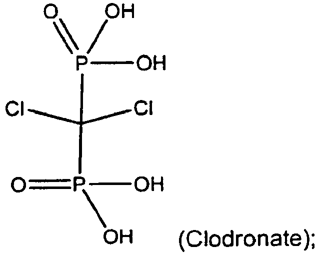 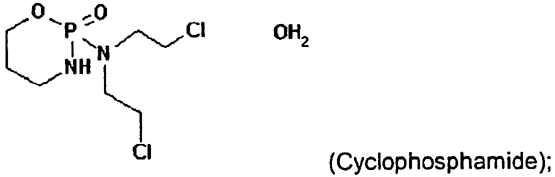 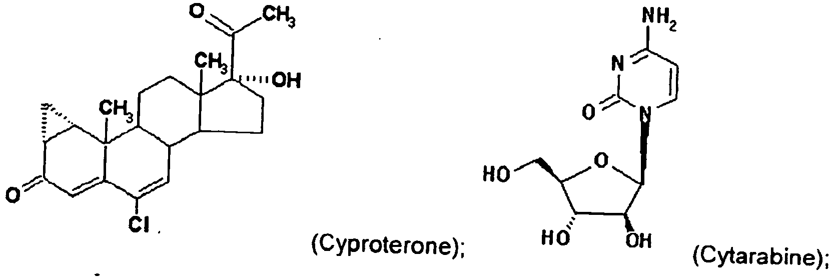 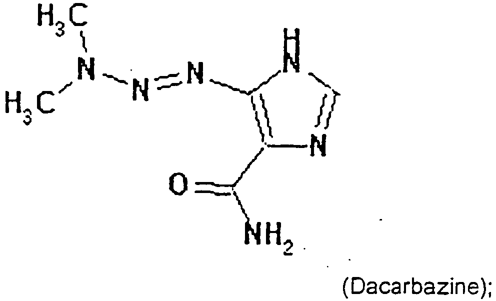 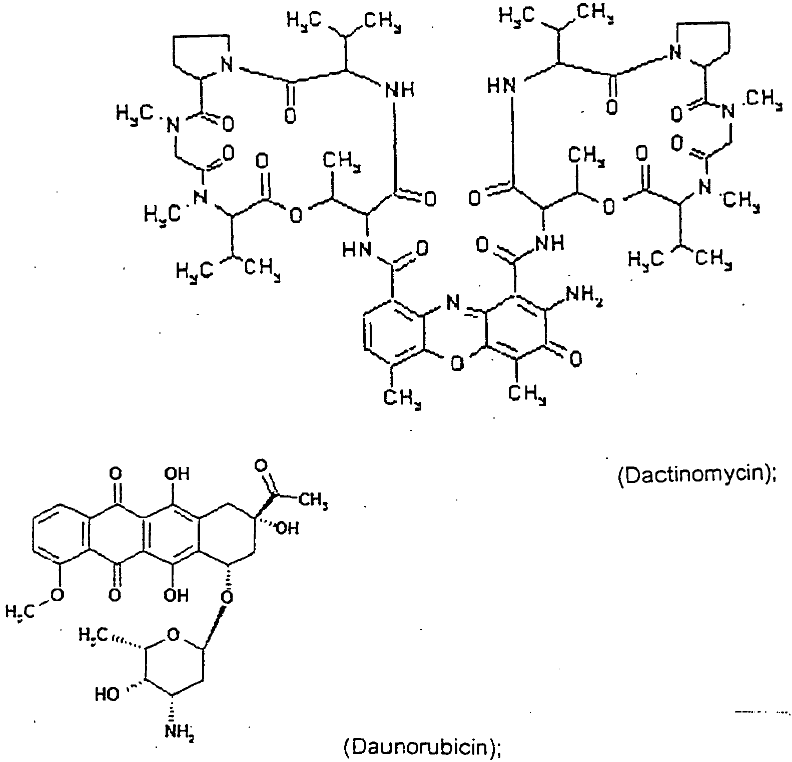 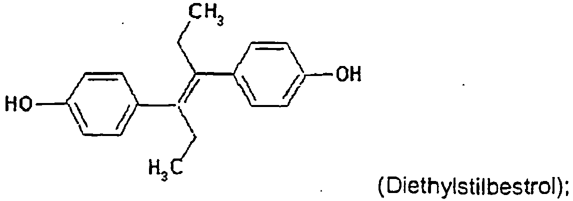 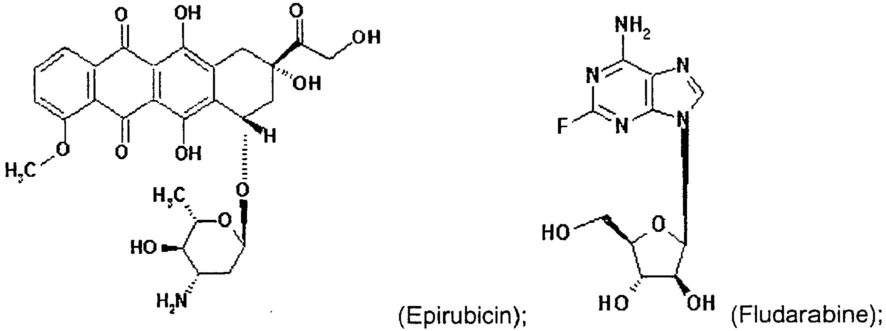 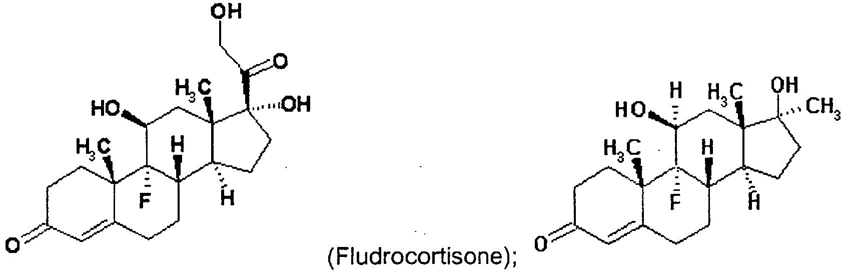 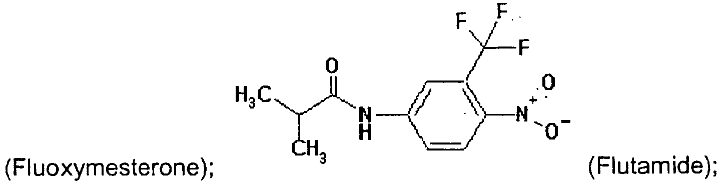 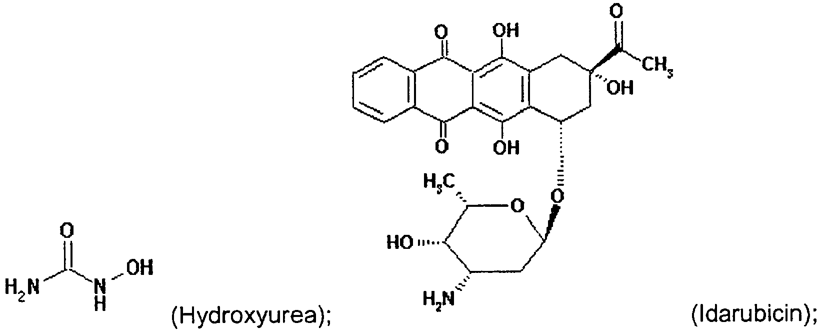 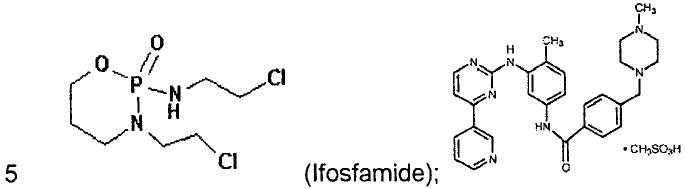 (Imatinib; sold as Gleevec® by Novartis Pharmaceuticals Corporation; East Hanover, NJ); 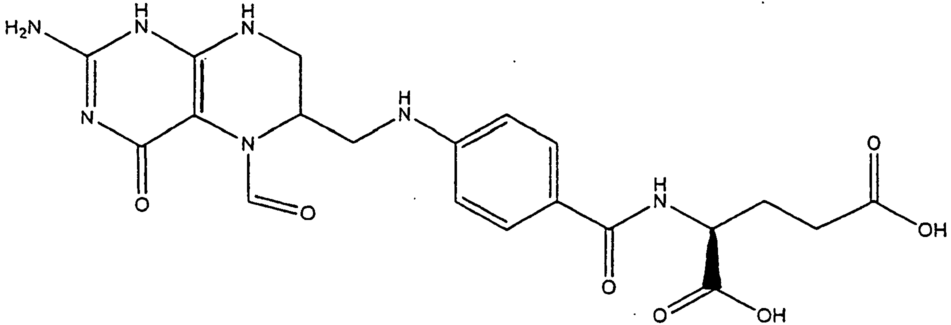 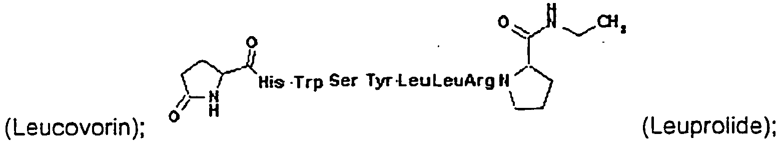 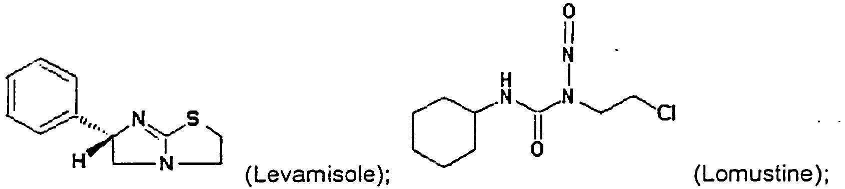 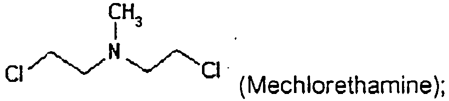 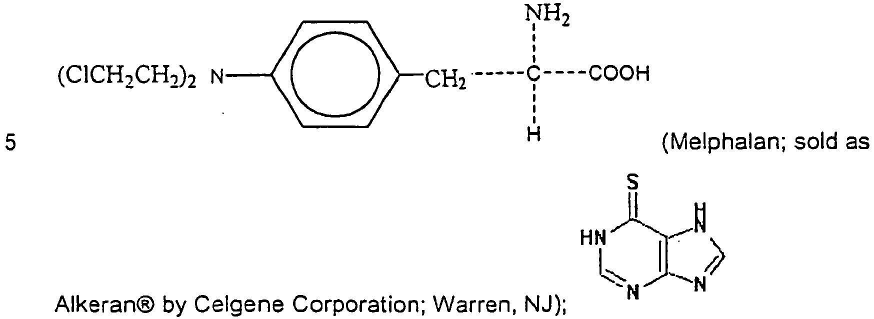 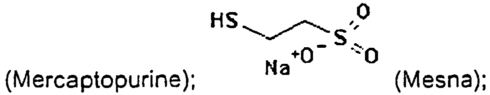 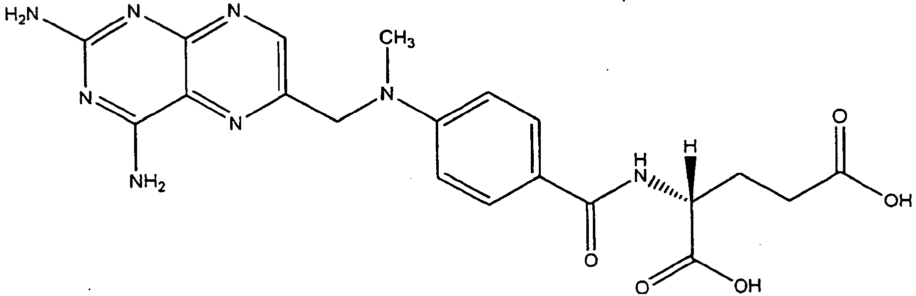 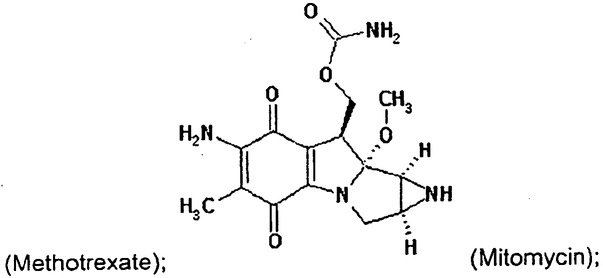 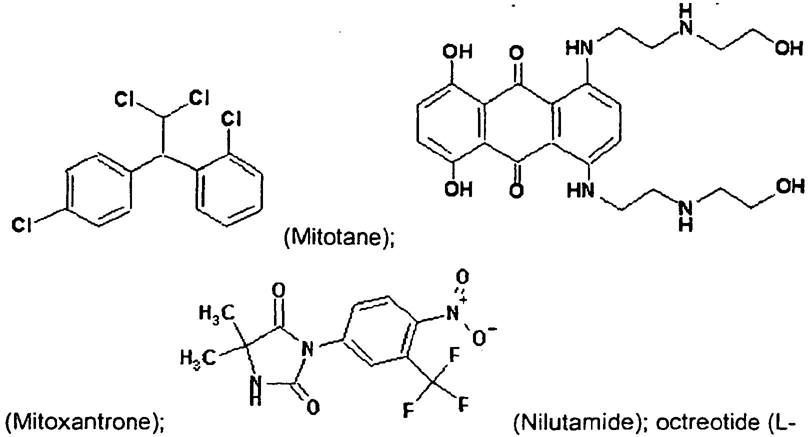 Cysteinamide, D-phenylalanyl- L-cysteinyl-L-phenylalanyl-D-tryptophyl-L-lysyl-L-threonyl-N-[2-hydroxy-1-(hydroxymethyl) propyl]-, cyclic (2_7)-disulfide; [R R*,R*)]; 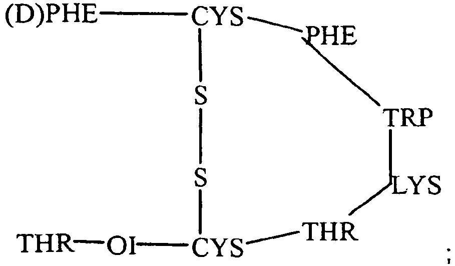 Katz et al., Clin Pharm. 8(4):255-73 (1989 ); sold as Sandostatin LAR® Depot; Novartis Pharm. Corp; E. Hanover, NJ); oxaliplatin ( 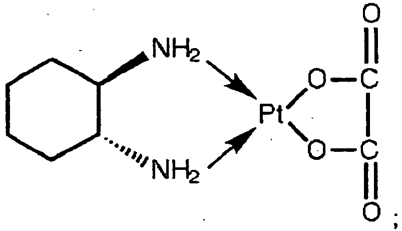 sold as Eloxatin™ by Sanofi-Synthelabo Inc.; New York, NY); 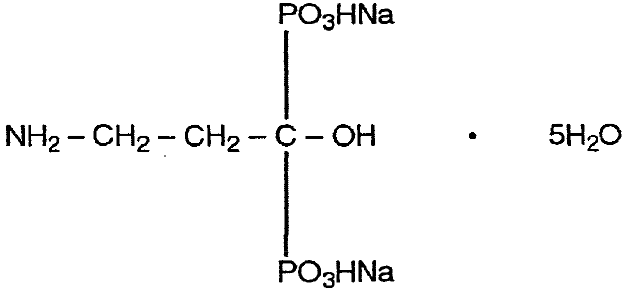 (Pamidronate; sold as Aredia® by Novartis Pharmaceuticals Corporation; East Hanover, NJ); 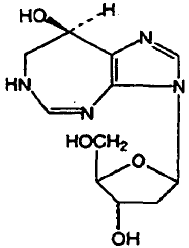 (Pentostatin; sold as Nipent® by Supergen; Dublin, CA); 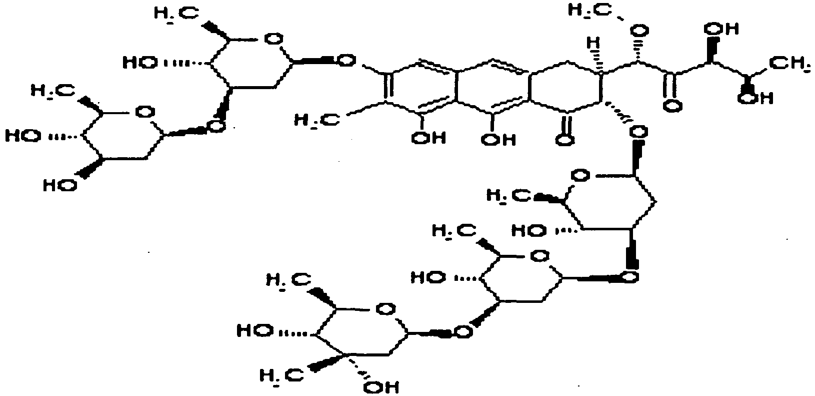 (Plicamycin); 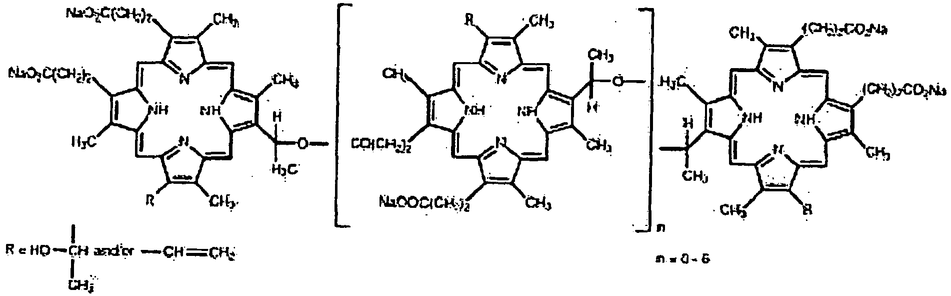 (Porfimer; sold as Photofrin® by Axcan Scandipharm Inc.; Birmingham, AL); 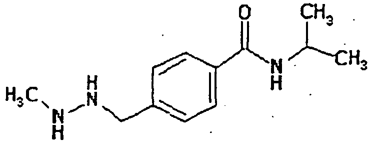 (Procarbazine); 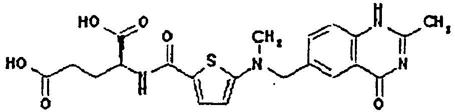 (Raltitrexed); Rituximab (sold as Rituxan® by Genentech, Inc.; South San Francisco, CA); 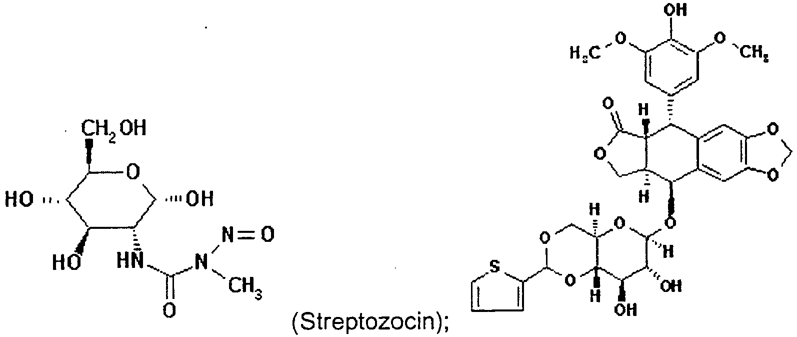 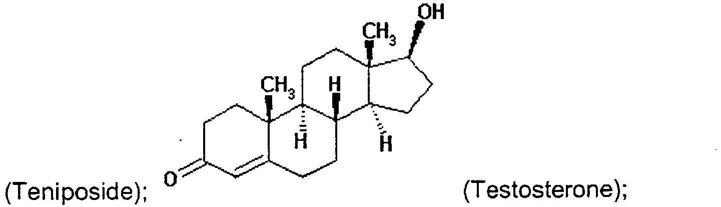 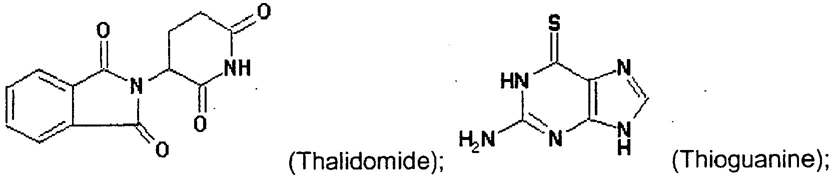 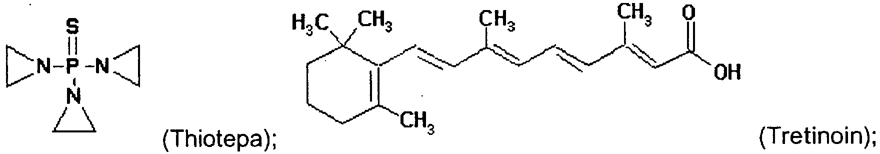 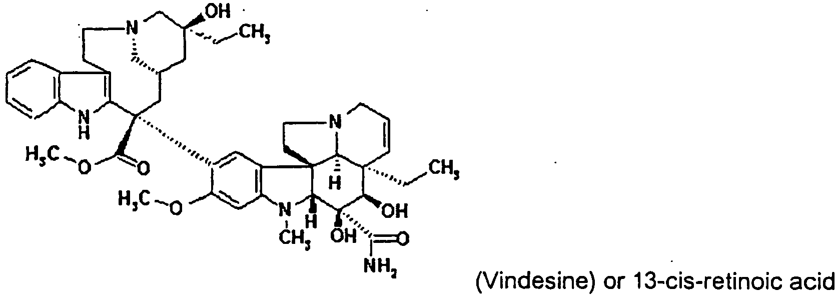 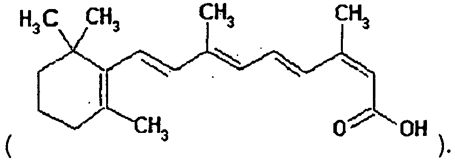 The further chemotherapeutic agent may be one or more of any of phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin, diftitox, gefitinib, bortezimib, paclitaxel, docetaxel, epithilone B, BMS-247550 (see *e.g*., Lee et al., Clin. Cancer Res. 7:1429-1437 (2001)), BMS-310705, droloxifene (3-hydroxytamoxifen), 4-hydroxytamoxifen, pipendoxifene, ERA-923, arzoxifene, fulvestrant, acolbifene, lasofoxifene (CP-336156), idoxifene, TSE-424, HMR-3339, ZK186619, topotecan, PTK787/ZK 222584 (Thomas et al., Semin Oncol. 30(3 Suppl 6):32-8 (2003)), the humanized anti-VEGF antibody Bevacizumab, VX-745 (Haddad, Curr Opin. Investig. Drugs 2(8):1070-6 (2001)), PD 184352 (Sebolt-Leopold, et al. Nature Med. 5: 810-816 (1999)), rapamycin, CCI-779 (Sehgal et al., Med. Res. Rev., 14:1-22 (1994); Elit, Curr. Opin. Investig. Drugs 3(8):1249-53 (2002)), LY294002, LY292223, LY292696, LY293684, LY293646 (Vlahos et al., J. Biol. Chem. 269(7): 5241-5248 (1994)), wortmannin, BAY-43-9006, (Wilhelm et al., Curr. Pharm. Des. 8:2255-2257 (2002)), ZM336372, L-779,450, any Raf inhibitor disclosed in Lowinger et al., Curr. Pharm Des. 8:2269-2278 (2002); flavopiridol (L86-8275/HMR 1275; Senderowicz, Oncogene 19(56): 6600-6606 (2000)) or UCN-01 (7-hydroxy staurosporine; Senderowicz, Oncogene 19(56): 6600-6606 (2000)).

The further chemotherapeutic agent may be , one or more of any of the compounds set forth in U.S. Patent 5,656,655, which discloses styryl substituted heteroaryl EGFR inhibitors; in U.S. Patent 5,646,153 which discloses bis mono and/or bicyclic aryl heteroaryl carbocyclic and heterocarbocyclic EGFR and PDGFR inhibitors; in U.S. Patent 5,679,683 which discloses tricyclic pyrimidine compounds that inhibit the EGFR; in U.S. Patent 5,616,582 which discloses quinazoline derivatives that have receptor tyrosine kinase inhibitory activity;in Fry et al., Science 265 1093-1095 (1994) which discloses a compound having a structure that inhibits EGFR (see Figure 1 of Fry *et al*.); in U.S. Patent 5,196,446 which discloses heteroarylethenediyl or heteroarylethenediylaryl compounds that inhibit EGFR; in Panek, et al., Journal of Pharmacology and Experimental Therapeutics 283: 1433-1444 (1997) which disclose a compound identified as PD166285 that inhibits the EGFR, PDGFR, and FGFR families of receptors-PD166285 is identified as 6- (2,6-dichlorophenyl)-2-(4-(2-diethylaminoethoxy)phenylamino)-8-methyl-8H-pyrido(2,3- d)pyrimidin-7-one.

The further chemotherapeutic agent may be one or more of any of pegylated or unpegylated interferon alfa-2a, pegylated or unpegylated interferon alfa-2b, pegylated or unpegylated interferon alfa-2c, pegylated or unpegylated interferon alfa n-1, pegylated or unpegylated interferon alfa n-3 and pegylated, unpegylated consensus interferon or albumin-interferon-alpha.

Disclosed are also methods of treatment comprising administering an IGF1R inhibitor in association with one or more antiemetics including, but not limited to, palonosetron (sold as Aloxi by MGI Pharma), aprepitant (sold as Emend by Merck and Co.; Rahway, NJ), diphenhydramine (sold as Benadryl® by Pfizer; New York, NY), hydroxyzine (sold as Atarax® by Pfizer; New York, NY), metoclopramide (sold as Reglan® by AH Robins Co,; Richmond, VA), Iorazepam (sold as Ativan® by Wyeth; Madison, NJ), alprazolam (sold as Xanax® by Pfizer; New York, NY), haloperidol (sold as Haldol® by Ortho-McNeil; Raritan, NJ), droperidol (Inapsine®), dronabinol (sold as Marinol® by Solvay Pharmaceuticals, Inc.; Marietta, GA), dexamethasone (sold as Decadron® by Merck and Co.; Rahway, NJ), methylprednisolone (sold as Medrol® by Pfizer; New York, NY), prochlorperazine (sold as Compazine® by GlaxoSmithKline; Research Triangle Park, NC), granisetron (sold as Kytril® by Hoffmann-La Roche Inc.; Nutley, NJ), ondansetron (sold as Zofran® by by Glaxosmithkline; Research Triangle Park, NC), dolasetron (sold as Anzemet® by Sanofi-Aventis; New York, NY), tropisetron (sold as Navoban® by Novartis; East Hanover, NJ).

Compositions comprising an antiemetic are useful for preventing or treating nausea; a common side effect of anti-cancer chemotherapy. Accordingly, disclosed are also methods for treating or preventing cancer in a subject by administering an IGF1R inhibitor optionally in association with one or more other chemotherapeutic agents (*e.g*., as described herein) and/or optionally in association with one or more antiemetics.

Disclosed are methods of treatment comprising administering an IGF1R inhibitor in association with a therapeutic procedure such as surgical tumorectomy or anti-cancer radiation treatment; optionally in association with a further chemotherapeutic agent and/or antiemetic, for example, as set forth above.

As discussed above, disclosed are methods wherein an IGF1R inhibitor is administered in association with a further anti-cancer chemotherapeutic agent or procedure. The term "in association with" indicates that the components of the combinations are to be formulated into a single composition for simultaneous delivery or formulated separately into two or more compositions (*e.g*., a kit). Furthermore, each component of a combination can be administered to a subject at a different time than when the other component is administered; for example, each administration may be given non-simultaneously (*e.g*., separately or sequentially) at several intervals over a given period of time. Moreover, the separate components may be administered to a subject by the same or by a different route (*e.g*., orally, intravenously, subcutaneously).

### Determination of IGFBP2 Levels

IGFBP2 levels may be measured by any of several methods which are very well known in the art; some of which are discussed *infra.*

IGFBP2 can be quantitated, for example, by simply hiring or contracting with a commercial laboratory to perform the assay. Alternatively, the practitioner can perform the assay himself. In an embodiment of the invention, IGFBP2 is quantitated by a radioimmunoassay (RIA) (see *e.g*., Smith et al., J. Clin. Endocrin. Metab. 77(5): 1294-1299 (1993); Cohen et al.., J. Clin. Endocrin. Metab. 76(4): 1031-1035 (1993); Dawczynski et al., Bone Marrow Transplant. 37:589-594 (2006); and Clemmons et al., J. Clin. Endocrin. Metab. 73:727-733 (1991)), western blot, western ligand blot (WLB) or by ELISA (enzyme linked immunosorbent assay). For example, in an embodiment of the invention, IGFBP2 in a sample of a patient's tumor tissue, plasma, blood or serum is quantitated.

In an embodiment of the invention, western ligand blots are performed as follows: Samples (2.5 µL) are electrophoresed on 10% polyacryl-amide-sodium dodecyl sulfate (SDS-PAGE) gels (*e.g*., 10, 12 or 14 %), electroblotted onto nitrocellulose, incubated with [¹²⁵I]-IGF-I, and exposed to film, *e.g*., for about 5-10 days. Each lane of the autoradiograph is developed, scanned and analyzed by densitometer.

In an embodiment of the invention, western blots are performed as follows: A sample is electrophoresed on a polyacrylamide-sodium dodecyl sulfate (SDS-PAGE) gel (*e.g*., 10, 12 or 14 %) and transferred onto nitrocellulose or some other suitable membrane. The membrane is then incubated with a primary antibody which binds to the protein being evaluated, optionally washed and then incubated with a detectably labeled secondary antibody that binds to the primary antibody and optionally washed again. The presence of the secondary antibody is then detected. For example, if the secondary antibody is labeled with a chemilluminescence label, the membrane is exposed to film and then the film is developed. In an embodiment of the invention, each lane of the autoradiograph is scanned and analyzed by densitometer.

In an embodiment of the invention, a RIA is performed as follows: IGFBP2 is iodinated by adding 0.5mCi [¹²⁵I]-sodium iodide to 0.1 ml, 0.5M phosphate buffer, pH7.5. Chloramines T (60 µM) is added and the mixture is incubated for 3 minutes. The percentage iodination is determined by adding an aliquot of the mixture to 1ml 10% bovine serum albumin followed by precipitation with an equal volume of ice cold 20% trichloroacetic acid. Additional chloramines-T is added when necessary to achieve 65% trichloroacetic acid precipitability and the reaction is terminated by the addition of sodium metabisulfite (final concentration 120 µM). The mixture is purified by Sepadex G-75 chromotagraphy in 0.01 M phosphate buffer pH7.5. Non-specific binding can be determined in the presence of 100 ng/ml pure human IGFBP2. [¹²⁵I]-IGFBP2 can be stored in siliconized tubes in 0.2 % BSA at -70°C. RIA can be performed by using 0.5 ml 0.03 M phosphate buffer, pH 7.4, containing 0.01M EDTA, 0.01 Tween-20 and 0.1 % bovine serum albumin. Test samples can be added by diluting the serum or plasma 1:10 then adding volumes of 10-40 µl and assays can, in an embodiment of the invention, be performed in duplicate. After incubation for 24 hours at 4°C, [¹²⁵I]-IGFBP2 (*e.g*., about 16000 cpm/tube) can be added and the incubation continued for another 16 hours. Four microliters of anti-IGFBP2 antiserum (*e.g*., rabbit antiserum) and a secondary antibody can be added and incubation can be continued for 1 hour at 4°C followed by 2 ml normal rabbit serum followed by a final 1 hour at 4°C. Bound and free [¹²⁵I]-IGFBP2 can be separated by centrifugation at 9000 X g for 30 minutes and bound [¹²⁵I]-IGFBP2 can be determined by γ-spectrometer. All unknown results can be read against a standard curve that contains *e.g*., 50 pg and 1 ng/tube of pure IGFBP2 (see Clemmons et al., J. Clin. Endocrin. Metab 73(4):727-733 (1991)).

Radioimmunoassays are based on the reaction between an antibody and an antigen whose concentration has to be quantified. A known quantity of radioactively labeled IGFBP2 is mixed with a dilution series of "cold" IGFBP2. The dilution series is brought to reaction with a fixed amount of anti-IGFBP2 antibody. Since cold and radioactively labeled IGFBP2 antigens compete with each other for the antibody binding sites, a high concentration of cold IGFBP2 will result in little radioactive IGFBP2 antigen bound to the antibody and vice versa. After a fixed time, a secondary antibody directed against the first anti-IGFBP2 antibody is used which leads to the formation of large complexes which upon centrifugation are counted with a radioactive counter. This fraction contains the "cold" and the radioactive antigen which has bound to the specific antibody, while the supernatant in the centrifugate contains the unbound antigen. The serially diluted probes yield points on a curve relating radioactive counts to the concentration of cold IGFBP2 antigen: a so-called (cold) reference curve. Using this reference curve, an unknown quantity of IGFBP2 antigen can be quantified by identification of the radioactive counts in the centrifugate and use of the reference curve which yields the unknown antigen concentration.

In an embodiment of the invention, an ELISA assay employs an antibody specific for human IGFBP2 coated on a 96-well plate. Standards and samples are pipetted into the wells and IGFBP2 present in a sample is bound to the wells by the immobilized antibody. The wells are washed and biotinylated anti-IGFBP2 antibody is added. After washing away unbound biotinylated antibody, HRP-conjugated streptavidin is pipetted to the wells. The wells are again washed, a TMB substrate solution is added to the wells and color develops in proportion to the amount of IGFBP2 bound. The Stop Solution changes the color from blue to yellow, and the intensity of the color is measured at 450 nm (see *e.g*., Human IGFBP-2 ELISA Kit from RayBiotech, Inc.; Norcross, GA; and Angervo M et al., Biochemical and Biophysical Research Communications 189: 1177-83 (1992); Kratz et al., Experimental Cell Research 202: 381-5 (1992); and Frost et al. Journal of Biological Chemistry 266: 18082-8 (1991)). A standard ELISA curve using known concentrations of IGFBP2 can be plotted and the concentration of IGFBP2 in the unknown sample (*e.g*., the serum of a patient) can be determined by comparing the signal observed therein with the signal observed in the standard.

Anti-IGFBP2 antibodies that can be used in an assay of the invention can be purchased commercially or easily generated by a practitioner using conventional methods known in the art. See *e.g*. Bourner et al., J. Cell. Biochem. 48:215-226 (1992) and Camacho-Hobner, C., et al., J. Biol. Chem 267:11949-11956 (1992) which describe the rabbit polyclonal anti-IGFBP2 antibody, ab4244 (Abcam, Inc.; Cambridge, MA). See *e.g*., Allander et al., Am. J. Pathology 161: 1587-1595 (2002) describing the goat anti-IGFBP2 polyclonal IgG, C-18 (Santa Cruz Biotechnology, Inc.; Santa Cruz, CA). See *e.g*., Suzuki, et al., J. Comp. Neurol. 482: 74-84 (2005); La et al. Endocrinology 145: 3443-3450 (2004); and Hoeflich et al., Biochem Biophys Res Commun. 324: 705-710 (2004) describing the anti-IGFBP2 goat polyclonal IgG, M-18 (Santa Cruz Biotechnology, Inc.; Santa Cruz, CA). See also, the anti-IGFBP2 rabbit polyclonal IgG, H-75 and the anti-IGFBP2 mouse monoclonal IgG1, C-10 (Santa Cruz Biotechnology, Inc.; Santa Cruz, CA).

### Diagnostics and Patient Selection

Disclosed is a method for diagnosing the presence of cancer or any other medical condition mediated by IGF1R expression or activity in a patient, for example, wherein the condition is cancer and the cancerous or tumor cells express IGF1R. The diagnostic method comprises determining if the patient exhibits elevated levels of IGFBP2. If the patient is determined to exhibit elevated IGFBP2, then the patient is determined to suffer from cancer or some other medical disorder mediated by IGF1R expression and/or activity.

The medical condition may be osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, autoimmune thyroiditis or Bechet's disease. In an embodiment of the invention, the diagnosis of cancer in the patient as set forth above is confirmed, *e.g*., using conventional techniques. For example, the presence of a tumor can be confirmed by X-ray, MRI, CT scan, PET scan, palpation, ultrasonography or surgery.

Diagnosis of the presence of cancer in a patient is followed by treatment with a therapeutically effective amount of an IGF1 R inhibitor or combination thereof with an anti-cancer therapeutic agent or anti-cancer procedure as set forth herein.

Normal or non-elevated levels of human IGFBP2 range from about 48-340 ng/ml (*e.g*., about 241 ng/ml ± about 28 ng/ml or ± about 10%; or about 150 ng/ml ± 61 ng/ml). In an embodiment of the invention, the human IGFBP2 level of a pediatric patient (*e.g*., about 2 months to about 1 year old) is about 263 ng/ml (in an embodiment ± 81 ng/ml). In an embodiment of the invention, the human IGFBP2 level of a pediatric patient (*e.g*., 15-18 years old) is about 136 ng/ml (in an embodiment ± 38 ng/ml).

In an embodiment of the invention, the normal IGFBP2 level is as determined by western ligand blots (WLB) or by radioimmunoassay (RIA). In an embodiment of the invention, IGFBP2 is measured in any bodily fluid of the patient, for example, blood, plasma, serum or tumor tissue.

In an embodiment of the invention, elevated or supranormal levels of IGFBP2 in a patient are any level that a practitioner of ordinary skill in the art would recognize as such. In an embodiment of the invention, an elevated or supranormal level of IGFBP2 which is over the range of 48-340 ng/ml or over about 241 ng/ml (*e.g*., as determined by WLB or RIA). In an embodiment of the invention, an elevated or supranormal level of IGFBP2 is at least about 50% to about 100% (*e.g*., 50%, 55%, 60%, 65%, 70%, 75%, 80%, 85%, 90%, 95%, 100%, 200%, 300%, 400% or 500%) higher than a normal level. In an embodiment of the invention, an elevated or supranormal level of IGFBP2 level is determined with respect to a particular patient. In such an embodiment a patient's IGFBP2 level is measured at an initial time point and measured at one or more points in the future. If one or more of the future measurements is higher than the initial measurement, the patient is determined to exhibit an elevated or supranormal IGFBP2 level.

The present invention further provides a method for selecting a patient suffering from a cancer likely to be responsive to an IGF1R inhibitor.

The cells of the cancerous tumor express IGF1R and the patient exhibits elevated IGFBP2 levels. If the patient is identified to possess a tumor that expresses IGF1 R or if the tumor is known to express IGF1 R and if the patient exhibits elevated or supranormal IGFBP2 levels or possesses a tumor known to be associated with elevated IGFBP2, then the patient is selected for treatment with an IGF1R inhibitor, *e.g*., as set forth herein.

### Dosage and method for monitoring and evaluating

### IGF1R inhibitor therapy

The present invention provides methods for quickly and conveniently evaluating various aspects of a given IGF1R inhibitor therapeutic regimen. For example, disclosed is a method for monitoring the effect of an IGF1R inhibitor on IGFBP2 concentration in the body of a subject administered said inhibitor comprising measuring IGFBP2 levels in the body of the subject over time. For example, in a more specific embodiment of the invention, an initial, baseline IGFBP2 level is measured before any dosage of IGF1R inhibitor is given. Following the commencement of an IGF1R treatment regimen, one or more measurements of IGFBP2 levels in the body of the subject (*e.g*., in the blood or plasma of the subject) are measured and compared.

For example, the present invention comprises a method for monitoring the effect of an IGF1R inhibitor (*e.g*., anti-IGF1R antibody) on the IGF1 receptor or any component of the IGF1R pathway in the body of a subject administered said inhibitor comprising evaluating IGFBP2 levels in the body of the subject over time; wherein the inhibitor is determined to inhibit the receptor or pathway if IGFBP2 levels are observed to decrease over time (*e.g*., by at least 51 %) following said administration; or wherein the inhibitor is determined not to inhibit the receptor or pathway if IGFBP2 levels are not observed to decrease over time (*e.g*., by at least 51%) following said administration. In an embodiment of the invention, an initial IGFBP2 level is measured before any dosage of IGF1R inhibitor is given. Following the commencement of an IGF1R inhibitor treatment regimen, one or more measurements of IGFBP2 levels in the body of the subject (*e.g.,* in the blood, serum or plasma of the subject) are measured and compared and the effect of the inhibitor on the receptor or pathway is then determined. In an embodiment of the invention, the level of IGFBP2 decrease or increase during the course of an IGF1R inhibitor regimen, is evaluated by a clinician in view of, *e.g*., the particularities of the subject's medical condition, status, sensitivities and history and weighed as one factor (*e.g*., among many) when deciding if the regimen is yielding an acceptable therapeutic effect. For example, in an embodiment of the invention, the IGFBP2 level is evaluated qualitatively for the purpose of gauging the sufficiency of the regimen.. When monitoring the effect of an IGF1R inhibitor on the IGF1 receptor is mentioned, this includes monitoring the effect of the inhibitor on the receptor itself as well as on any member of the IGF1R signaling pathway.

Disclosed is a method for evaluating dosage of an IGF1R inhibitor (*e.g*., the amount of the dosage and/or the frequency of the dosage and/or the mode of administration of the dosage) administered to a subject comprising administering a dose of said inhibitor to said subject and evaluating IGFBP2 levels in the body of the subject over time; wherein said dosage is determined to be insufficient if IGFBP2 levels are not observed to decrease (*e.g*., by at least 51 %) over time following said administration; or wherein said dosage is determined to be sufficient if IGFBP2 levels are observed to decrease over time (*e.g*., by at least 51 %) following said administration. In a more specific example, an initial baseline IGFBP2 level is measured before any dosage of IGF1R inhibitor is given. Following the commencement of an IGF1R treatment regimen, one or more measurements of IGFBP2 levels in the body of the subject (*e.g*., in the blood or plasma of the subject) are measured and compared and the sufficiency of the dosage is then determined. In an example, an IGF1R inhibitor dosage is adjusted up or down so that IGFBP2 levels, when elevated in a subject receiving the inhibitor, return to normal levels. Normal, low and elevated levels of IGFBP2 are known to any practitioner of ordinary skill in the art and are also discussed herein.

The present disclosure also includes a method for determining if a subject has a medical condition that is responsive to an IGF1R inhibitor comprising administering said inhibitor to said subject and evaluating IGFBP2 levels in the body of the subject over time; wherein said condition is determined to be unresponsive to said inhibitor if the IGFBP2 levels are not observed to decrease over time following said administration. If the subject proves to be essentially unresponsive to an IGF1R inhibitor, for example wherein IGFBP2 levels, and, thus, the IGF1R pathway itself does not decrease in response to the inhibitor, then the inhibitor therapy may be discontinued. Alternatively, the dosage can be increased so as to determine if the IGF1R pathway becomes responsive upon exposure to greater dosages. In a more specific example of the invention, an initial, baseline IGFBP2 level is measured before any dosage of IGF1R inhibitor is given. Following the commencement of an IGF1R treatment regimen, one or more measurements of IGFBP2 levels in the body of the subject (*e.g*., in the blood or plasma of the subject) are measured and compared and whether the medical condition in the subject is responsive or unresponsive is then determined.

The present disclosure also provides a method for selecting a dose of an IGF1R inhibitor comprising administering a dose of said inhibitor to a subject with a medical condition mediated by IGF1R expression or activity and evaluating IGFBP2 levels in the body of the subject; wherein said dosage is selected if IGFBP2 levels are observed to decrease by at least 51 % of an IGFBP2 level measured prior to first administration of said inhibitor following said administration. In an example, the method comprises (i) measuring an IGFBP2 level in the body of said subject before treatment with said inhibitor; (ii) administering one or more doses (*i.e*., doses of a single given amount such as 10 mg/kg given one or more times) of said inhibitor to said subject; (iii) measuring an IGFBP2 level in the body of said subject following said administration; (iv) comparing the level of IGFBP2 measured in step (i) with the level of IGFBP2 measured in step (iii); wherein said dose is selected if IGFBP2 levels are observed to decrease by at least 51 % of an IGFBP2 level measured prior to first administration of said inhibitor following said administration. For example, if the dosage is.selected, treatment of the subject at the selected dosage is continued.

Also disclosed is a method for treating a medical condition, in a subject, mediated by IGF1R expression or activity comprising (i) measuring an IGFBP2 level in the body of said subject prior to any administration of an IGF1R inhibitor; (ii) administering one or more doses of an IGF1R inhibitor to said subject; (iii) measuring an IGFBP2 level in the body of said subject following said administration; (iv) comparing the level of IGFBP2 measured in step (i) with the level of IGFBP2 measured in step (iii); and (v) increasing dosage of said inhibitor if the IGFBP2 level does not decrease by at least 51 % following said administration; or maintaining dosage if the IGFBP2 level does decrease by at least 51% following said administration.

The dosage of said inhibitor is determined to be insufficient or is not selected; or the inhibitor is determined to not inhibit IGF1R or its pathway; or the subject is not determined to be responsive to the IGF1R inhibitor if IGFBP2 levels are determined not to decrease by at least about 51 % of the initial, pre-treatment IGFBP2 level. Optionally, the dosage is increased if the IGFBP2 levels do not drop sufficiently. For example, the amount of dosage or the frequency of dosage may be increased if said dosage is determined to be insufficient. In an example, the initial dosage that is evaluated is between about 0.3 mg/kg and 20 mg/kg (*e.g*., 1 mg/kg, 3 mg/kg, 10 mg/kg), once a week.

If, for example, IGFBP2 levels do decrease by at least about 51 %, and subsequently increase above about 51 %, then dosage of the IGF1R inhibitor may be increased. If the increased dosage leads to a decrease in IGFBP2 levels to the originally set 51 % target, that increase dosage may then be selected or determined to be sufficient and maintained.

Dosage of an IGF1R inhibitor may be decreased if IGFBP2 levels decrease significantly more than 51%; for example, if a physician determines that the IGFBP2 levels have decreased to a dangerously low level.

In connection with any of the methods discussed herein, effects of IGF1 receptor inhibitors on the IGF1, receptor pathway are evaluated. The effects on the pathway include, but are not limited to, modulation of IGF1R kinase activity, Sos-1, Ras, Raf, Mek, Erk, PKA, PI3 kinase activity, Grb2 activity, AKT kinase activity or MAP kinase activity such that a reduction of cell (*e.g*., malignant cell) growth or survival or an increase in cellular apoptosis (*e.g*., of malignant cells) results wherein IGFBP2 levels are the marker for modulation of the pathway.

In an example an IGF1R inhibitor is administered to a patient at a "therapeutically effective dosage" or "therapeutically effective amount" which preferably inhibits a disease or condition (*e.g*., tumor growth) to any extent. As discussed herein, the proper dosage can be adjusted according to observations made by the clinician, physician or veterinarian.

The term "therapeutically effective amount" or "therapeutically effective dosage" means that amount or dosage of an IGF1R inhibitor (*e.g*., an anti-IGF1R antibody or antigen-binding fragment thereof) that will elicit a biological or medical response of a tissue, system, subject or host that is being sought by the administrator (such as a researcher, doctor or veterinarian) which includes survival of the subject (*e.g*., for 3 months, 6 months, 1 year, 2 years, 3 years, 4 years or 5 years after completing an IGF1R inhibitor regimen) and/or any measurable alleviation of the signs, symptoms and/or clinical indicia of cancer (*e.g*., tumor growth or survival) and/or the prevention, slowing or halting of progression or metastasis of cancer to any degree. Furthermore, an inhibitor or its dose is evaluated to determine if IGF1R is "sufficiently" inhibited; the effect sought through evaluation of IGFBP2 includes any of the biological or medical responses discussed above. One of ordinary skill in the art would be able to determine such amounts based on such factors as the subject's size, the severity of the subject's symptoms, and the particular composition or route of administration selected.

Administration of IGF1R inhibitor may be by injection proximal to the site of the target (*e.g*., tumor). In an embodiment, a therapeutically effective daily dose of IGF1R inhibitor or pharmaceutical composition thereof is administered as two, three, four, five, six or more sub-doses administered separately at appropriate intervals throughout the day. In an embodiment, a "therapeutically effective" dosage of any anti-IGF1R antibody (*e.g*., mature 19D12/15H12 LCF/HCA) is in the range of about 0.3 mg/kg (body weight) to about 20 mg/kg (*e.g*., 0.3 mg/kg, 1 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg or 20 mg/kg) about once per week to about once every 3 weeks (*e.g*., about once every 1 week or once every 2 weeks or once every 3 weeks). A "therapeutically effective dosage" of a chemotherapeutic agent (*e.g*., an IGF1R inhibitor) is, whenever possible, as set forth in the Physicians' Desk Reference 2003 (Thomson Healthcare; 57th edition (November 1, 2002)) which is herein incorporated by reference. For example, in an embodiment of the invention, a therapeutically effective dosage of NVP-ADW-742 is about 1 mg/kg/day to about 50 mg/kg/day (*e.g*., 5 mg/kg/day, 10 mg/kg/day, 15 mg/kg/day, 20 mg/kg/day, 25 mg/kg/day, 30 mg/kg/day, 35 mg/kg/day, 40 mg/kg/day, 45 mg/kg/day).

A physician or clinician can, optionally, also adjust the dosage of an IGF1R inhibitor using conventional techniques and clinical indicia in addition to IGFBP2 levels as discussed herein; such additional techniques and indicia are discussed below. For example, a clinician can evaluate the actual size and progress of the tumor being treated. The size and progress of a tumor can also be easily determined, for example, by X-ray, magnetic resonance imaging (MRI) or visually in a surgical procedure. In general, tumor size and proliferation can be measured by use of a thymidine PET scan (see *e.g*., Wells et al., Clin. Oncol. 8: 7-14 (1996)). Generally, the thymidine PET scan includes the injection of a radioactive tracer, such as [2-¹¹C]-thymidine, followed by a PET scan of the patient's body (Vander Borght et al., Gastroenterology 101: 794-799, 1991; Vander Borght et al., J. Radiat. Appl. Instrum. Part A, 42: 103-104 (1991)). Other tracers that can be used include [¹⁸F]-FDG (18-fluorodeoxyglucose), **[¹²⁴1]I**UdR (5-[124I]iodo-2'-deoxyuridine), [⁷⁶Br]BrdUrd (Bromodeoxyuridine), [¹⁸F]FLT (3'-deoxy-3'fluorothymidine) or [¹¹C]FMAU (2'-fluoro-5-methyl-1-ß-D-arabinofuranosyluracil).

For example, neuroblastoma progress can also be monitored, by a physician or veterinarian, by a variety of methods, and the dosing regimen can be altered accordingly. Methods by which to monitor neuroblastoma include, for example, CT scan (*e.g*., to monitor tumor size), MRI scan (*e.g*., to monitor tumor size), chest X-ray (*e.g*., to monitor tumor size), bone scan, bone marrow biopsy (*e.g*., to check for metastasis to the bone marrow), hormone tests (levels of hormones like epinephrine), complete blood test (CBC) (*e.g*., to test for anemia or other abnormality), testing for catecholamines (a neuroblastoma tumor marker) in the urine or blood, a 24 hour urine test for check for homovanillic acid (HMA) or vanillyl mandelic acid (VMA) levels (neuroblastoma markers) and an MIBG scan (scan for injected I¹²³-labeled metaiodobetaguanidine; *e.g*., to monitor adrenal tumors).

For example, rhabdomyosarcoma progress can also be monitored, by the physician or veterinarian, by a variety of methods, and the dosing regimen can be altered accordingly. Methods by which to monitor rhabdomyosarcoma include, for example tumor biopsy, CT scan (*e.g*., to monitor tumor size), MRI scan (*e.g*., to monitor tumor size), CT scan of the chest (*e.g*., to monitor metastases), bone scan (*e.g*., to monitor metastases), bone marrow biopsy (*e.g*., to monitor metastases), spinal tap (*e.g*., to check for metastasis into the brain) and a thorough physical exam.

For example, osteosarcoma progress can also be monitored, by the physician or veterinarian, by a variety of methods, and the dosing regimen can be altered accordingly. Methods by which to monitor osteosarcoma include, for example, X-ray of the affected area or of the chest (*e.g*., to check for spread to the lungs), CT scan of the affected area, blood tests (*e.g*., to measure alkaline phosphatase levels), computer tomography scan (CT) of the chest to see if the cancer has spread to the lungs, open biopsy, or a bone scan to see if the cancer has spread to other bones.

For example, Wilm's cancer progress can also be monitored, by the physician or veterinarian, by a variety of methods, and the dosing regimen can be altered accordingly. Methods by which to monitor Wilm's cancer include abdominal computer tomography scan (CT), abdominal ultrasound, blood and urine tests to evaluate kidney and liver function, chest X-ray to check for metastasis, magnetic resonance imaging (MRI), blood tests and urinalysis to assay kidney function and biopsy.

For example, pancreatic cancer progress can also be monitored, by the physician or veterinarian, by a variety of methods, and the dosing regimen can be altered accordingly. Methods by which to monitor pancreatic cancer include blood tests to check for tumor markers CA 19-9 and/or carcinoembryonic antigen (CEA), an upper GI series (*e.g*., a barium swallow), endoscopic ultrasonography; endoscopic retrograde cholangiopancreatography (an X-ray of the pancreatic duct and bile ducts); percutaneous transhepatic cholangiography (an X-ray of the bile duct), abdominal ultrasound imaging or abdominal computer tomography scan (CT).

For example, breast cancer progress can also be monitored, by the physician or veterinarian, by a variety of methods, and the dosing regimen can be altered accordingly. Methods by which to monitor breast cancer include mammography, aspiration or needle biopsy or palpation.

For example, colorectal cancer progress can also be monitored, by the physician or veterinarian, by a variety of methods, and the dosing regimen can be altered accordingly. Methods by which to monitor colorectal cancer include computer tomography scan (CT), MRI scan, chest X-ray, PET scan, fecal occult blood tests (FOBTs), flexible proctosigmoidoscopy, total colonoscopy, and barium enema.

For example, gastric cancer progress can also be monitored, by the physician or veterinarian, by a variety of methods, and the dosing regimen can be altered accordingly. Methods by which to monitor gastric cancer include esophagogastroduodenoscopy (EGD), double-contrast barium swallow, endoscopic biopsy, computed tomographic (CT) scanning, magnetic resonance imagine (MRI) or endoscopic ultrasonography (EUS).

For example, bladder cancer progress can also be monitored, by the physician or veterinarian, by a variety of methods, and the dosing regimen can be altered accordingly. Methods by which to monitor bladder cancer include urinalysis to detect elevated levels of tumor markers (*e.g*., nuclear matrix protein (NMP22)) in the urine, urinalysis to detect microscopic hematuria, urine cytology to detect cancer cells by examining cells flushed from the bladder during urination, bladder cystoscopy, intravenous pyelogram (IVP), retrograde pyelography, chest X ray to detect metastasis, computed tomography (CT), bone scan, MRI scan, PET scan or biopsy.

For example, lung cancer progress can also be monitored, by the physician or veterinarian, by a variety of methods, and the dosing regimen can be altered accordingly. Methods by which to monitor lung cancer include chest X-ray, CT scan, low-dose helical CT scan (or spiral CT scan), MRI scan, PET scan, bone scan, sputum cytology, bronchoscopy, mediastinoscopy, biopsy (*e.g*., needle or surgical), thoracentesis or blood tests to detect PTH (parathyroid hormone), CEA (carcinogenic antigen) or CYFRA21-1 (cytokeratin fragment 19).

For example, prostate cancer progress can also be monitored, by the physician or veterinarian, by a variety of methods, and the dosing regimen can be altered accordingly. Methods by which to monitor prostate cancer include digital rectal examination, transrectal ultrasound, blood tests taken to check the levels of prostate specific antigen (PSA) and prostatic acid phosphatase (PAP), biopsy, bone scan and CT scan.

For example, cervical cancer progress can also be monitored, by the physician or veterinarian, by a variety of methods, and the dosing regimen can be altered accordingly. Methods by which to monitor cervical cancer include PAP smear, pelvic exam, colposcopy, cone biopsy, endocervical curettage, X-ray, CT scan, cystoscopy and proctoscopy.

### Therapeutic Methods and Administration

An IGF1R inhibitor can be used to inhibit or reduce the growth or proliferation of any a malignant cell or treat a medical condition mediated by IGF1R. Such treatment or inhibition or reduction of growth or proliferation of a cell, in a subject's body, can be achieved by administering a therapeutically effective dosage of the IGF1 R inhibitor which is adjustable or alterable according to observations relating to IGFBP2 levels in the patient's body (*e.g.,* as discussed herein). Any tumor associated with or known to be associated with IGF1R expression and with elevated IGFBP2 levels, *e.g*., as per knowledge commonly held in the art, for example, as expressed in scientific literature, is suitable for treatment with an IGF1R inhibitor, *e.g*, as discussed herein.

IGFBP2 may serve as a marker for efficacy of an IGF1R inhibitor. An example includes a method for assessing whether an IGF1R inhibitor inhibits growth or survival of a tumor in a patient being treated for said tumor by being administered said IGF1 R inhibitor; or for assessing efficacy of said inhibitor in said patient comprising: - determining IGFBP2 levels in the patient over time; wherein said tumor growth or survival is determined to be inhibited or said inhibitor is determined to be efficacious if said IGFBP2 levels decrease or remain unchanged over time during said treatment and wherein said tumor growth or survival is determined not to be inhibited or said inhibitor is determined not to be efficacious if said IGFBP2 levels increase over time.

A cancer or other medical condition which is treatable with an IGF1R inhibitor using the methods of the present invention includes osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, autoimmune thyroiditis and Bechet's disease.

The term "patient" or "subject" includes any organism, preferably an animal, more preferably a mammal (*e.g*., rat, mouse, dog, cat, rabbit) and most preferably a human.

As stated above, in an embodiment of the invention, where possible, an IGF1R inhibitor is administered to a subject in accordance with the Physicians' Desk Reference 2003 (Thomson Healthcare; 57th edition (November 1, 2002)) or as set forth herein.

An IGF1R inhibitor can be administered by an invasive route such as by injection. Administration by a non-invasive route (*e.g*., orally; for example, in a pill, capsule or tablet) is also possible.

An anti-IGF1R antibody (*e.g*., 15H12/19D12 LCF/HCA), or pharmaceutical composition thereof, may be administered intravenously, subcutaneously, intramuscularly, intraarterially or intratumorally.

An IGF1R inhibitor can be administered with medical devices.known in the art. For example, a pharmaceutical composition of the invention can be administered by injection with a hypodermic needle.

The pharmaceutical compositions of the invention may also be administered with a needleless hypodermic injection device; such as the devices disclosed in U.S. Patent Nos. 6,620,135; 6,096,002; 5,399,163; 5,383,851; 5,312,335; 5,064,413; 4,941,880; 4,790,824 or 4,596,556.

Examples of well-known implants and modules for administering pharmaceutical compositions include: U.S. Patent No. 4,487,603, which discloses an implantable micro-infusion pump for dispensing medication at a controlled rate; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments. Many other such implants, delivery systems, and modules are well known to those skilled in the art.

The present invention relates to methods for treating medical conditions mediated by expression or activity of IGF1 R. Expression of IGF1 R by a patient's tumor cells can be determined using conventional techniques commonly held in the art. For example, IGF1R expression can be identified by western blot analysis (*e.g*., of biopsied tumor cells) using any of several anti-IGF1R antibodies which are commercially available (*e.g*., N-20, C-20 or H-60 from Santa Cruz Biotechnology; Santa Cruz, CA; alpha IR-3 from Oncogene Research/Calbiochem; San Diego, CA). Alternatively, certain cancers are simply known by practitioners of ordinary skill in the art to express IGF1R. Expression of IGFBP2 can be assayed *e.g*., as set forth above.

### Pharmaceutical Compositions

An IGF1R inhibitor may be incorporated into a pharmaceutical composition, along with a pharmaceutically acceptable carrier, suitable for administration to a subject *in vivo.* The scope of the present disclosure includes pharmaceutical compositions which are suitable to be administered to a subject by any route (parenteral or non-parenteral) including, for example, oral, ocular, topical, pulmonary (inhalation), intratumoral injection, intravenous injection, subcutaneous injection or intramuscular injection.

For general information concerning formulations, see, *e.g*., Gilman, et al., (eds.) (1990), The Pharmacological Bases of Therapeutics, 8th Ed., Pergamon Press; A. Gennaro (ed.), Remington's Pharmaceutical Sciences, 18th Edition, (1990), Mack Publishing Co., Easton, Pennsylvania.; Avis, et al., (eds.) (1993) Pharmaceutical Dosage Forms: Parenteral Medications Dekker, New York; Lieberman, et al., (eds.) (1990) Pharmaceutical Dosage Forms: Tablets Dekker, New York; and Lieberman, et al., (eds.) (1990), Pharmaceutical Dosage Forms: Disperse Systems Dekker, New York, Kenneth A. Walters (ed.) (2002) Dermatological and Transdermal Formulations (Drugs and the Pharmaceutical Sciences), Vol 119, Marcel Dekker.

If the IGF1R inhibitor is an anti-IGF1 R antibody (*e.g*., mature 15H12/19D12 LCB/HCB, LCC/HCB, LCF/HCA or LCD/HCA), the pharmaceutical composition comprises sodium acetate trihydrate (*e.g*., USP) at 2.30 g/l; glacial acetic acid (*e.g*.,USP/Ph. Eur.) at 0.18 g/l; sucrose extra pure (*e.g*., NF, Ph. Eur, BP) at 70.0 g/l; the antibody at any concentration such as 20.0 g/l and water for injection (*e.g*., USP/Ph. Eur); pH about 5.5. In an embodiment of the invention, the composition is lyophilized/dessicated (lacking the water component) and is reconstituted (by adding water) at a point prior to use.

Pharmaceutically acceptable carriers are conventional and very well known in the art. Examples include aqueous and nonaqueous carriers, stabilizers, antioxidants, solvents, dispersion media, coatings, antimicrobial agents, buffers, serum proteins, isotonic and absorption delaying agents, and the like that are physiologically compatible. The carrier may be suitable for injection into a subject's body.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Examples of pharmaceutically-acceptable antioxidants include: water soluble antioxidants such as ascorbic acid, cysteine hydrochloride, sodium bisulfate, sodium metabisulfite, sodium sulfite and the like; and oil-soluble antioxidants such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Prevention of the presence of microorganisms may be ensured both by sterilization procedures, and by the inclusion of various antimicrobial agents such as EDTA, EGTA, paraben, chlorobutanol, phenol sorbic acid, and the like.

Suitable buffers which may be included in the pharmaceutical compositions of the invention include L-histidine-based buffers, phosphate-based buffers (*e.g*., phosphate buffered saline, pH ≅ 7), sorbate-based buffers or glycine-based buffers.

Serum proteins which may be included in the pharmaceutical compositions of the invention may include human serum albumin.

Isotonic agents, such as sugars (*e.g*., sucrose), ethanol, polyalcohols (*e.g*., glycerol, propylene glycol, liquid polyethylene glycol, mannitol or sorbitol), sodium citrate or sodium chloride (*e.g*., buffered saline) may also be included in the pharmaceutical compositions of the invention. In an embodiment of the invention, the sugar, for example, glucose or sucrose is present at a high concentration (*e.g*., about 10-100 mg/ml, *e.g*., 50 mg/ml, 60 mg/ml or 70 mg/ml).

Prolonged absorption of an injectable pharmaceutical form may be brought about by the inclusion of agents which delay absorption such as aluminum monostearate and/or gelatin.

Dispersions can also be prepared in glycerol, liquid polyethylene glycols, and mixtures thereof and in oils.

Pharmaceutically acceptable carriers include sterile aqueous solutions or dispersions and sterile powders for the extemporaneous preparation of sterile injectable solutions or dispersions. The use of such media and agents for pharmaceutically active substances is well known in the art.

Sterile injectable solutions comprising an anti-IGF1R antibody can be prepared by incorporating the antibody or antigen-binding fragment thereof in the required amount in an appropriate solvent, optionally with one or a combination of ingredients enumerated above, as required, followed by sterilization microfiltration. Generally, dispersions are prepared by incorporating the antibody into a sterile vehicle that contains a basic dispersion medium and the required other ingredients from those enumerated above. In the case of sterile powders for the preparation of sterile injectable solutions, possible methods of preparation are vacuum drying and freeze-drying (lyophilization) that yield a powder of the active ingredient plus any additional, desired ingredients therein.

An anti-IGF1R antibody of the invention may be in a pharmaceutical formulation comprising a therapeutically effective amount of said antibody, a buffer and sucrose. For example, in an embodiment of the invention, the buffer is any one of phosphate buffer, citrate buffer, histidine buffer, glycine buffer or acetate buffer. The pharmaceutical formulation can be within any suitable pH range. In an embodiment of the invention, the pH is about 5.0, 5.5, 6.0, 7.5, or between about 5.5 and about 6 or between about 5 and about 7.

An IGF1R inhibitor including an anti-IGF1R antibody or antigen-binding fragment thereof can be orally administered. Pharmaceutical compositions for oral administration may contain, in addition to the antibody or antigen-binding fragment thereof, additives such as starch (*e.g*., potato, maize or wheat starch or cellulose), starch derivatives (*e.g*., microcrystalline cellulose or silica), sugars (*e.g*., lactose), talc, stearate, magnesium carbonate or calcium phosphate. In order to ensure that oral compositions comprising an antibody or antigen-binding fragment of the invention are well tolerated by the patient's digestive system, mucus formers or resins may be included. It may also be desirable to improve tolerance by formulating the antibody or antigen-binding fragment in a capsule which is insoluble in the gastric juices. An exemplary pharmaceutical composition of this invention in the form of a capsule is prepared by filling a standard two-piece hard gelatin capsule with the antibody or antigen-binding fragment of the invention in powdered form, lactose, talc and magnesium stearate. Oral administration of immunoglobulins has been described (Foster, et al., (2001) Cochrane Database System rev. 3:CD001816)

An IGF1 R inhibitor may also be administered by inhalation. A suitable pharmaceutical composition for inhalation may be an aerosol. An exemplary pharmaceutical composition for inhalation of an antibody or antigen-binding fragment of the invention can include: an aerosol container with a capacity of 15-20 ml comprising the antibody or antigen-binding fragment of the invention, a lubricating agent, such as polysorbate 85 or oleic acid, dispersed in a propellant, such as freon, preferably in a combination of 1,2-dichlorotetrafluoroethane and difluorochloromethane.

The composition could be in an appropriate aerosol container adapted for either intranasal or oral inhalation administration.

### Kits and Articles of Manufacture

Kits and articles of manufacture disclosed herein include an IGF1R inhibitor, combined, in an embodiment of the invention, with a pharmaceutically acceptable carrier, in a pharmaceutical formulation, for example in a pharmaceutical dosage form such as a pill, a powder, an injectable liquid or reconstitutable powder thereof, a tablet, dispersible granules, a capsule, a cachet or a suppository. See for example, Gilman et al. (eds.) (1990), The Pharmacological Bases of Therapeutics, 8th Ed., Pergamon Press; and Remington's Pharmaceutical Sciences, supra, Easton, Penn.; Avis et al. (eds.) (1993) Pharmaceutical Dosage Forms: Parenteral Medications Dekker, New York; Lieberman et al. (eds.) (1990) Pharmaceutical Dosage Forms: Tablets Dekker, New York; and Lieberman et al. (eds.) (1990), Pharmaceutical Dosage Forms: Disperse Systems Dekker, New York.

The kits and articles of manufacture also include information, for example in the form of a package insert or label, indicating that the target of the IGF1R inhibitory agent is IGF1R and that cancer patients (*e.g*., patients with a tumor expressing IGF1R) exhibiting elevated levels of IGFBP2 are likely to be responsive to an IGF1R inhibitor *e.g*, as discussed herein. In an embodiment of the invention, the label indicates that efficacy of the IGF1R inhibitor in a patient can be evaluated by monitoring IGFBP2 levels in the patient as set forth herein. Furthermore, the label indicates that dosage of the IGF1R inhibitor can be evaluated by the methods discussed herein or that the effect of the inhibitor on IGF1R or any member of the IGF1R pathway can be evaluated by the methods discussed herein.

The insert or label may take any form, such as paper or on electronic media such as a magnetically recorded medium (*e.g*., floppy disk) or a CD-ROM.

The label or insert may also include other information concerning the pharmaceutical compositions and dosage forms in the kit or article of manufacture. Generally, such information aids patients and physicians in using the enclosed pharmaceutical compositions and dosage forms effectively and safely. For example, the following information regarding the IGF1R inhibitory agent may be supplied in the insert: pharmacokinetics, pharmacodynamics, clinical studies, efficacy parameters, indications and usage, contraindications, warnings, precautions, adverse reactions, overdosage, proper dosage and administration, how supplied, proper storage conditions, references and patent information.

Disclosed is further a method for manufacturing an IGF1R inhibitor or a pharmaceutical composition thereof comprising a pharmaceutically acceptable carrier said method comprising combining, in a package, the inhibitor or composition; and a label conveying that the inhibitor or composition dosage or the inhibitor's or composition's inhibition of IGF1R or any member of the IGF1R pathway may be evaluated using any of the methods discussed herein.

### Examples

This section is intended to further describe the present invention and should not be construed to further limit the invention. Any composition or method set forth herein constitutes part of the present invention.

### Example 1: Treatment of anti-IGF1R Mab 19D12 decreased IGFBP2 level in xenograft tumors.

This example demonstrated that anti-IGF1R (comprising mature polypeptide Ig chains of the amino acid sequence of SEQ ID NOs: 8 and 10) decreased the level of IGFBP2 per microgram of total tumor protein in neuroblastoma tumor models.

Athymic nude mice were inoculated with SK-N-MC or SK-N-AS (human neuroblastoma) tumor cells in the right flank, subcutaneously, along with Matrigel (1:1 cells:gel). In these experiments, 5 x 10⁶ cells/mouse in a 1:1 mix with regular matrigel were inoculated subcutaneously. Tumor size was measured with calipers and the data was entered into the labcat program. Mice were grouped with an average tumor size of 100 mm³. Mice were dosed twice per week, intraperitoneally (i.p.) with antibody 19D12. Tumor size and mouse body weight was measured twice weekly after treatment.

Tumors were dissected out at the end of the studies, snap frozen, and stored at -80°C until analysis. Frozen xenograft tumor tissues were homogenized and lysed in buffer containing 50 mM Hepes, pH 7.4, 150 mM NaCl, 10% glycerol, 1% Triton X-100, 1.5 mM MgCl₂, 2 mM Na₃VO₄ and protease inhibitor cocktail (CompleteTM, Roche). Samples were spun for 13,000 rpm for 10 minutes at 4°C after incubation on ice for 30 minutes. Supernatants were collected and protein concentrations of the lysates were determined by Bio-Rad assay.

**Table 3: IGFBP2 level in SK-N-MC xenograft tumors**

| | **Average** | | |
|---|---|---|---|
| **Treatment (n=10)** | **IGFBP2 Level (pg/ug)*** | **Tumor volume (mm³)** | **SD** |
| IgG1 Control | 4.66 | 665 | 0.78 |
| 0.004 mg 19D12/IgG1 | 1.99 | 331 | 0.48 |
| 0.1 mg 19D12/IgG1 | 1.98 | 344 | 0.34 |
| 0.5 mg 19D12/IgG1 | 1.79 | 335 | 0.47 |

**Table 4: IGFBP2 level in SK-N-AS xenograft tumors**

| | **Average** | | |
|---|---|---|---|
| **Treatment (n=5)** | **IGFBP2 Level (pg/ug)** | **Tumor volume (mm³)** | **SD** |
| IgG1 Control | 4.57 | 1735 | 2.36 |
| 0.5 mg 19D12/IgG1 | 0.40 | 327 | 0.21 |

| | | | |
|---|---|---|---|
| picograms of IGFBP2 per microgram of total tumor protein. | | | |

The present invention relates to methods for evaluating an IGF1R inhibitor regimen (*e.g*., the dosage of the inhibitor), administered to a subject, by observing IGFBP2 levels in the subject. Decreasing IGFBP2 levels in the tumor correlate with inhibition of the IGF1R pathway as well as with inhibition of the downstream effects of the pathway, *e.g*., tumor growth. The decrease of IGFBP2 in the tumor tissue should reflect a decrease in IGFBP2 in the blood of the subject receiving the IGF1R inhibitor since IGFBP2 is a secreted protein. The data in this example support this point.

### Example 2: Treatment of anti-IGF1R Mab 19D12 decreased serum IGFBP2 level in monkeys

This example demonstrates that IGFBP2 levels drop in monkeys receiving an anti-IGF1R antibody.

***Dosing.*** Monkeys in group C1 were dosed with vehicle control (placebo) once weekly for 13 weeks starting at Day 0. Monkeys in group T1 were dosed with anti-IGF1R Mab 19D12 (comprising mature polypeptide Ig chains of the amino acid sequence of SEQ ID NOs: 8 and 10) once weekly at 10 mg/mg for 13 weeks starting at Day 0.

Blood samples were collected via the femoral artery/vein at indicated time points into a serum separator tube and centrifuged to obtain the serum. Serum samples were stored at -80°C until analysis.

***Measurement of IGFBP2.*** The R&D Duoset Human IGFBP2 ELISA Development System was chosen and the following protocol was used: Plates were coated with 100ul of 2ug/ml anti-Hu IGFBP2 overnight at 4°C. After each step, plates were rinsed 4 x 250 ul of wash buffer. 100ul of block buffer was added for 1 hour. And a rinse was performed. Standards and diluted samples were added and incubated 2 hours at room temperature on a shaker. A rinse was performed. 100ul of 100ng/ml of secondary anti-IGFBP2 antibody as the detection antibody was added for 2 hours with shaking. A rinse was performed. Added 100ul Streptavidin-HRP for 20 minutes with shaking. A rinse was performed. Added 100ul of a 1:1 mix of substrate solution for 20 minutes with shaking. Added 50ul of stop solution. Tapped plate to mix thoroughly. Read at 450 nM. The standard curve was reduced with a 4 parameter curve fit with SOFTmax Pro software.

The results of the foregoing experiments were as follows:

**Table 5. IGFBP2 levels observed in monkeys dosed with placebo or with anti-IGF1R antibody.**

| **Animal** | **SEX** | **Dose Group** | **Time Point** | **IGFBP2 (ng/ml)** |
|---|---|---|---|---|
| 101 | M | C1 | WEEK-5 | 151 |
| 101 | M | C1 | WEEK-3 | 153 |
| 101 | M | C1 | Day 1 | 169 |
| 101 | M | C1 | Day 4 | 154 |
| 101 | M | C1 | Day 7 | 148 |
| 101 | M | C1 | Day 91 | 176 |
| 102 | M | C1 | WEEK-5 | 151 |
| 102 | M | C1 | WEEK-3 | 170 |
| 102 | M | C1 | Day 1 | 195 |
| 102 | M | C1 | Day 4 | 154 |
| 102 | M | C1 | Day 7 | 187 |
| 102 | M | C1 | Day 91 | 158 |
| 103 | M | C1 | WEEK-5 | 74 |
| 103 | M | C1 | WEEK-3 | 90 |
| 103 | M | C1 | Day 1 | 89 |
| 103 | M | C1 | Day 4 | 88 |
| 103 | M | C1 | Day 7 | 96 |
| 103 | M | C1 | Day 91 | 116 |
| 104 | M | C1 | WEEK-5 | 165 |
| 104 | M | C1 | WEEK -3 | 199 |
| 104 | M | C1 | Day 1 | 184 |
| 104 | M | C1 | Day 4 | 170 |
| 104 | M | C1 | Day 7 | 168 |
| 104 | M | C1 | Day 91 | 191 |
| 105 | M | C1 | WEEK -5 | 77 |
| 105 | M | C1 | WEEK -3 | 90 |
| 105 | M | C1 | Day 1 | 84 |
| 105 | M | C1 | Day 4 | 95 |
| 105 | M | C1 | Day 7 | 76 |
| 105 | M | C1 | Day 91 | 89 |
| 106 | M | C1 | WEEK -5 | 120 |
| 106 | M | C1 | WEEK -3 | 142 |
| 106 | M | C1 | Day 1 | 135 |
| 106 | M | C1 | Day 4 | 114 |
| 106 | M | C1 | Day 7 | 128 |
| 106 | M | C1 | Day 91 | 148 |
| 501 | F | C1 | WEEK -5 | 93 |
| 501 | F | C1 | WEEK -3 | 119 |
| 501 | F | C1 | Day 1 | 154 |
| 501 | F | C1 | Day 4 | 131 |
| 501 | F | C1 | Day 7 | 117 |
| 501 | F | C1 | Day 91 | 145 |
| 502 | F | C1 | WEEK -5 | 134 |
| 502 | F | C1 | WEEK -3 | 211 |
| 502 | F | C1 | Day 1 | 205 |
| 502 | F | C1 | Day 4 | 161 |
| 502 | F | C1 | Day 7 | 174 |
| 502 | F | C1 | Day 91 | 227 |
| 503 | F | C1 | WEEK -5 | 130 |
| 503 | F | C1 | WEEK -3 | 158 |
| 503 | F | C1 | Day 1 | 132 |
| 503 | F | C1 | Day 4 | 154 |
| 503 | F | C1 | Day 7 | 136 |
| 503 | F | C1 | Day 91 | 150 |
| 504 | F | C1 | WEEK -5 | 75 |
| 504 | F | C1 | WEEK -3 | 66 |
| 504 | F | C1 | Day 1 | 65 |
| 504 | F | C1 | Day 4 | 62 |
| 504 | F | C1 | Day 7 | 65 |
| 504 | F | C1 | Day 91 | 56 |
| 505 | F | C1 | WEEK -5 | 64 |
| 505 | F | C1 | WEEK -3 | 68 |
| 505 | F | C1 | Day 1 | 87 |
| 505 | F | C1 | Day 4 | 70 |
| 505 | F | C1 | Day 7 | 69 |
| 505 | F | C1 | Day 91 | 66 |
| 506 | F | C1 | WEEK -5 | 85 |
| 506 | F | C1 | WEEK -3 | 86 |
| 506 | F | C1 | Day 1 | 93 |
| 506 | F | C1 | Day 4 | 81 |
| 506 | F | C1 | Day 7 | 79 |
| 506 | F | C1 | Day 91 | 78 |
| | | | | |
| 1001 | M | T1 | WEEK -5 | 166 |
| 1001 | M | T1 | WEEK -3 | 173 |
| 1001 | M | T1 | Day 1 | 169 |
| 1001 | M | T1 | Day 4 | 92 |
| 1001 | M | T1 | Day 7 | 72 |
| 1001 | M | T1 | Day 91 | 78 |
| 1002 | M | T1 | WEEK -5 | 147 |
| 1002 | M | T1 | WEEK -3 | 136 |
| 1002 | M | T1 | Day 1 | 146 |
| 1002 | M | T1 | Day 4 | 99 |
| 1002 | M | T1 | Day 7 | 86 |
| 1002 | M | T1 | Day 91 | 79 |
| 1003 | M | T1 | WEEK -5 | 121 |
| 1003 | M | T1 | WEEK -3 | 160 |
| 1003 | M | T1 | Day 1 | 114 |
| 1003 | M | T1 | Day 4 | 88 |
| 1003 | M | T1 | Day 7 | 69 |
| 1003 | M | T1 | Day 91 | 80 |
| 1004 | M | T1 | WEEK -5 | 156 |
| 1004 | M | T1 | WEEK -3 | 188 |
| 1004 | M | T1 | Day 1 | 184 |
| 1004 | M | T1 | Day 4 | 163 |
| 1004 | M | T1 | Day 7 | 158 |
| 1004 | M | T1 | Day 91 | 144 |
| 1005 | M | T1 | WEEK -5 | 141 |
| 1005 | M | T1 | WEEK -3 | 163 |
| 1005 | M | T1 | Day 1 | 175 |
| 1005 | M | T1 | Day 4 | 126 |
| 1005 | M | T1 | Day 7 | 121 |
| 1005 | M | T1 | Day 91 | 170 |
| 1006 | M | T1 | WEEK -5 | 110 |
| 1006 | M | T1 | WEEK -3 | 114 |
| 1006 | M | T1 | Day 1 | 113 |
| 1006 | M | T1 | Day 4 | 80 |
| 1006 | M | T1 | Day 7 | 80 |
| 1006 | M | T1 | Day 91 | 95 |
| 1501 | F | T1 | WEEK -5 | 124 |
| 1501 | F | T1 | WEEK -3 | 127 |
| 1501 | F | T1 | Day 1 | 138 |
| 1501 | F | T1 | Day 4 | 114 |
| 1501 | F | T1 | Day 7 | 94 |
| 1501 | F | T1 | Day 91 | 82 |
| 1502 | F | T1 | WEEK -5 | 141 |
| 1502 | F | T1 | WEEK -3 | 138 |
| 1502 | F | T1 | Day 1 | 145 |
| 1502 | F | T1 | Day 4 | 92 |
| 1502 | F | T1 | Day 7 | 72 |
| 1502 | F | T1 | Day 91 | 85 |
| 1503 | F | T1 | WEEK -5 | 281 |
| 1503 | F | T1 | WEEK -3 | 316 |
| 1503 | F | T1 | Day 1 | 253 |
| 1503 | F | T1 | Day 4 | 274 |
| 1503 | F | T1 | Day 7 | 296 |
| 1503 | F | T1 | Day 91 | 274 |
| 1504 | F | T1 | WEEK -5 | 100 |
| 1504 | F | T1 | WEEK -3 | 106 |
| 1504 | F | T1 | Day 1 | 89 |
| 1504 | F | T1 | Day 4 | 65 |
| 1504 | F | T1 | Day 7 | 62 |
| 1504 | F | T1 | Day 91 | 63 |
| 1505 | F | T1 | WEEK -5 | 70 |
| 1505 | F | T1 | WEEK -3 | 70 |
| 1505 | F | T1 | Day 1 | 60 |
| 1505 | F | T1 | Day 4 | 44 |
| 1505 | F | T1 | Day 7 | 43 |
| 1505 | F | T1 | Day 91 | 58 |
| 1506 | F | T1 | WEEK -5 | 96 |
| 1506 | F | T1 | WEEK -3 | 110 |
| 1506 | F | T1 | Day 1 | 107 |
| 1506 | F | T1 | Day 4 | 92 |
| 1506 | F | T1 | Day 7 | 70 |
| 1506 | F | T1 | Day 91 | 55 |

The C1 group received a placebo and the T1 group received the antibody.

### Example 3: Treatment of anti-IGF1R Mab 19D12 decreased serum IGFBP2 level in health human subjects

This example demonstrates that IGFBP2 levels decrease in response to IGF1R inhibition with an anti-IGF1 R antibody.

Following an initial sampling of blood for the determination of baseline, untreated IGFBP2 levels, health human subjects were given a single intravenous infusion of anti-IGF1R antibody (comprising mature polypeptide Ig chains of the amino acid sequence of SEQ ID NOs: 8 and 10) at 0.3 mg/kg, 1.0 mg/kg, 3.0 mg/kg, 10.0 mg/kg, and 20.0 mg/kg for 60 minutes. After treatment, blood was taken for analysis of IGFBP2 at day 1 (before the dose) and at 3, 6, 8, 10, 15 and 57 ("endpoint") days post-dose. On days 15, 16 and 17, all subjects were also injected with recombinant human IGF-1 (subcutaneously, BID). The data gathered from these experiments is set forth below in Table 6.

**Table 6. Summary of IGFBP2 levels observed in healthy human subjects administered the indicated doses of anti-IGF1R antibody.**

| **Dose Group*** | **Time Points Post Dose** | **IGFBP2(ng/ml)** | **SD** |
|---|---|---|---|
| 0.3 mg/kg | Baseline | 241.9 | 129.6 |
| | Day 3 | 169.4 | 83.5 |
| | Day6 | 153 | 103.9 |
| | Day8 | 157 | 127.9 |
| | Day10 | 200 | 172.5 |
| | Day15 | 292 | 177.5 |
| | Endpoint | 520 | 326.9 |
| | | | |
| 1.0 mg/kg | Baseline | 268 | 119.8 |
| | Day 3 | 180 | 97.7 |
| | Day6 | 154 | 126.3 |
| | Day8 | 125 | 85.8 |
| | Day10 | 170 | 170.6 |
| | Day15 | 180 | 147.9 |
| | Endpoint | 279 | 196.2 |
| | | | |
| 3.0 mg/kg | Baseline | 237 | 122.7 |
| | Day 3 | 154 | 58.9 |
| | Day6 | 119 | 54.3 |
| | Day8 | 116 | 55.5 |
| | Day10 | 99 | 50.0 |
| | Day15 | 109 | 41.9 |
| | Endpoint | 183 | 110.0 |
| | | | |
| 10 mg/kg | Baseline | 247 | 60.1 |
| | Day 3 | 156 | 51.2 |
| | Day6 | 123 | 53.2 |
| | Day8 | 129 | 49.4 |
| | Day10 | 118 | 68.7 |
| | Day15 | 136 | 89.9 |
| | Endpoint | 211 | 172.0 |
| 20 mg/kg | Baseline | 190 | 100.1 |
| | Day 3 | 129 | 45.2 |
| | Day6 | 84 | 30.6 |
| | Day8 | 83 | 32.2 |
| | Day10 | 99 | 38.6 |
| | Day15 | 97 | 18.9 |
| | Endpoint | 139 | 64.1 |

The IGFBP2 values are the mean levels observed in 8 subjects, in each dose group, which included both 6 subjects dosed with the antibody and 2 subjects dosed with a placebo.

Saturation of the IGF1 receptors in a subject receiving the anti-IGF1R antibody correlates with a reduction of IGFBP2 levels by at least 51 % of the baseline IGFBP2 level.

Furthermore, the present disclosure relates to the following items:
1. A method for monitoring the effect of an IGF1R inhibitor on IGF1R receptor in the body of a subject administered said inhibitor comprising evaluating IGFBP2 levels in the body of the subject over time.
2. The method of item 1 wherein the inhibitor is determined to inhibit the receptor if IGFBP2 levels are observed to decrease over time following said administration; or wherein the inhibitor is determined not to inhibit the receptor if IGFBP2 levels are not observed to decrease over time following said administration.
3. The method of item 1 wherein the subject suffers from a medical condition mediated by IGF1R activity or expression and wherein the inhibitor is determined to inhibit the receptor sufficiently if IGFBP2 levels are observed to decrease by at least 51% over time following a first administration of said inhibitor; or wherein the inhibitor is determined not to inhibit the receptor sufficiently if IGFBP2 levels are not observed to decrease by at least 51% over time following a first administration of said inhibitor.
4. The method of item 1 comprising:
   (i) measuring an IGFBP2 level in the body of said subject;
   (ii) administering one or more doses of said inhibitor to said subject;
   (iii) measuring an IGFBP2 level in the body of said subject following said administration;
   (iv) comparing the level of IGFBP2 measured in step (i) with the level of IGFBP2 measured in step (iii);
   wherein the inhibitor is determined to inhibit the receptor if IGFBP2 levels are observed to decrease over time following said administration; or wherein the inhibitor is determined not to inhibit the receptor if IGFBP2 levels are not observed to decrease over time following said administration.
5. The method of item 1 wherein the IGF1R inhibitor is an antibody or antigen-binding fragment thereof that binds specifically to IGF1R.
6. The method of item 5 wherein the antibody or fragment comprises one or more complementarity determining regions (CDRs) selected from the group consisting of:
   RASQSIGSSLH (SEQ ID NO: 99);
   YASQSLS (SEQ ID NO: 100);
   HQSSRLPHT (SEQ ID NO: 101);
   SFAMH (SEQ ID NO:102)
   GFTFSSFAMH (SEQ ID NO: 107);
   VIDTRGATYYADSVKG (SEQ ID NO: 103); and
   LGNFYYGMDV (SEQ ID NO: 104);
   or a mature fragment of a light chain immunoglobulin which comprises the amino acid sequence of SEQ ID NO: 2, 4, 6 or 8;
   or a mature fragment of a heavy chain immunoglobulin which comprises the amino acid sequence of SEQ ID NO: 10 or 12; or a pharmaceutical composition thereof which comprises a pharmaceutically acceptable carrier.
7. The method of item 1 wherein the subject suffers from a medical disorder mediated by IGF1R expression or activity.
8. The method of item 7 wherein the disorder is a member selected from the group consisting of: osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, auto-immune thyroiditis and Bechet's disease.
9. The method of item 1 wherein the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticiiimumab, ipilimumab, gossypol, Bio 111, 131-I-TM-601, ALT-110, BIO 140, CC 8490, cilengitide, gimatecan, IL 13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 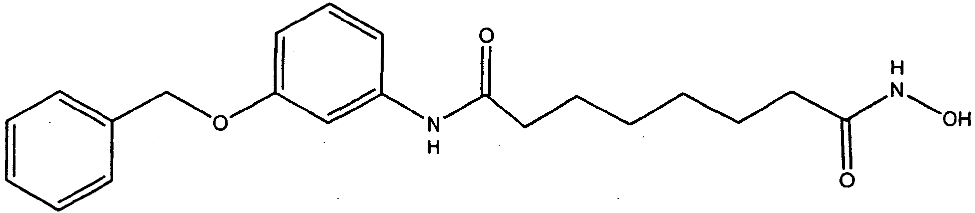 , CG-781, CG-1521 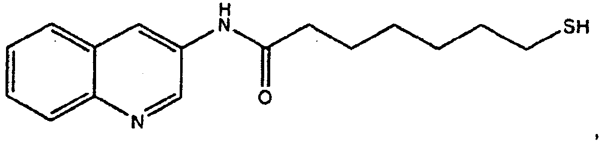 SB-556629, chlamydocin, JNJ-16241199, 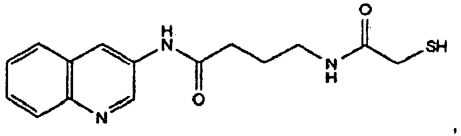 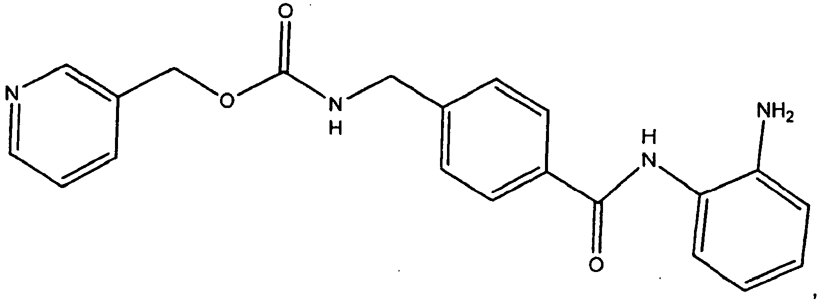 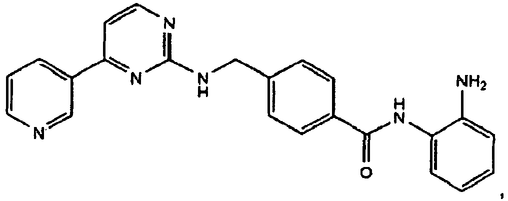 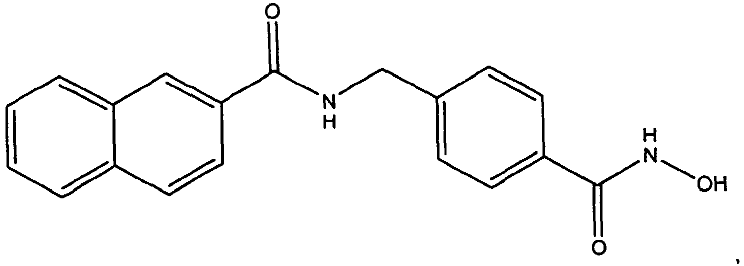 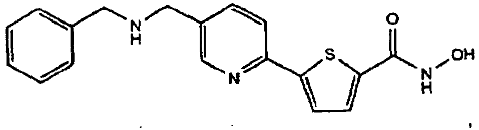 vorinostat, etoposide, gemcitabine, doxorubicin, liposomal doxorubicin, 5'-deoxy-5-fluorouridine, vincristine, temozoiomide, ZK-304709, seliciclib; PD0325901, AZD-6244, capecitabine, L-Glutamic acid, N-[4-[2-(2-amino-4,7-dihydro-4-oxo-1H - pyrrolo[2,3-d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 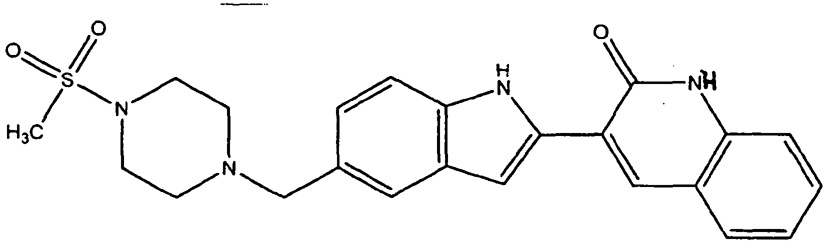 ); 3-[5-(methylsulfonylpiperadinemethyl)-indolyl]-quinolone, vatalanib, AG-013736, AVE-0005, the acetate salt of [D-Ser(Bu t) 6 ,Azgly 10](pyro-Glu-His-Trp-Ser-Tyr-D-Ser(Bu t)-Leu-Arg-Pro-Azgly-NH₂ acetate [C₅₉H₈₄N₁₈O₁₄ ·(C₂H₄O₂)ₓ where x = 1 to 2.4], goserelin acetate, leuprolide acetate, triptorelin pamoate, sunitinib, sunitinib malate, medroxyprogesterone acetate, hydroxyprogesterone caproate, megestrol, acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, lonafarnib, 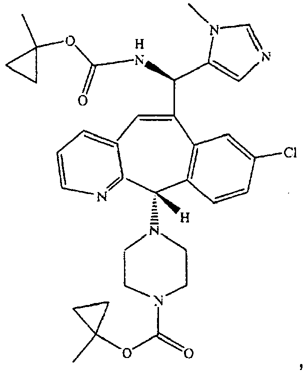 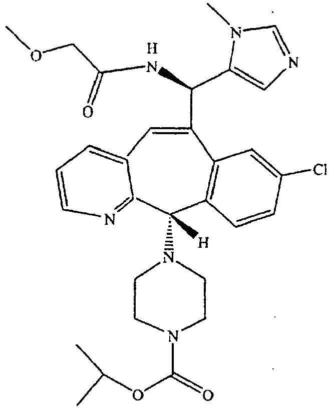 BMS-214662, tipifarnib; amifostine, NVP-CAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisoione, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, , diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.
10. The method of item 1 wherein the IGFBP2 level is determined using a radioimmunoassay (RIA), a western blot or an enzyme linked immunosorbent assay (ELISA) of a sample from the subject.
11. The method of item 1 wherein IGFBP2 is measured in blood, serum or plasma from the subject.
12. A method for evaluating dosage of an IGF1R inhibitor administered to a subject with a medical condition mediated by IGF1R expression or activity comprising administering a dose of said inhibitor to said subject and evaluating IGFBP2 levels in the body of the subject; wherein said dosage is determined to be insufficient if IGFBP2 levels are not observed to decrease by at least 51% of an IGFBP2 level measured prior to first administration of said inhibitor following said administration; or wherein said dosage is determined to be sufficient if IGFBP2 levels are observed to decrease by at least 51% of an IGFBP2 level measured prior to first administration of said inhibitor following said administration.
13. The method of item 12 comprising:
   (i) measuring an IGFBP2 level in the body of said subject before any treatment with said inhibitor;
   (ii) administering one or more doses of said inhibitor to said subject;
   (iii) measuring an IGFBP2 level in the body of said subject following said administration;
   (iv) comparing the level of IGFBP2 measured in step (i) with the level of IGFBP2 measured in step (iii);
   wherein said dosage is determined to be insufficient if IGFBP2 levels are not observed to decrease by at least 51 % over time following said administration; or wherein said dosage is determined to be sufficient if IGFBP2 levels are observed to decrease by at least 51 % over time following said administration.
14. The method of item 12 wherein the IGF1R inhibitor is an antibody or antigen-binding fragment thereof that binds specifically to IGF1R.
15. The method of item 14 wherein the antibody or fragment comprises one or more complementarity determining regions (CDRs) selected from the group consisting of:
   RASQSIGSSLH (SEQ ID NO: 99);
   YASQSLS (SEQ ID NO: 100);
   HQSSRLPHT (SEQ ID NO: 101);
   SFAMH (SEQ ID NO:102)
   GFTFSSFAMH (SEQ ID NO: 107);
   VIDTRGATYYADSVKG (SEQ ID NO: 103); and
   LGNFYYGMDV (SEQ ID NO: 104);
   or a mature fragment of a light chain immunoglobulin which comprises the amino acid sequence of SEQ ID NO: 2, 4, 6 or 8; or a mature fragment of a heavy chain immunoglobulin which comprises the amino acid sequence of SEQ ID NO: 10 or 12; or a pharmaceutical composition thereof which comprises a pharmaceutically acceptable carrier.
16. The method of item 15 wherein the antibody or fragment is a monoclonal antibody.
17. The method of item 12 wherein the disorder is a member selected from the group consisting of: osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, autoimmune thyroiditis and Bechet's disease.
18. The method of item 12 wherein the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 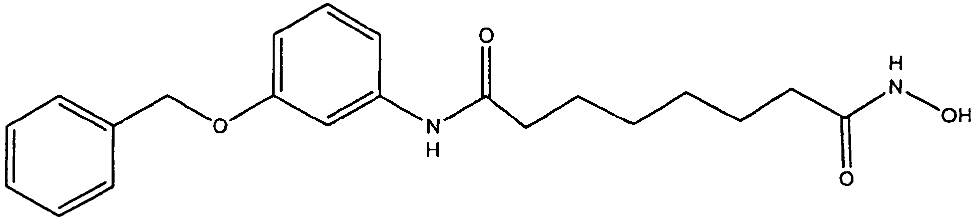 , CG-781, CG-1521, 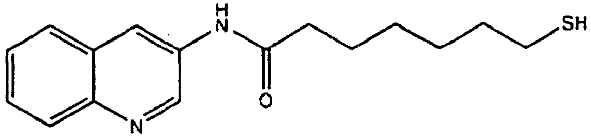 SB-556629, chlamydocin, JNJ-16241199, 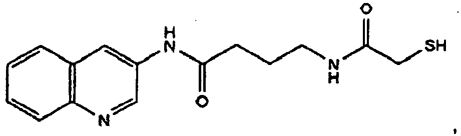 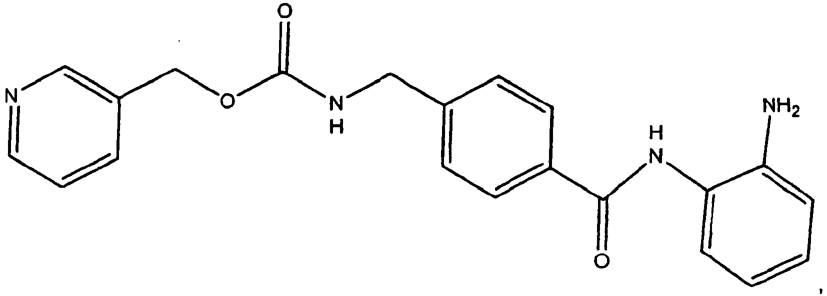 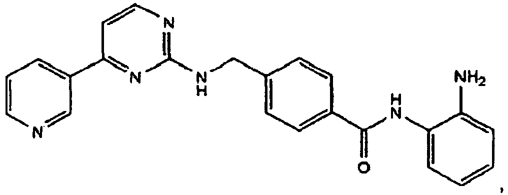 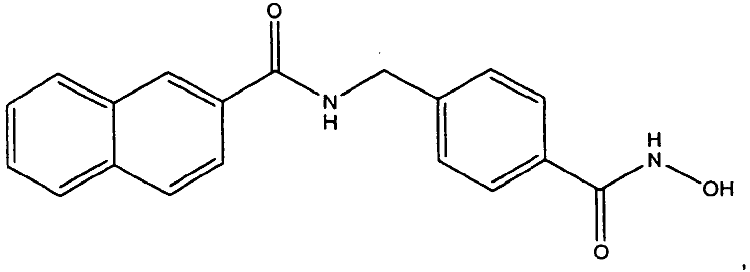 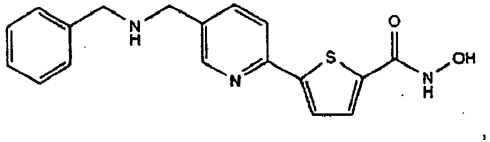 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N -[4-[2-(2-amino-4,7-dihydro-4-oxo-1 H - pyrrolo[2,3- d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 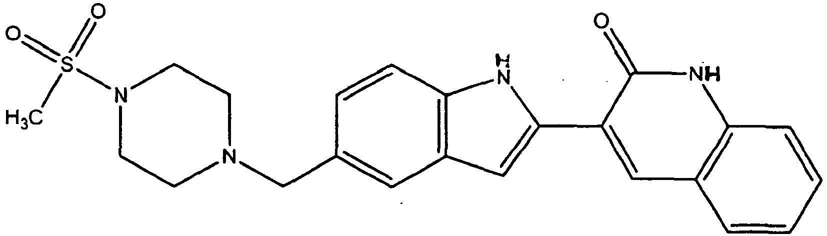 ); 3-[5-(methylsulfonylpiperadinemethyl)-indolyl]-quinolone, vatalanib, AG-013736, AVE-0005, the acetate salt of [D-Ser(Bu t) 6 ,Azgly 10] (pyro-Glu-His-Trp-Ser-Tyr-D-Ser(Bu t )-Leu-Arg-Pro-Azgly-NH₂ acetate [C₅₉H₈₄N₁₈O₁₄ ·(C₂H₄O₂)ₓ were x = 1 to 2.4], goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, Ionafarnib, 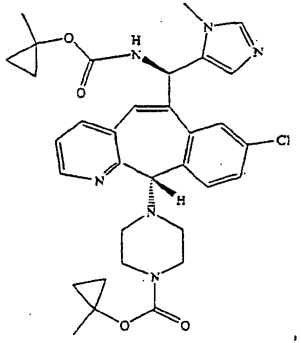 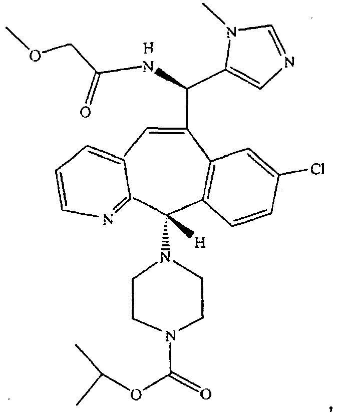 BMS-214662, tipifarnib; amifostine, NVP-LAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesria, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, , diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.
19. The method of item 12 wherein the IGFBP2 level is determined using a radioimmunoassay (RIA), a western blot or an enzyme linked immunosorbent assay (ELISA) of a sample from the subject.
20. The method of item 12 wherein IGFBP2 is measured in blood, serum or plasma from the subject.
21. A method for determining if a subject has a medical condition that is responsive to an IGF1R inhibitor comprising administering said inhibitor to said subject and evaluating IGFBP2 levels in the body of the subject over time; wherein said condition is determined to be unresponsive to said inhibitor if the IGFBP2 levels are not observed to decrease over time following said administration; or wherein the condition is determined to be responsive to said inhibitor if the IGFBP2 levels are observed to decrease over time following said administration.
22. The method of item 21 comprising:
   (i) measuring an IGFBP2 level in the body of said subject;
   (ii) administering one or more doses of said inhibitor to said subject;
   (iii) measuring an IGFBP2 level in the body of said subject following said administration;
   (iv) comparing the level of IGFBP2 measured in step (i) with the level of IGFBP2 measured in step (iii); wherein said condition is determined to be unresponsive to said inhibitor if the IGFBP2 levels are not observed to decrease over time following said administration; or wherein the condition is determined to be responsive to said inhibitor if the IGFBP2 levels are observed to decrease over time following said administration.
23. The method of item 21 wherein the IGF1R inhibitor is an antibody or antigen-binding fragment thereof that binds specifically to IGF1R.
24. The method of item 23 wherein the antibody or fragment comprises one or more complementarity determining regions (CDRs) selected from the group consisting of:
   RASQSIGSSLH (SEQ ID NO: 99);
   YASQSLS (SEQ ID NO: 100);
   HQSSRLPHT (SEQ ID NO: 101);
   SFAMH (SEQ ID NO:102)
   GFTFSSFAMH (SEQ ID NO: 107);
   VIDTRGATYYADSVKG (SEQ ID NO: 103); and
   LGNFYYGMDV (SEQ ID NO: 104);
   or a mature fragment of a light chain immunoglobulin which comprises the amino acid sequence of SEQ ID NO: 2, 4, 6 or 8; or a mature fragment of a heavy chain immunoglobulin which comprises the amino acid sequence of SEQ ID NO: 10 or 12; or a pharmaceutical composition thereof which comprises a pharmaceutically acceptable carrier.
25. The method of item 22 wherein step (i) is performed on a subject prior to any administration of said inhibitor.
26. The method of item 21 wherein the disorder is a member selected from the group consisting of: osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, autoimmune thyroiditis and Bechet's disease.
27. The method of item 21 wherein the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, obiimersen, ticilimumab, ipilimumab, gossypol, Bio 111, 131-I-TM-501, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 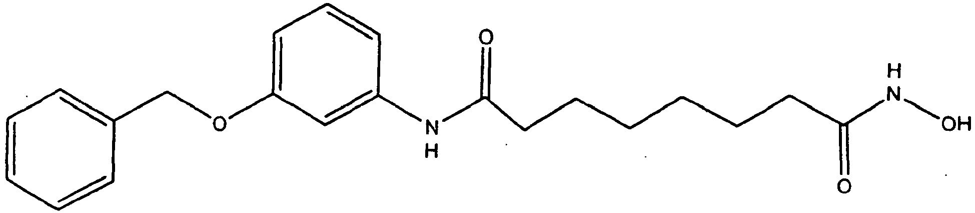 , CG-781, CG-1521, 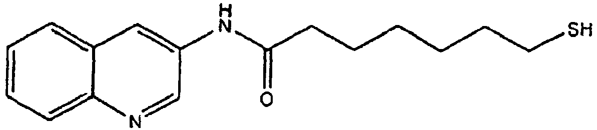 556629, chlamydocin, JNJ-16241199, , SB- 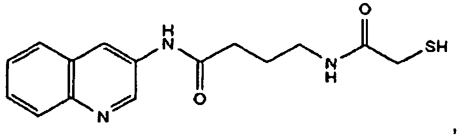 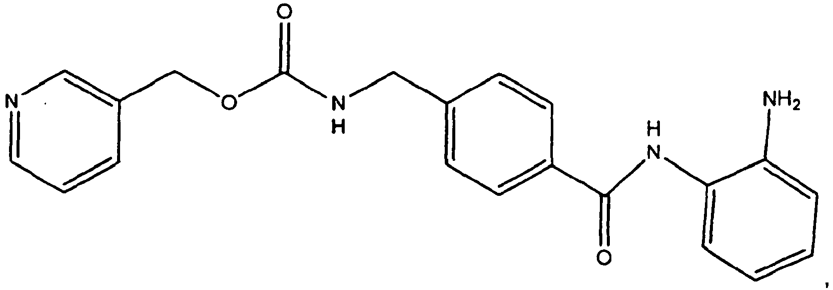 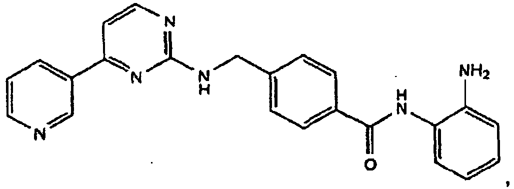 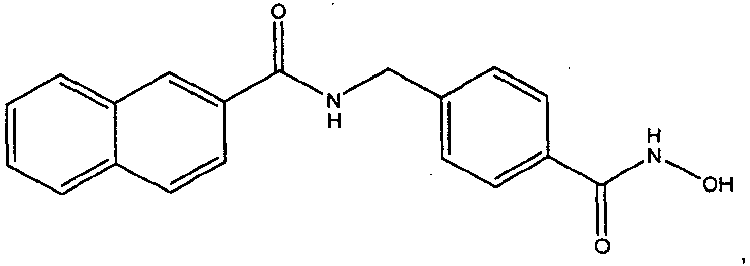 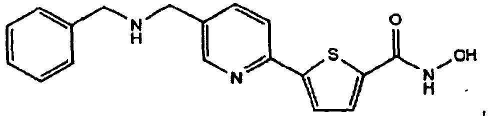 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N -[4-[2-(2-amino-4,7-dihydro-4-oxo-1 H - pyrrolo[2,3- d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 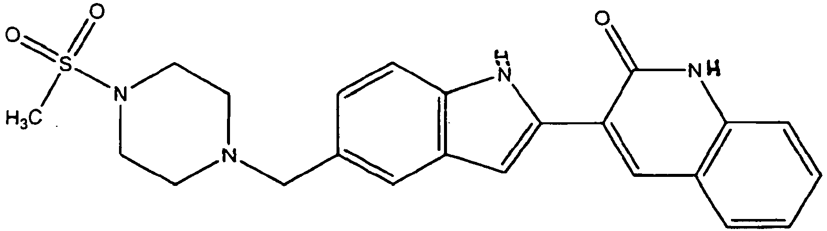 ); 3-[5-(methyfsulfonylpiperadinemethyl)-indolyl]-quinolone, vatalanib, AG-013736, AVE-0005, the acetate salt of [D-Ser(Bu t) 6 ,Azgly 10 ] (pyro-Glu-His-Trp-Ser-Tyr-D-Ser(Bu t )-Leu-Arg-Pro-Azgly-NH₂ acetate [C₅₉H₈₄N₁₈O₁₄ ·(C₂H₄O₂)ₓ where x = 1 to 2.4], goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, lonafarnib 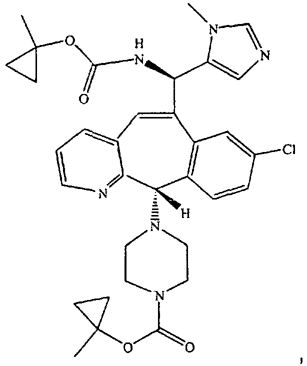 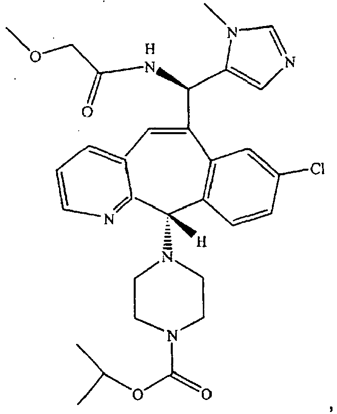 BMS-214662, tipifarnib; amifostine, NVP-LAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, , diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.
28. The method of item 21 wherein the IGFBP2 level is determined using a radioimmunoassay (RIA), a western blot or an enzyme linked immunosorbent assay (ELISA) of a sample from the subject.
29. The method of item 21 wherein IGFBP2 is determined in blood, serum or plasma from the subject.
30. A method for monitoring the effect of an IGF1R inhibitor on IGFBP2 concentration in the body of a subject administered said inhibitor comprising measuring IGFBP2 levels in the body of the subject over time during a course of treatment with said inhibitor.
31. The method of item 30 comprising:
   (i) measuring an IGFBP2 concentration in the body of said subject;
   (ii) administering one or more doses of said inhibitor to said subject;
   (iii) measuring an IGFBP2 concentration in the body of said subject following said administration;
   (iv) comparing the level of IGFBP2 measured in step (i) with the level of IGFBP2 measured in step (iii); wherein the inhibitor is determined to lower the IGFBP2 concentration if the level measured in step (i) is higher than the concentration measured in step (iii); and wherein the inhibitor is determined not to lower the IGFBP2 concentration if the level measured in step (i) is not higher than the concentration measured in step (iii).
32. The method of item 31 wherein step (i) is performed prior to any administration of said inhibitor.
33. A method for treating a medical condition, in a subject, mediated by IGF1 R expression or activity comprising:
   (i) measuring an IGFBP2 level in the body of said subject prior to any administration of an IGF1 R inhibitor;
   (ii) administering one or more doses of an IGF1 R inhibitor to said subject;
   (iii) measuring an IGFBP2 level in the body of said subject following said administration;
   (iv) comparing the level of IGFBP2 measured in step (i) with the level of IGFBP2 measured in step (iii); and
   (v) increasing dosage of said inhibitor if the IGFBP2 level does not decrease by at least 51 % following said administration; or maintaining or decreasing dosage if the IGFBP2 level does decrease by at least 51 % following said administration.
34. The method of item 33 wherein the IGF1R inhibitor is an antibody or antigen-binding fragment thereof that binds specifically to IGF1R.
35. The method of item 34 wherein the antibody or fragment comprises one or more complementarity determining regions (CDRs) selected from the group consisting of:
   RASQSIGSSLH (SEQ ID NO: 99);
   YASQSLS (SEQ ID NO: 100);
   HQSSRLPHT (SEQ ID NO: 101);
   SFAMH (SEQ ID NO:102)
   GFTFSSFAMH (SEQ ID NO: 107);
   VIDTRGATYYADSVKG (SEQ ID NO: 103); and
   LGNFYYGMDV (SEQ ID NO: 104);
   or a mature fragment of a light chain immunoglobulin which comprises the amino acid sequence of SEQ ID NO: 2, 4, 6 or 8; or a mature fragment of a heavy chain immunoglobulin which comprises the amino acid sequence of SEQ ID NO: 10 or 12; or a pharmaceutical composition thereof which comprises a pharmaceutically acceptable carrier:
36. The method of item 33 wherein the antibody or fragment is a monoclonal antibody.
37. The method of item 33 wherein the disorder is a member selected from the group consisting of: osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, autoimmune thyroiditis and Bechet's disease.
38. The method of item 33 wherein the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bd-2 inhibitor, an HDAC inhibitor, a c-MET inhibitor, a PARP inhibitor, a Cdk inhibitor, an EGFR TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipiiimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 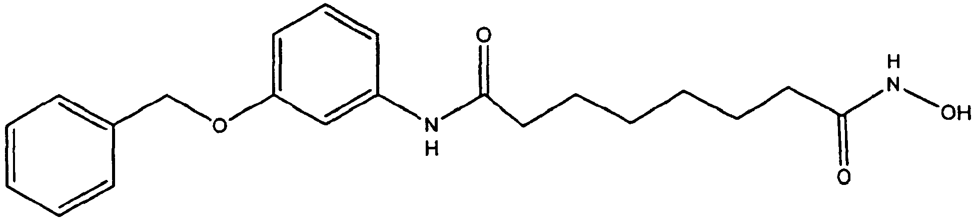 , CG-781, CG-1521, 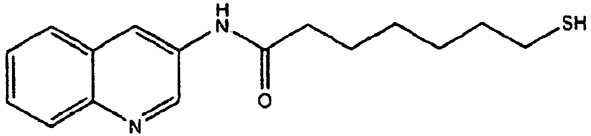 SB-556629, chlamydocin, JNJ-16241199, 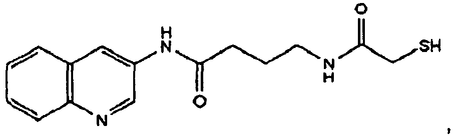 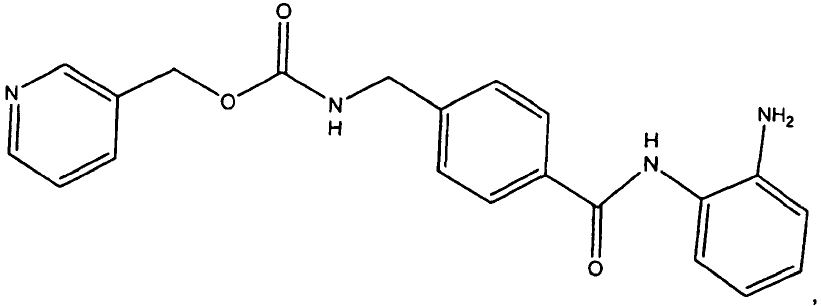 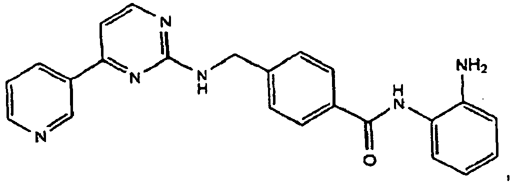 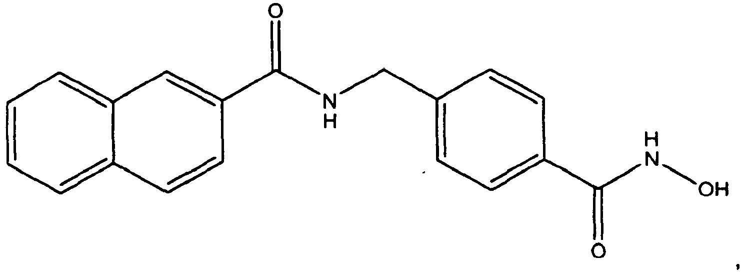 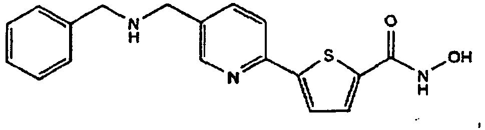 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244; capecitabine, L-Glutamic acid, N -[4-[2-(2-amino-4,7-dihydro-4-oxo-1 H - pyrrolo[2,3- d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 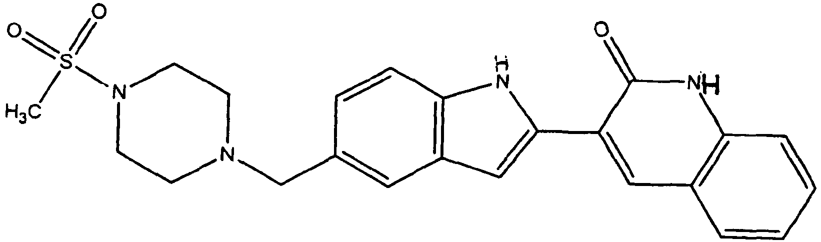 ); 3-[5-(methylsulfonyipiperadinemethyl)-indolyl]-quinolone, vatalanib, AG-013736, AVE-0005, the acetate salt of [D-Ser(Bu t) 6 ,Azgly 10] (pyro-Glu-His-Trp-Ser-Tyr-D-Ser(Bu t )-Leu-Arg-Pro-Azgly-NH ₂ acetate [C₅₉H₈₄N₁₈O₁₄ · (C₂H₄O₂)ₓ where x = 1 to 2.4], goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, Ionafarnib, 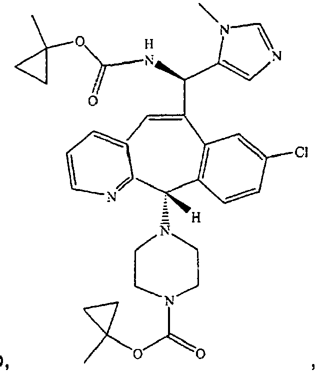 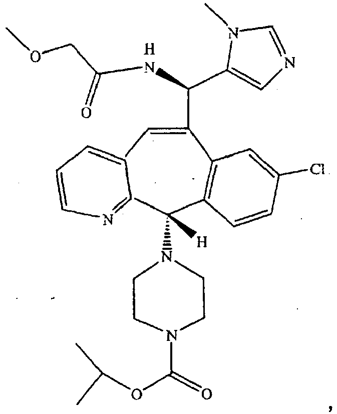 BMS-214662, tipifarnib; amifostine, NVP-LAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.
39. A method for selecting a dose of an IGF1 R inhibitor comprising administering a dose of said inhibitor to a subject with a medical condition mediated by IGF1 R expression or activity and evaluating IGFBP2 levels in the body of the subject; wherein said dosage is selected if IGFBP2 levels are observed to decrease, following said administration, by at least 51 % of an IGFBP2 level measured prior to first administration of said inhibitor.
40. The method of item 39 comprising:
   (i) measuring an IGFBP2 level in the body of said subject before first treatment with said inhibitor;
   (ii) administering one or more doses of said inhibitor to said subject;
   (iii) measuring an IGFBP2 level in the body of said subject following said administration;
   (iv) comparing the level of IGFBP2 measured in step (i) with the level of IGFBP2 measured in step (iii);
   wherein said dose is selected if IGFBP2 levels are observed to decrease, following said administration, by at least 51% of an IGFBP2 level measured before first treatment with said inhibitor; or wherein the dosage is not selected if IGFBP2 levels are not observed to decrease, following said administration, by at least 51 % of an IGFBP2 level measured before first treatment with said inhibitor.
41. The method of item 39 wherein the IGF1 R inhibitor is an antibody or antigen-binding fragment thereof that binds specifically to IGF1 R.
42. The method of item 41 wherein the antibody or fragment comprises one or more complementarity determining regions (CDRs) selected from the group consisting of:
   RASQSIGSSLH (SEQ ID NO: 99);
   YASQSLS (SEQ ID NO: 100);
   HQSSRLPHT (SEQ ID NO: 101);
   SFAMH (SEQ ID NO:102)
   GFTFSSFAMH (SEQ ID NO: 107);
   VIDTRGATYYADSVKG (SEQ ID NO: 103); and
   LGNFYYGMDV (SEQ ID NO: 104);
   or a mature fragment of a light chain immunoglobulin which comprises the amino acid sequence of SEQ ID NO: 2, 4, 6 or 8; or a mature fragment of a heavy chain immunoglobulin which comprises the amino acid sequence of SEQ ID NO: 10 or 12; or a pharmaceutical composition thereof which comprises a pharmaceutically acceptable carrier.
43. The method of item 39 wherein the disorder is a member selected from the group consisting of: osteosarcoma, rhabdomyosarcoma, neuroblastoma, any pediatric cancer, kidney cancer, leukemia, renal transitional cell cancer, Werner-Morrison syndrome, acromegaly, bladder cancer, Wilm's cancer, ovarian cancer, pancreatic cancer, benign prostatic hyperplasia, breast cancer, prostate cancer, bone cancer, lung cancer, gastric cancer, colorectal cancer, cervical cancer, synovial sarcoma, diarrhea associated with metastatic carcinoid, vasoactive intestinal peptide secreting tumors, gigantism, psoriasis, atherosclerosis, smooth muscle restenosis of blood vessels and inappropriate microvascular proliferation, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, Burkitt Lymphoma, mycosis fungoides, seary syndrome, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma, a myeloproliferative disorder, polycythemia vera, thrombocythemia, idiopathic myelfibrosis, soft tissue sarcoma, thyroid cancer, endometrial cancer, carcinoid cancer, germ cell tumors, liver cancer, gigantism, psoriasis, atherosclerosis, smooth muscle restenosis of blood vessels, inappropriate microvascular proliferation, acromegaly, gigantism, psoriasis, atherosclerosis, smooth muscle restenosis of blood vessels or inappropriate microvascular proliferation, Grave's disease, multiple sclerosis, systemic lupus erythematosus, Hashimoto's Thyroiditis, Myasthenia Gravis, auto-immune thyroiditis and Bechet's disease.
44. The method of item 39 wherein the subject is also administered one or more members selected from the group consisting of everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 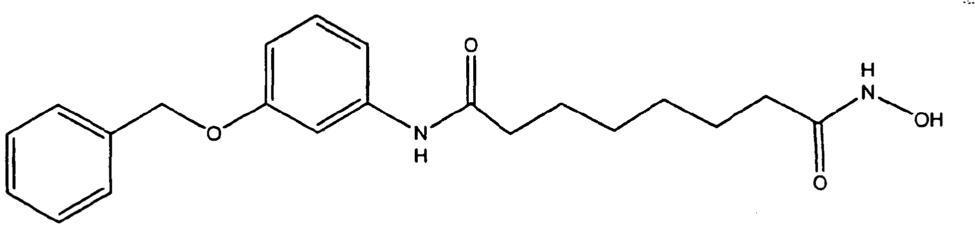 , CG-781, CG-1521, 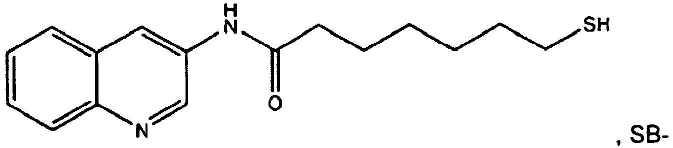 556629, chlamydocin, JNJ-16241199, 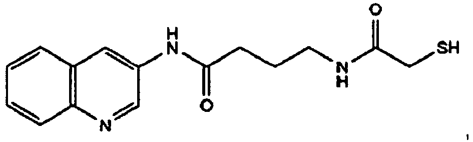 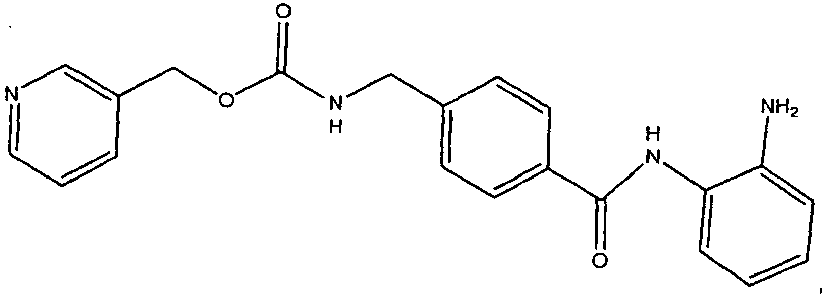 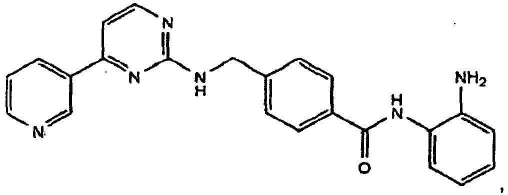 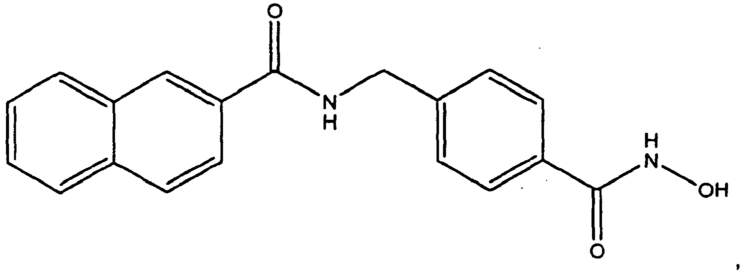 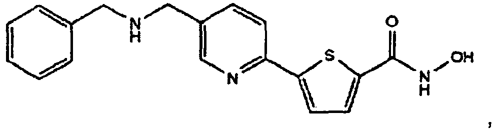 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N -[4-[2-(2-amino-4,7-dihydro-4-oxo-1 H - pyrrolo[2,3- d]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 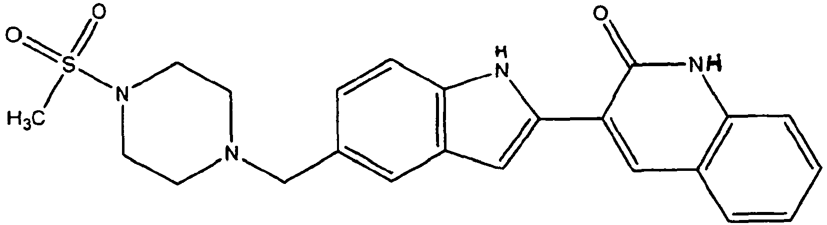 ); 3-[5-(methylsulfonylpiperadinemethyl)-indolyl]-quinolone, vatalanib, AG-013736, AVE-0005, the acetate salt of [D-Ser(Bu t) 6, Azgly 10 ] (pyro-Glu-His-Trp-Ser-Tyr-D-Ser(Bu t )-Leu-Arg-Pro-Azgly-NH ₂ acetate [C₅₉H₈₄N₁₈O₁₄ ·(C₂H₄O₂)ₓ where x = 1 to 2.4], goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, Ionafarnib, 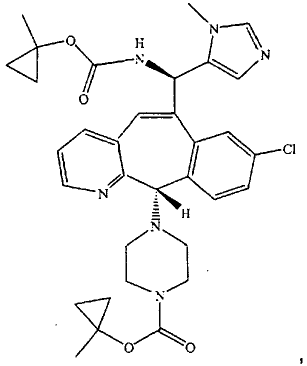 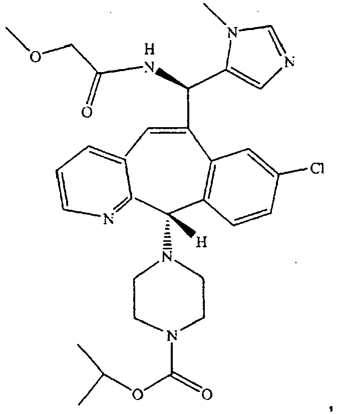 BMS-214662, tipifarnib; amifostine, NVP-LAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.

## Claims

1. A method for monitoring the effect of an IGF1R inhibitor on the IGF1 receptor in a mammalian subject that suffers from cancer, for determining if a mammalian subject that suffers from cancer has a cancer that is responsive to an IGF1R inhibitor; or for evaluating a dosage of an IGF1R inhibitor over time in a mammalian subject that suffers from cancer comprising the steps of:
(i) measuring an IGFBP2 level in a sample of tumor tissue, plasma, blood or serum of said subject before the IGF1R inhibitor is given;
(ii) measuring an IGFBP2 level in the sample of said subject following administration to said subject of one or more doses of the IGF1R inhibitor; and
(iii) comparing the level of IGFBP2 measured in step (i) with the level of IGFBP2 measured in step (ii);
wherein the inhibitor is determined to inhibit the receptor if IGFBP2 levels are observed to decrease over time following said administration; wherein the cancer is determined to be responsive to said inhibitor if the IGFBP2 levels are observed to decrease over time following said administration; or wherein said dosage is determined to be sufficient if IGFBP2 levels are observed to decrease by at least 51 % of an IGFBP2 level measured prior to first administration of said inhibitor following said administration.

2. The method of claim 1 for monitoring the effect of an IGF1R inhibitor on the IGF1 receptor.

3. The method of claim 2, wherein for monitoring the effect of an IGF1R inhibitor on the IGF1 receptor, the inhibitor is determined to inhibit the receptor sufficiently if IGFBP2 levels are observed to decrease by at least 51% over time following a first administration of said inhibitor.

4. The method of claim 1 for determining if a mammalian subject that suffers from a cancer has a cancer that is responsive to an IGF1R inhibitor.

5. The method of claim 1 for evaluating a dosage of an IGF1R inhibitor over time in a mammalian subject that suffers from cancer.

6. The method of claim 5, wherein for evaluating a dosage of an IGF1R inhibitor over time in a mammalian subject that suffers from cancer, the dosage is selected if said dosage is determined to be sufficient.

7. The method of claim 1, wherein the IGF1R inhibitor is an antibody or antigen-binding fragment thereof that binds specifically to IGF1R.

8. The method of claim 7, wherein the antibody or fragment comprises a light chain immunoglobulin comprising CDR-L1 comprising the amino acid sequence RASQSIGSSLH (SEQ ID NO: 99); CDR-L2 comprising the amino acid sequence YASQSLS (SEQ ID NO: 100); or CDR-L3 comprising the amino acid sequence HQSSRLPHT (SEQ ID NO: 101); or wherein the antibody or fragment comprises a heavy chain immunoglobulin comprising CDR-H1 comprising the amino acid sequence SFAMH (SEQ ID NO:102) or GFTFSSFAMH (SEQ ID NO: 107); CDR-H2 comprising the amino acid sequence VIDTRGATYYADSVKG (SEQ ID NO: 103); or CDR-H3 comprising the amino acid sequence LGNFYYGMDV (SEQ ID NO: 104), or a pharmaceutical composition thereof which comprises a pharmaceutically acceptable carrier; or, wherein the antibody or fragment thereof comprises a mature fragment of a light chain immunoglobulin which comprises the amino acid sequence as set forth in SEQ ID NO: 2, 4, 6 or 8; or a mature fragment of a heavy chain immunoglobulin which comprises the amino acid sequence as set forth in SEQ ID NO: 10 or 12; or a pharmaceutical composition thereof which comprises a pharmaceutically acceptable carrier.

9. The method of claim 1, wherein cancer is selected from the group consisting of: osteosarcoma, rhabdomyosarcoma, neuroblastoma, pediatric cancer, kidney cancer, leukemia, renal transitional cell cancer, bladder cancer, Wilm's cancer, ovarian cancer, pancreatic cancer, breast cancer, prostate cancer, bone cancer, lung cancer, gastric cancer, colorectal cancer, cervical cancer, synovial sarcoma, vasoactive intestinal peptide secreting tumors, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, haemotological malignancy, chronic lymphoblastic leukemia, chronic myelomonocytic leukemia, acute lymphoblastic leukemia, acute lymphocytic leukemia, acute myelogenous leukemia, acute myeloblastic leukemia, chronic myeloblastic leukemia, Hodgekin's disease, non-Hodgekin's lymphoma, chronic lymphocytic leukemia, chronic myelogenous leukemia, hairy cell leukemia, mast cell leukemia, mast cell neoplasm, follicular lymphoma, diffuse large cell lymphoma, mantle cell lymphoma, Burkitt Lymphoma, mycosis fungoides, cutaneous T-cell lymphoma, chronic myeloproliferative disorders, a central nervous system tumor, brain cancer, glioblastoma, non-glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma, a myeloproliferative disorder, soft tissue sarcoma, thyroid cancer, endometrial cancer, carcinoid cancer, germ cell tumors, liver cancer.

10. The method of claim 1, wherein the subject is also administered one or more members selected from the group consisting of everolimus, trabectedin, abraxane, TLK 286, AV-299, DN-101, pazopanib, GSK690693, RTA 744, ON 0910.Na, AZD 6244, AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase inhibitor, a VEGF trap receptor, pemetrexed, erlotinib, dasatanib, nilotinib, decatanib, panitumumab, amrubicin, oregovomab, Lep-etu, nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 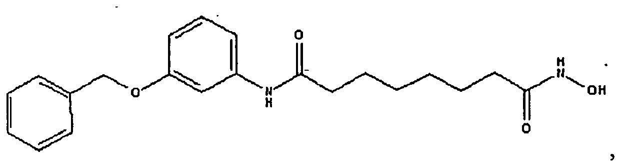 CG-781, CG-1521, 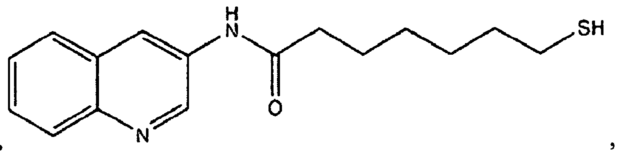 SB-556629, chlamydocin, JNJ-16241199, 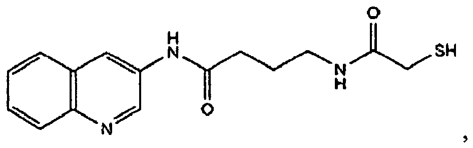 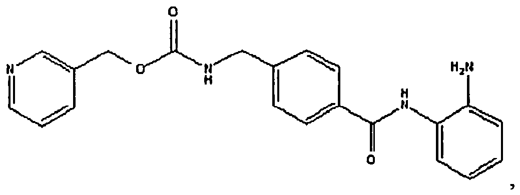 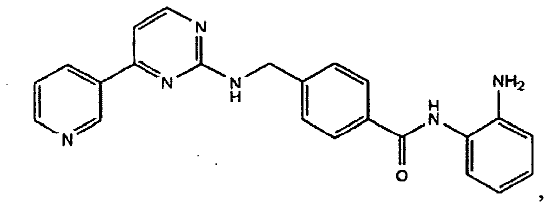 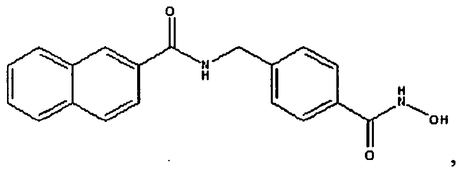 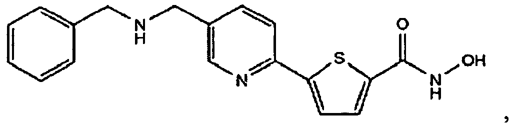 vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N -[4-[2-(2-amino-4,7-dihydro-4-oxo-1 H -pyrrolo[2,3- d ]pyrimidin-5-yl)ethyl]benzoyl]-, disodium salt, heptahydrate, camptothecin, irinotecan; a combination of irinotecan, 5-fluorouracil and leucovorin; PEG-labeled irinotecan, FOLFOX regimen, tamoxifen, toremifene citrate, anastrazole, exemestane, letrozole, DES(diethylstilbestrol), estradiol, estrogen, conjugated estrogen, bevacizumab, IMC-1C11, CHIR-258, 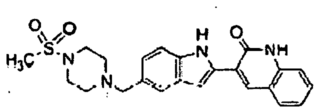 ); 3-[5-(methylsulfonylpiperadinemethyl)-indolyl]-quinolone, vatalanib, AG-013736, AVE-0005, goserelin acetate, leuprolide acetate, triptorelin pamoate, sunitinib, sunitinib malate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib, canertinib, ABX-EGF antibody, erbitux, EKB-569, PKI-166, GW-572016, Ionafarnib, 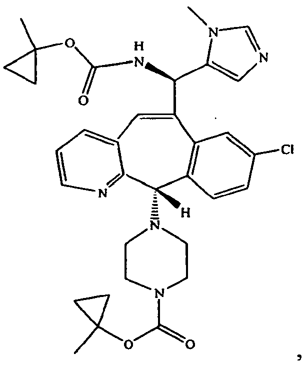 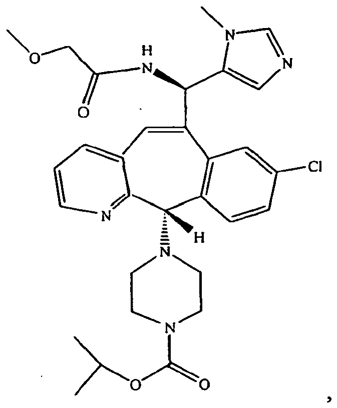 BMS-214662, tipifarnib; amifostine, NVP-LAQ824, suberoyl analide hydroxamic acid, valproic acid, trichostatin A, FK-228, SU11248, sorafenib, KRN951, aminoglutethimide, amsacrine, anagrelide, L-asparaginase, Bacillus Calmette-Guerin (BCG) vaccine, bleomycin, buserelin, busulfan, carboplatin, carmustine, chlorambucil, cisplatin, cladribine, clodronate, cyclophosphamide, cyproterone, cytarabine, dacarbazine, dactinomycin, daunorubicin, diethylstilbestrol, epirubicin, fludarabine, fludrocortisone, fluoxymesterone, flutamide, hydroxyurea, idarubicin, ifosfamide, imatinib, leucovorin, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa.

11. The method of claim 8, wherein the inhibitor is an antibody comprising a light chain immunoglobulin variable region comprising CDR-L1 comprising the amino acid sequence RASQSIGSSLH (SEQ ID NO: 99); CDR-L2 comprising the amino acid sequence YASQSLS (SEQ ID NO: 100); and CDR-L3 comprising the amino acid sequence HQSSRLPHT (SEQ ID NO: 101); and a heavy chain immunoglobulin variable region comprising CDR-H1 comprising the amino acid sequence SFAMH (SEQ ID NO:102) or GFTFSSFAMH (SEQ ID NO: 107); CDR-H2 comprising the amino acid sequence VIDTRGATYYADSVKG (SEQ ID NO: 103); and CDR-H3 comprising the amino acid sequence LGNFYYGMDV (SEQ ID NO: 104).

12. The method of claim 7, wherein the inhibitor is an antibody comprising an immunoglobulin light chain variable region comprising the amino acid sequence as set forth in SEQ ID NO: 38; and an immunoglobulin heavy chain variable region comprising the amino acid sequence as set forth in SEQ ID NO: 40.

13. The method of claim 11, wherein the antibody comprises a light chain immunoglobulin variable region comprising amino acids 20 to 128 of the amino acid sequence as set forth in SEQ ID NO: 8; and a heavy chain immunoglobulin variable region comprising amino acids 20 to 137 of the amino acid sequence as set forth in SEQ ID NO: 10.

14. The method of any of the preceding claims, wherein the cancer is colorectal cancer or lung cancer.

15. The method of any of the preceding claims, wherein the subject is a human.

16. The method of any of the preceding claims, wherein the IGFBP2 level is measured using a radioimmunoassay (RIA), a western blot or an enzyme linked immunosorbent assay (ELISA).

## Patentansprüche

1. Ein Verfahren, um die Wirkung eines IGF1R-Inhibitors auf den IGF1-Rezeptor bei einem an Krebs leidenden Säugersubjekt zu verfolgen, um zu ermitteln, ob ein an Krebs leidendes Säugersubjekt einen Krebs besitzt, der auf einen IGF1R-Inhibitor anspricht, oder um eine Dosis eines IGF1R-Inhibitors bei einem an Krebs leidenden Säugersubjekt im Zeitverlauf zu untersuchen, umfassend die Schritte:
(i) Messen eines IGFBP2-Spiegels in einer Tumorgewebe-, Plasma-, Blut- oder Serumprobe des Subjekts, bevor der IGF1R-Inhibitor verabreicht wird,
(ii) Messen eines IGFBP2-Spiegels in der Probe des Subjekts nach der Verabreichung von einer oder mehreren Dosen des IGF1R-Inhibitors an das Subjekt, und
(iii) Vergleichen des in Schritt (i) gemessenen IGFBP2-Spiegels mit dem in Schritt (ii) gemessenen IGFBP2-Spiegel,
wobei der Inhibitor als den Rezeptor inhibierend befunden wird, wenn zu beobachten ist, dass die IGFBP2-Spiegel im Zeitverlauf nach der Verabreichung abnehmen, wobei der Krebs als auf den Inhibitor ansprechend befunden wird, wenn zu beobachten ist, dass die IGFBP2-Spiegel im Zeitverlauf nach der Verabreichung abnehmen, oder wobei die Dosis als ausreichend befunden wird, wenn zu beobachten ist, dass die IGFBP2-Spiegel nach der Verabreichung um wenigstens 51 % abnehmen, verglichen mit einem vor der ersten Verabreichung des Inhibitors gemessenen IGFBP2-Spiegel.

2. Das Verfahren nach Anspruch 1 zur Verfolgung der Wirkung eines IGF1R-Inhibitors auf den IGF1-Rezeptor.

3. Das Verfahren nach Anspruch 2, wobei zur Verfolgung der Wirkung eines IGF1R-Inhibitors auf den IGF1-Rezeptor der Inhibitor als den Rezeptor ausreichend inhibierend befunden wird, wenn zu beobachten ist, dass die IGFBP2-Spiegel im Zeitverlauf nach einer ersten Verabreichung des Inhibitors um wenigstens 51 % abnehmen.

4. Das Verfahren nach Anspruch 1 zur Ermittlung, ob ein an Krebs leidendes Säugersubjekt einen Krebs besitzt, der auf einen IGF1R-Inhibitor anspricht.

5. Das Verfahren nach Anspruch 1 zur Untersuchung einer Dosis eines IGF1R-Inhibitors im Zeitverlauf bei einem an Krebs leidenden Säugersubjekt.

6. Das Verfahren nach Anspruch 5, wobei zur Untersuchung einer Dosis eines IGF1R-Inhibitors im Zeitverlauf bei einem an Krebs leidenden Säugersubjekt die Dosis gewählt wird, wenn die Dosis als ausreichend befunden wird.

7. Das Verfahren nach Anspruch 1, wobei der IGF1R-Inhibitor ein Antikörper oder ein Antigen-bindendes Fragment davon ist, das sich spezifisch an IGF1R bindet.

8. Das Verfahren nach Anspruch 7, wobei der Antikörper oder das Fragment ein Immunglobulin mit leichter Kette umfasst, das CDR-L1, das die Aminosäuresequenz RASQSIGSSLH (SEQ ID NO: 99) umfasst, CDR-L2, das die Aminosäuresequenz YASQSLS (SEQ ID NO: 100) umfasst, oder CDR-L3, das die Aminosäuresequenz HQSSRLPHT (SEQ ID NO: 101) umfasst; umfasst, oder wobei der Antikörper oder das Fragment ein Immunglobulin mit schwerer Kette umfasst, das CDR-H1, das die Aminosäuresequenz SFAMH (SEQ ID NO: 102) oder GFTFSSFAMH (SEQ ID NO: 107) umfasst, CDR-H2, das die Aminosäuresequenz VIDTRGATYYADSVKG (SEQ ID NO: 103) umfasst, oder CDR-H3, das die Aminosäuresequenz LGNFYYGMDV (SEQ ID NO: 104) umfasst, umfasst, oder eine pharmazeutische Zusammensetzung davon, die einen pharmazeutisch annehmbaren Träger umfasst, oder wobei der Antikörper oder das Fragment davon ein reifes Fragment eines Immunglobulins mit leichter Kette umfasst, das die wie in SEQ ID NO: 2, 4, 6 oder 8 angegebene Aminosäuresequenz umfasst, oder ein reifes Fragment eines Immunglobulins mit schwerer Kette umfasst, das die wie in SEQ ID NO: 10 oder 12 angegebene Aminosäuresequenz umfasst, oder eine pharmazeutisch annehmbare Zusammensetzung davon, die einen pharmazeutisch annehmbaren Träger umfasst.

9. Das Verfahren nach Anspruch 1, wobei der Krebs ausgewählt ist aus der Gruppe, bestehend aus: Osteosarkom, Rhabdomyosarkom, Neuroblastom, Krebs bei Kindern, Nierenkrebs, Leukämie, renales Übergangszellkarzinom, Blasenkrebs, Wilms-Tumor, Ovarialkarzinom, Bauchspeicheldrüsenkrebs, Brustkrebs, Prostatakrebs, Knochenkrebs, Lungenkrebs, Magenkrebs, kolorektales Karzinom, Gebärmutterhalskrebs, Synovialsarkom, vasoaktives intestinales Peptid sekretierende Tumore, Kopf- und Halskrebs, Plattenepithelkarzinom, multiples Myelom, Solitär-Plasmozytom, Nierenzellkarzinom, Retinoblastom, Keimzelltumore, Hepatoblastom, hepatozelluläres Karzinom, Melanom, Rhabdoid-Tumor der Niere, Ewing-Sarkom, Chondrosarkom, hämatologische Malignizität, chronische lymphoblastische Leukämie, chronische myelomonozytäre Leukämie, akute lymphoblastische Leukämie, akute lymphatische Leukämie, akute myeloische Leukämie, akute myeloblastische Leukämie, chronische myeloblastische Leukämie, Morbus Hodgkin, Non-Hodgkins-Lymphom, chronische lymphatische Leukämie, chronische myeloische Leukämie, Haarzellleukämie, Mastzellleukämie, Mastzellblastom, follikuläres Lymphom, diffuses großzelliges Lymphom, Mantelzelllymphom, Burkitt-Lymphom, Mycosis fungoides, kutanes T-Zell-Lymphom, chronische myeloproliferative Störungen, ein Zentralnervensystemtumor, Gehirntumor, Glioblastom, Nicht-Glioblastom-Gehirntumor, Meningiom, Hypophysenadenom, vestibuläres Schwannom, ein primitiver neuroektodermaler Tumor, Medulloblastom, Astrozytom, anaplastisches Astrozytom, Oligodendrogliom, Ependymom, eine myeloproliferative Störung, Weichgewebesarkom, Schilddrüsenkrebs, Endometrialkarzinom, karzinoider Krebs, Keimzelltumore, Leberkrebs.

10. Das Verfahren nach Anspruch 1, wobei dem Subjekt auch ein oder mehrere Elemente verabreicht werden, ausgewählt aus der Gruppe, bestehend aus Everolimus, Trabectedin, Abraxan, TLK 286, AV-299, DN-101, Pazopanib, GSK690693, RTA 744, ON 0910.Na, AZD 6244, AMN-107, TKI-258, GSK461364, AZD 1152, Enzastaurin, Vandetanib, ARQ-197, MK-0457, MLN8054, PHA-739358, R-763, AT-9263, einem FLT-3-Inhibitor, einem VEGFR-Inhibitor, einem EGFR-TK-Inhibitor, einem Aurora-Kinase-Inhibitor, einem PIK-1-Modulator, einem Bcl-2-Inhibitor, einem HDAC-Inhibitor, einem c-MET-Inhibitor, einem PARP-Inhibitor, einem Cdk-Inhibitor, einem Anti-HGF-Antikörper, einem P13-Kinase-Inhibitor, einem AKT-Inhibitor, einem JAK/STAT-Inhibitor, einem Checkpoint-1- oder -2-Inhibitor, einem Inhibitor der fokalen Adhäsionskinase, einem Map-Kinase-Inhibitor, einem VEGF-Trap-Rezeptor, Pemetrexed, Erlotinib, Dasatanib, Nilotinib, Decatanib, Panitumumab, Amrubicin, Oregovomab, Lep-etu, Nolatrexed, azd2171, Batabulin, Ofatumumab, Zanolimumab, Edotecarin, Tetrandrin, Rubitecan, Tesmilifen, Oblimersen, Ticilimumab, Ipilimumab, Gossypol, Bio 111, 131-I-TM-601, ALT-1 10, BIO 140, CC 8490, Cilengitid, Gimatecan, IL13-PE38QQR, INO 1001, IPdR, KRX-0402, Lucanthon, LY 317615, Neuradiab, Vitespan, Rta 744, Sdx 102, Talampanel, Atrasentan, Xr 311, Romidepsin, ADS-100380, 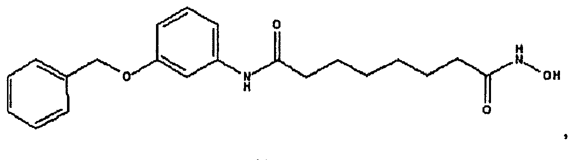 CG-781, CG-1521, 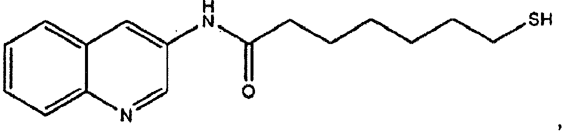 SB-556629, Chlamydocin , JNJ-16241199, 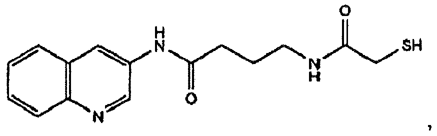 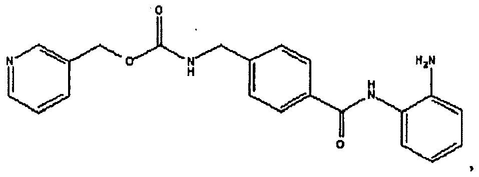 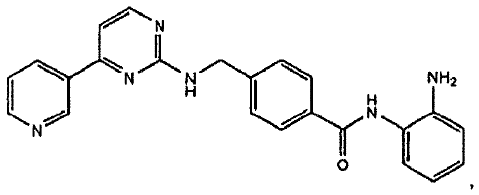 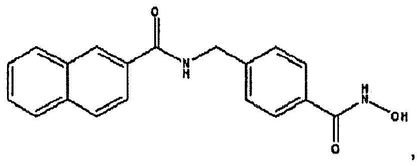 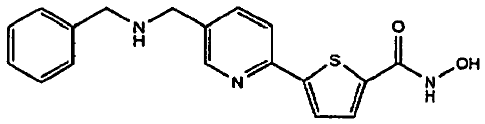 Vorinostat, Etoposid, Gemcitabin, Doxorubicin, Liposomal-Doxorubicin, 5'-Desoxy-5-fluoruridin, Vincristin, Temozolomid, ZK-304709, Seliciclib, PD0325901, AZD-6244, Capecitabin, L-Glutaminsäure, N-[4-[2-(2-Amino-4,7-dihydro-4-oxo-1 H-pyrrolo[2,3-d]pyrimidin-5-yl)ethyl]benzoyl]-Dinatriumsalz-Heptahydrat, Camptothecin, Irinotecan, einer Kombination aus Irinotecan, 5-Fluoruracil und Leucovorin, PEG-markiertem Irinotecan, FOLFOX-Regime, Tamoxifen, Toremifencitrat, Anastrazol, Exemestan, Letrozol, DES (Diethylstilbestrol), Östradiol, Östrogen, konjugiertem Östrogen, Bevacizumab, IMC-1C11, CHIR-258, 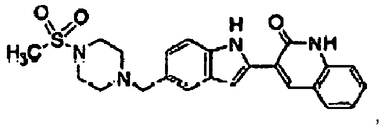 3-[5-(Methylsulfonylpiperadinmethyl)indolyl]chinolon, Vatalanib, AG-013736, AVE-0005, Goserelinacetat, Leuprolidacetat, Triptorelinpamoat, Sunitinib, Sunitinib-Malat, Medroxyprogesteronacetat, Hydroxyprogesteroncaproat, Megestrolacetat, Raloxifen, Bicalutamid, Flutamid, Nilutamid, Megestrolacetat, CP-724714, TAK-165, HKI-272, Erlotinib, Lapatanib, Canertinib, ABX-EGF-Antikörper, Erbitux, EKB-569, PKI-166, GW-572016, Lonafarnib, 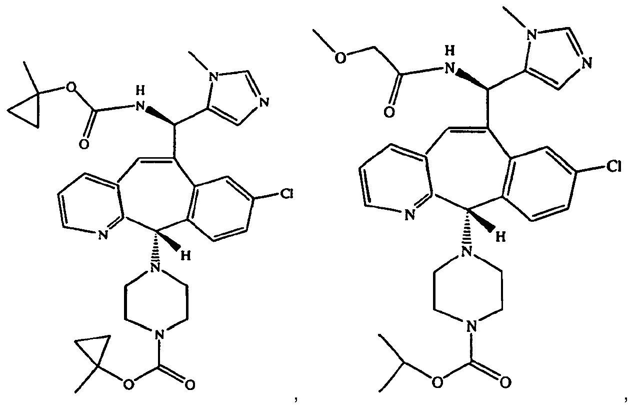 BMS-214662, Tipifarnib, Amifostin, NVP-LAQ824, Suberoylanilid-Hydroxamsäure, Valproinsäure, Trichostatin A, FK-228, SU11248, Sorafenib, KRN951, Aminoglutethimid, Amsacrin, Anagrelid, L-Asparaginase, Bacillus-Calmette-Guerin(BCG)-Vakzin, Bleomycin, Buserelin, Busulfan, Carboplatin, Carmustin, Chlorambucil, Cisplatin, Cladribin, Clodronat, Cyclophosphamid, Cyproteron, Cytarabin, Dacarbazin, Dactinomycin, Daunorubicin, Diethylstilbestrol, Epirubicin, Fludarabin, Fludrocortison, Fluoxymesteron, Flutamid, Hydroxyharnstoff, Idarubicin, Ifosfamid, Imatinib, Leucovorin, Leuprolid, Levamisol, Lomustin, Mechlorethamin, Melphalan, 6-Mercaptopurin, Mesna, Methotrexat, Mitomycin, Mitotan, Mitoxantron, Nilutamid, Octreotid, Oxaliplatin, Pamidronat, Pentostatin, Plicamycin, Porfimer, Procarbazin, Raltitrexed, Rituximab, Streptozocin, Teniposid, Testosteron, Thalidomid, Thioguanin, Thiotepa, Tretinoin, Vindesin, 13-cis-Retinsäure, Phenylalaninlost, Uracillost, Estramustin, Altretamin, Floxuridin, 5-Desoxyuridin, Cytosin-Arabinosid, 6-Mercaptopurin, Desoxycoformycin, Calcitriol, Valrubicin, Mithramycin, Vinblastin, Vinorelbin, Topotecan, Razoxin, Marimastat, COL-3, Neovastat, BMS-275291, Squalamin, Endostatin, SU5416, SU6668, EMD121974, Interleukin-12, IM862, Angiostatin, Vitaxin, Droloxifen, Idoxyfen, Spironolacton, Finasterid, Cimitidin, Trastuzumab, Denileukin Diftitox, Gefitinib, Bortezimib, Paclitaxel, cremophorfreiem Paclitaxel, Docetaxel, Epithilon B, BMS-247550, BMS-310705, Droloxifen, 4-Hydroxytamoxifen, Pipendoxifen, ERA-923, Arzoxifen, Fulvestrant, Acolbifen, Lasofoxifen, Idoxifen, TSE-424, HMR-3339, ZK186619, Topotecan, PTK787/ZK 222584, VX-745, PD 184352, Rapamcyin, 40-O-(2-Hydroxyethyl)rapamycin, Temsirolimus, AP-23573, RAD001, ABT-578, BC-210, LY294002, LY292223, LY292696, LY293684, LY293646, Wortmannin, ZM336372, L-779,450, PEG-Filgrastim, Darbepoetin, 5-Fluoruracil, Erythropoietin, Granulozyten-Kolonie-stimulierendem Faktor, Zolendronat, Prednison, Cetuximab, Granulozyten-Makrophagen-Kolonie-stimulierendem Faktor, Histrelin, pegyliertem Interferon-alfa-2a, Interferon-alfa-2a, pegyliertem Interferon-alfa-2b, Interferon-alfa-2b, Azacitidin, PEG-L-Asparaginase, Lenalidomid, Gemtuzumab, Hydrokortison, Interleukin-11, Dexrazoxan, Alemtuzumab, All-trans-Retinsäure, Ketoconazol, Interleukin-2, Megestrol, Immunglobulin, Stickstofflost, Methylprednisolon, Ibritgumomab-Tiuxetan, Androgenen, Decitabin, Hexamethylmelamin, Bexaroten, Tositumomab, Arsentrioxid, Kortison, Editronat, Mitotan, Cyclosporin, Liposomal-Daunorubicin, Edwina-Asparaginase, Strontium 89, Casopitant, Netupitant, einem NK-1-Rezeptor-Antagonisten, Palonosetron, Aprepitant, Diphenhydramin, Hydroxyzin, Metoclopramid, Lorazepam, Alprazolam, Haloperidol, Droperidol, Dronabinol, Dexamethason, Methylprednisolon, Prochlorperazin, Granisetron, Ondansetron, Dolasetron, Tropisetron, Pegfilgrastim, Erythropoietin, Epoetin-alfa und Darbepoetin-alfa.

11. Das Verfahren nach Anspruch 8, wobei der Inhibitor ein Antikörper ist, der eine variable Region eines Immunglobulins mit leichter Kette, umfassend CDR-L1, das die Aminosäuresequenz RASQSIGSSLH (SEQ-ID Nr.: 99) umfasst, CDR-L2, das die Aminosäuresequenz YASQSLS (SEQ ID Nr.: 100) umfasst, und CDR-L3, das die Aminosäuresequenz HQSSRLPHT (SEQ-ID Nr.: 101) umfasst, und eine variable Region eines Immunglobulins mit schwerer Kette, umfassend CDR-H1, das die Aminosäuresequenz SFAMH (SEQ-ID Nr.: 102) oder GFTFSSFAMH (SEQ-ID Nr.: 107) umfasst, CDR-H2, das die Aminosäuresequenz VIDTRGATYYADSVKG (SEQ-ID Nr.: 103) umfasst, und CDR-H3, das die Aminosäuresequenz LGNFYYGMDV (SEQ-ID Nr.: 104) umfasst, umfasst.

12. Das Verfahren nach Anspruch 7, wobei der Inhibitor ein Antikörper ist, der eine variable Region eines Immunglobulins mit leichter Kette, umfassend die Aminosäuresequenz wie in SEQ-ID Nr.: 38 angegeben, und eine variable Region eines Immunglobulins mit schwerer Kette, umfassend die Aminosäuresequenz wie in SEQ-ID Nr.: 40 angegeben, umfasst.

13. Das Verfahren nach Anspruch 11, wobei der Antikörper eine variable Region eines Immunglobulins mit leichter Kette, umfassend die Aminosäuren 20 bis 128 der in SEQ-ID Nr.: 8 angegebenen Aminosäuresequenz, und eine variable Region eines Immunglobulins mit schwerer Kette, umfassend die Aminosäuren 20 bis 137 der in SEQ-ID Nr.: 10 angegebenen Aminosäuresequenz, umfasst.

14. Das Verfahren nach einem der vorhergehenden Ansprüche, wobei der Krebs kolorektales Karzinom oder Lungenkrebs ist.

15. Das Verfahren nach einem der vorhergehenden Ansprüche, wobei das Subjekt ein Mensch ist.

16. Das Verfahren nach einem der vorhergehenden Ansprüche, wobei der IGFBP2-Spiegel mit Hilfe eines Radioimmunassays (RIA), eines Western-Blots oder eines enzymgekoppelten Immunadsorptionstests (ELISA) gemessen wird.

## Revendications

1. Procédé destiné à contrôler l'effet d'un inhibiteur d'IGF1R sur le récepteur d'IGF1 chez un sujet mammalien qui souffre d'un cancer, afin de déterminer si un sujet mammalien qui souffre d'un cancer a un cancer qui répond à un inhibiteur d'IGF1R ou destiné à évaluer une dose d'un inhibiteur d'IGFIR au cours du temps chez un sujet mammalien qui souffre d'un cancer, comprenant les étapes consistant à:
(i) mesurer un taux d'IGFBP2 dans un échantillon de tissu tumoral, de plasma, de sang ou de sérum dudit sujet préalablement à l'administration de l'inhibiteur d'IGF1R;
(ii) mesurer un taux d'IGFBP2 dans l'échantillon dudit sujet suite à l'administration audit sujet d'une ou de plusieurs doses de l'inhibiteur d'IGF1R; et
(iii) comparer le taux d'IGFBP2 mesuré à l'étape (i) au taux d'IGFBP2 mesuré à l'étape (ii);
où il est déterminé que l'inhibiteur inhibe le récepteur si les taux d'IGFBP2 sont observés diminuer au cours du temps suite à ladite administration; où il est déterminé que le cancer répond audit inhibiteur si les taux d'IGFBP2 sont observés diminuer au cours du temps suite à ladite administration; ou bien où ladite dose est déterminée être suffisante si les taux d'IGFBP2 sont observés diminuer d'au moins 51% par rapport à un taux d'IGFBP2 mesuré avant la première administration dudit inhibiteur, suite à ladite administration.

2. Procédé selon la revendication 1, destiné à contrôler l'effet d'un inhibiteur d'IGF1R sur le récepteur d'IGF1.

3. Procédé selon la revendication 2, dans lequel pour contrôler l'effet d'un inhibiteur d'IGF1R sur le récepteur d'IGF1, il est déterminé que l'inhibiteur inhibe suffisamment le récepteur si les taux d'IGFBP2 sont observés diminuer d'au moins 51 % au cours du temps suite à une première administration dudit inhibiteur.

4. Procédé selon la revendication 1, destiné à déterminer si un sujet mammalien qui souffre d'un cancer a un cancer qui répond à un inhibiteur d'IGF1R.

5. Procédé selon la revendication 1, destiné à évaluer une dose d'un inhibiteur d'IGF1R au cours du temps chez un sujet mammalien qui souffre d'un cancer.

6. Procédé selon la revendication 5, dans lequel pour évaluer une dose d'un inhibiteur d'IGF1R au cours du temps chez un sujet mammalien qui souffre d'un cancer, la dose est sélectionnée s'il est déterminé que ladite dose est suffisante.

7. Procédé selon la revendication 1, dans lequel l'inhibiteur d'IGF1R est un anticorps ou un fragment de celui-ci se liant à l'antigène qui se lie spécifiquement à IGF1R.

8. Procédé selon la revendication 7, dans lequel l'anticorps ou le fragment comprend une immunoglobuline à chaîne légère comprenant CDR-L1 comprenant la séquence d'acides aminés RASQSIGSSLH (SEQ ID NO: 99); CDR-L2 comprenant la séquence d'acides aminés YASQSLS (SEQ ID NO: 100) ou CDR-L3 comprenant la séquence d'acides aminés HQSSRLPHT (SEQ ID NO: 101); ou bien dans laquelle l'anticorps ou le fragment comprend une immunoglobuline à chaîne lourde comprenant CDR-H1 comprenant la séquence d'acides aminés SFAMH (SEQ ID NO: 102) ou GFTFSSFAMH (SEQ ID NO: 107); CDR-H2 comprenant la séquence d'acides aminés VIDTRGATYYADSVKG (SEQ ID NO: 103) ou CDR-H3 comprenant la séquence d'acides aminés LGNFYYGMDV (SEQ ID NO: 104), ou une composition pharmaceutique de celui-ci qui comprend un véhicule pharmaceutiquement acceptable; ou bien dans laquelle l'anticorps ou le fragment de celui-ci comprend un fragment mature d'une immunoglobuline à chaîne légère qui comprend la séquence d'acides aminés telle que présentée dans les SEQ ID NO: 2, 4, 6 ou 8 ou un fragment mature d'une immunoglobuline à chaîne lourde qui comprend la séquence d'acides aminés telle que présentée dans les SEQ ID NO: 10 ou 12; ou une composition pharmaceutique de celui-ci qui comprend un véhicule pharmaceutiquement acceptable.

9. Procédé selon la revendication 1, dans lequel le cancer est sélectionné parmi le groupe consistant en: ostéosarcome, rhabdomyosarcome, neuroblastome, cancer de l'enfant, cancer du rein, leucémie, cancer du rein à cellules transitionnelles, cancer de la vessie, cancer de Wilm, cancer de l'ovaire, cancer du pancréas, cancer du sein, cancer de la prostate, cancer des os, cancer du poumon, cancer de l'estomac, cancer colorectal, cancer cervical, sarcome synovial, tumeurs sécrétant le peptide intestinal vasoactif, cancer de la tête et du cou, carcinome à cellules squameuses, myélome multiple, plasmacytome solitaire, cancer des cellules rénales, rétinoblastome, tumeurs des cellules germinales, hépatoblastome, carcinome hépatocellulaire, mélanome, tumeur rhabdoïde du rein, sarcome d'Ewing, chondrosarcome, malignité hématologique, leucémie lymphoblastique chronique, leucémie myélomonocytaire chronique, leucémie lymphoblastique aiguë, leucémie lymphocytaire aiguë, leucémie myéloïde aiguë, leucémie myéloblastique aiguë, leucémie myéloblastique chronique, maladie de Hodgkin, lymphome non hodgkinien, leucémie lymphocytaire chronique, leucémie myéloïde chronique, leucémie à lymphocytes villeux, leucémie à mastocytes, néoplasme à mastocytes, lymphome folliculaire, lymphome disséminé à grandes cellules, lymphome à cellules du manteau, lymphome de Burkitt, mycose fongoïde, lymphome cutané à cellules T, troubles myéloprolifératifs chroniques, tumeur du système nerveux central, cancer du cerveau, glioblastome, cancer du cerveau non à glioblastome, méningiome, adénome pituitaire, schwannome vestibulaire, tumeur neuroectodermique primitive, médulloblastome, astrocytome, astrocytome anaplasique, oligodendrogliome, épendymome, un trouble myéloprolifératif, sarcome des tissus mous, cancer de la thyroïde, cancer de l'endomètre, cancer carcinoïde, tumeurs des cellules germinales, cancer du foie.

10. Procédé selon la revendication 1, dans lequel un ou plusieurs éléments sélectionnés parmi le groupe consistant en évérolimus, trabectédine, abraxane, TLK 286, AV-299, DN-101, pazopanib, GSK690693, RTA 744, ON 0910.Na, AZD 6244, AMN-107, TKI-258, GSK461364, AZD 1152, enzastaurine, vandétanib, ARQ-197, MK-0457, MLN8054, PHA-739358, R-763, AT-9263, un inhibiteur de FLT-3, un inhibiteur de VEGFR, un inhibiteur de EGFR TK, un inhibiteur d'aurora kinase, un modulateur de PIK-1, un inhibiteur de Bcl-2, un inhibiteur de HDAC, un inhibiteur de c-MET, un inhibiteur de PARP, un inhibiteur de Cdk, un anticorps anti-HGF, un inhibiteur de PI 3-kinase, un inhibiteur d'AKT, un inhibiteur de JAK/STAT, un inhibiteur de checkpoint 1 ou 2, un inhibiteur de la kinase d'adhésion focale, un inhibiteur de Map kinase, un récepteur piège pour le VEGF, pémétrexed, erlotinib, dasatanib, nilotinib, décatanib, panitumumab, amrubicine, orégovomab, LEP-ETU, nolatrexed, azd2171, batabuline, ofatumumab, zanolimumab, édotécarine, tétrandrine, rubitécan, tesmilifène, oblimersen, ticilimumab, ipilimumab, gossypol, Bio 111, 131-I-TM-601, ALT-110, BIO 140, CC 8490, cilengitide, gimatécan, IL13-PE38QQR, INO 1001, IPdR, KRX-0402, lucanthone, LY 317615, neuradiab, vitespan, Rta 744, Sdx 102, talampanel, atrasentan, Xr 311, romidepsin, ADS-100380, 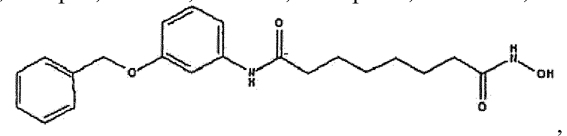 CG-781, CG-1521, 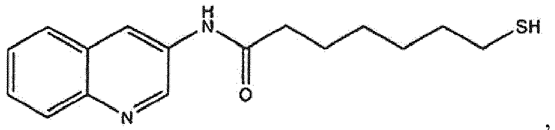 SB-556629, chlamydocine, JNJ-16241199, 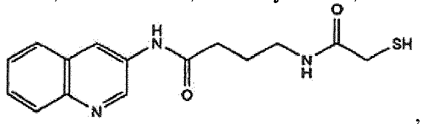 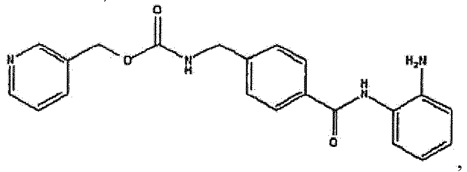 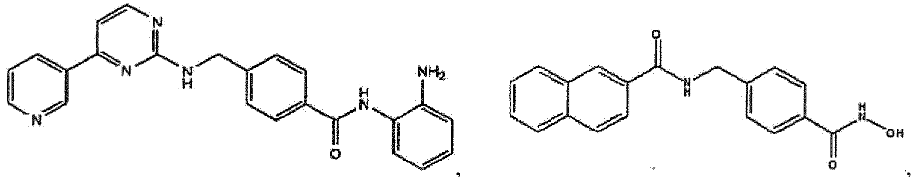 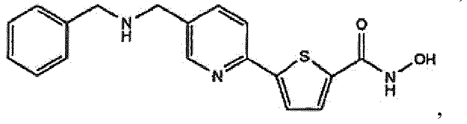 voronistat, étoposide, gemcitabine, doxorubicine, doxorubicine liposomique, 5'-désoxy-5-fluorouridine, vincristine, témozolomide, ZK-304709, séliciclib; PD0325901, AZD-6244, capécitabine, acide L-glutamique, N-[4-[2-(2-amino-4,7-dihydro-4-oxo-1H-pyrrolo[2,3-d]pyrimidin-5-yl)éthyl]benzoyle]-, sel disodique, heptahydrate, camptothécine, irinotécan; une combinaison d'irinotécan, de 5-fluorouracile et de leucovorine; irinotécan marqué au PEG, régime FOLFOX, tamoxifène, citrate de torémifène, anastrazole, exémestane, létrozole, DES (diéthylstilbestrol), estradiol, oestrogène, oestrogène conjugué, bévacizumab, IMC-IC11, CHIR-258, 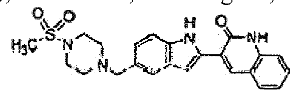 ); 3-[5-(méthylsulfonylpipéradineméthyl)-indolyl]-quinolone, vatalanib, AG-013736, AVE-0005, acétate de gosériline, acétate de leuprolide, pamoate de triptoréline, sunitinib, malate de sunitinib, acétate de médroxyprogestérone, caproate d'hydroxyprogestérone, acétate de mégestrol, raloxifène, bicalutamide, flutamide, nilutamide, acétate de mégestrol, CP-724714; TAK-165, HKI-272, erlotinib, lapatanib, canertinib, anticorps ABX-EGF, Erbitux, EKB-569, PKI-166, GW-572016, lonafarnib, 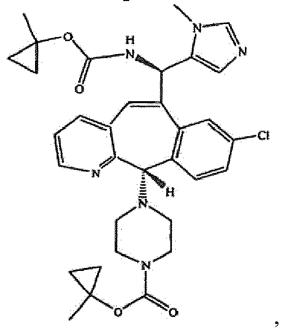 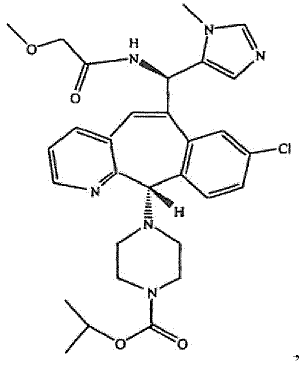 BMS-214662, tipifarnib; amifostine, NVP-LAQ824, acide suberoylanalide hydroxamique, acide valproïque, trichostatine A, FK-228, SU11248, sorafénib, KRN951, aminoglutéthimide, amsacrine, anagrélide, L-asparaginase, vaccin à bacille de Calmette et Guérin (BCG), bléomycine, buséréline, busulfan, carboplatine, carmustine, chlorambucil, cisplatine, cladribine, clodronate, cyclophosphamide, cyprotérone, cytarabine, dacarbazine, dactinomycine, daunorubicine, diéthylstilbestrol, épirubicine, fludarabine, fludrocortisone, fluoxymestérone, flutamide, hydroxyurée, idarubicine, ifosfamide, imatinib, leucovorine, leuprolide, lévamisole, lomustine, méchloréthamine, melphalan, 6-mercaptopurine, mesna, méthotrexate, mitomycine, mitotane, mitoxantrone, nilutamide, octréotide, oxaliplatine, pamidronate, pentostatine, plicamycine, porfimer, procarbazine, raltitrexed, rituximab, streptozocine, téniposide, testostérone, thalidomide, thioguanine, thiotépa, trétinoïne, vindésine, acide13-cis-rétinoïque, moutarde à la phénylalanine, moutarde à l'uracile, estramustine, altrétamine, floxuridine, 5-désoxyuridine, cytosine arabinoside, 6-mercaptopurine, désoxycoformycine, calcitriol, valrubicine, mithramycine, vinblastine, vinorelbine, topotécan, razoxine, marimastat, COL-3, néovastat, BMS-275291, squalamine, endostatine, SU5416, SU6668, EMD121974, interleukine-12, IM862, angiostatine, vitaxine, droloxifène, idoxyfène, spironolactone, finastéride, cimitidine, trastuzumab, dénileukine diftitox, géfitinib, bortézimib, paclitaxel, paclitaxel sans crémophore, docétaxel, épithilone B, BMS-247550, BMS-310705, droloxifène, 4-hydroxytamoxifène, pipendoxifène, ERA-923, arzoxifène, fulvestrant, acolbifène, lasofoxifène, idoxifène, TSE-424, HMR-3339, ZK186619, topotécan, PTK787/ZK 222584, VX-745, PD 184352, rapamycine, 40-O-(2-hydroxyéthyl)-rapamycine, temsirolimus, AP-23573, RAD001, ABT-578, BC-210, LY294002, LY292223, LY292696, LY293684, LY293646, wortmannin, ZM336372, L-779,450, PEG-filgrastim, darbépoétine, 5-fluorouracile, érythropoïétine, facteur de stimulation des colonies granulocytaires, zolendronate, prednisone, cétuximab, facteur de stimulation des colonies de macrophages granulocytaires, histréline, interféron alpha 2a pégylé, interféron alpha 2a, interféron alpha 2b pégylé, interféron alpha 2b, azacitidine, PEG-L-asparaginase, lénalidomide, gemtuzumab, hydrocortisone, interleukine 11, dexrazoxane, alemtuzumab, acide tout-transrétinoïque, kétoconazole, interleukine 2, mégestrol, globuline immune, moutarde azotée, méthylprednisolone, ibritgumomab tiuxétan, androgènes, décitabine, hexaméthylmélamine, bexarotène, tositumomab, trioxyde d'arsénic, cortisone, éditronate, mitotane, cyclosporine, daunorubicine liposomique, Edwina-asparaginase, strontium 89, casopitant, nétupitant, un antagoniste du récepteur de NK-1, palonosétron, aprépitant, diphenhydramine, hydroxyzine, métoclopramide, lorazépam, alprazolam, halopéridol, dropéridol, dronabinol, déxaméthasone, méthylprednisolone, prochlorpérazine, granisétron, ondansétron, dolasétron, tropisétron, pegfilgrastim, erythropoïétine, époétine alfa et darbépoétine alfa, sont aussi administrés au sujet.

11. Procédé selon la revendication 8, dans lequel l'inhibiteur est un anticorps comprenant une région variable d'immunoglobuline à chaîne légère comprenant CDR-L1, comprenant la séquence d'acides aminés RASQSIGSSLH (SEQ ID NO: 99); CDR-L2, comprenant la séquence d'acides aminés YASQSLS (SEQ ID NO: 100) et CDR-L3, comprenant la séquence d'acides aminés HQSSRLPHT (SEQ ID NO: 101), et une région variable d'immunoglobuline à chaîne lourde comprenant CDR-H1, comprenant la séquence d'acides aminés SFAMH (SEQ ID NO: 102) ou GFTFSSFAMH (SEQ ID NO: 107); CDR-H2, comprenant la séquence d'acides aminés VIDTRGATYYADSVKG (SEQ ID NO: 103) et CDR-H3, comprenant la séquence d'acides aminés LGNFYYGMDV (SEQ ID NO: 104).

12. Procédé selon la revendication 7, dans lequel l'inhibiteur est un anticorps comprenant une région variable de chaîne légère d'immunoglobuline comprenant la séquence d'acides aminés telle que présentée dans la SEQ ID NO: 38, et une région variable de chaîne lourde d'immunoglobuline comprenant la séquence d'acides aminés telle que présentée dans la SEQ ID NO: 40.

13. Procédé selon la revendication 11, dans lequel l'anticorps comprend une région variable d'immunoglobuline à chaîne légère comprenant les acides aminés 20 à 128 de la séquence d'acides aminés telle que présentée dans la SEQ ID NO: 8, et une région variable d'immunoglobuline à chaîne lourde comprenant les acides aminés 20 à 137 de la séquence d'acides aminés telle que présentée dans la SEQ ID NO: 10.

14. Procédé selon l'une quelconque des revendications précédentes, dans lequel le cancer est un cancer colorectal ou un cancer du poumon.

15. Procédé selon l'une quelconque des revendications précédentes, dans lequel le sujet est un être humain.

16. Procédé selon l'une quelconque des revendications précédentes, dans lequel le taux d'IGFBP2 est mesuré par un dosage radioimmunologique (RIA), par la technique Western Blot ou par un dosage par immunosorbant lié à une enzyme (ELISA).


### SEQUENCE LISTING

<110> Schering Corporation
<120> IGFBP22 Biomarker
<130> P1622 EP/1 S3
<150> 60/818,004
   <151> 2006-06-30
<160> 107
<170> PatentIn version 3.3
<210> 1
   <211> 384
   <212> DNA
   <213> Artificial Sequence
<220>
   <221> Source
   <223> /note= "Description of Artificial Sequence: light chain C"
<220>
   <221> CDS
   <222> (1)..(384)
<400> 1
   atg tcg cca tca caa ctc att ggg ttt ctg ctg ctc tgg gtt cca gcc      48
   Met Ser Pro Ser Gln Leu Ile Gly Phe Leu Leu Leu Trp Val Pro Ala
   1               5                   10                  15
   tcc agg ggt gaa att gtg ctg act cag agc cca gac tct ctg tct gtg      96
   Ser Arg Gly Glu Ile Val Leu Thr Gln Ser Pro Asp Ser Leu Ser Val
               20                  25                  30
   act cca ggc gag aga gtc acc atc acc tgc cgg gcc agt cag agc att     144
   Thr Pro Gly Glu Arg Val Thr Ile Thr Cys Arg Ala Ser Gln Ser Ile
           35                  40                  45
   ggt agt agc tta cac tgg tac cag cag aaa cca ggt cag tct cca aag     192
   Gly Ser Ser Leu His Trp Tyr Gln Gln Lys Pro Gly Gln Ser Pro Lys
       50                  55                  60
   ctt ctc atc aag tat gca tcc cag tcc ctc tca ggg gtc ccc tcg agg     240
   Leu Leu Ile Lys Tyr Ala Ser Gln Ser Leu Ser Gly Val Pro Ser Arg
   65                  70                  75                  80
   ttc agt ggc agt gga tct ggg aca gat ttc acc ctc acc atc agt agc     288
   Phe Ser Gly Ser Gly Ser Gly Thr Asp Phe Thr Leu Thr Ile Ser Ser
                   85                  90                  95
   ctc gag gct gaa gat gct gca gcg tat tac tgt cat cag agt agt cgt     336
   Leu Glu Ala Glu Asp Ala Ala Ala Tyr Tyr Cys His Gln Ser Ser Arg
               100                 105                 110
   tta cct cac act ttc ggc caa ggg acc aag gtg gag atc aaa cgt acg     384
   Leu Pro His Thr Phe Gly Gln Gly Thr Lys Val Glu Ile Lys Arg Thr
           115                 120                 125
<210> 2
   <211> 128
   <212> PRT
   <213> Artificial Sequence
<220>
   <221> Source
   <223> /note= "Description of Artificial Sequence: Synthetic Construct"
<400> 2
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Asp Ser Leu Ser Val
               20                  25                  30
   Thr Pro Gly Glu Arg Val Thr Ile Thr Cys Arg Ala Ser Gln Ser Ile
           35                  40                  45
   Gly Ser Ser Leu His Trp Tyr Gln Gln Lys Pro Gly Gln Ser Pro Lys
       50                  55                  60
   Leu Leu Ile Lys Tyr Ala Ser Gln Ser Leu Ser Gly Val Pro Ser Arg
   65                  70                  75                  80
   Phe Ser Gly Ser Gly Ser Gly Thr Asp Phe Thr Leu Thr Ile Ser Ser
                   85                  90                  95
   Leu Glu Ala Glu Asp Ala Ala Ala Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 3
   <211> 384
   <212> DNA
   <213> Artificial Sequence
<220>
   <221> Source
   <223> /note= "Description of Artificial Sequence: light chain D"
<220>
   <221> CDS
   <222> (1)..(384)
<400> 3
   atg tcg cca tca caa ctc att ggg ttt ctg ctg ctc tgg gtt cca gcc       48
   Met Ser Pro Ser Gln Leu Ile Gly Phe Leu Leu Leu Trp Val Pro Ala
   1               5                   10                  15
   tcc agg ggt gaa att gtg ctg act cag agc cca gac tct ctg tct gtg       96
   Ser Arg Gly Glu Ile Val Leu Thr Gln Ser Pro Asp Ser Leu Ser Val
               20                  25                  30
   act cca ggc gag aga gtc acc atc acc tgc cgg gcc agt cag agc att      144
   Thr Pro Gly Glu Arg Val Thr Ile Thr Cys Arg Ala Ser Gln Ser Ile
           35                  40                  45
   ggt agt agc tta cac tgg tac cag cag aaa cca ggt cag tct cca aag      192
   Gly Ser Ser Leu His Trp Tyr Gln Gln Lys Pro Gly Gln Ser Pro Lys
       50                  55                  60
   ctt ctc atc aag tat gca tcc cag tcc ctc tca ggg gtc ccc tcg agg      240
   Leu Leu Ile Lys Tyr Ala Ser Gln Ser Leu Ser Gly Val Pro Ser Arg
   65                  70                  75                  80
   ttc agt ggc agt gga tct ggg aca gat ttc acc ctc acc atc agt agc      288
   Phe Ser Gly Ser Gly Ser Gly Thr Asp Phe Thr Leu Thr Ile Ser Ser
                   85                  90                  95
   ctc gag gct gaa gat ttc gca gtg tat tac tgt cat cag agt agt cgt      336
   Leu Glu Ala Glu Asp Phe Ala Val Tyr Tyr Cys His Gln Ser Ser Arg
               100                 105                 110
   tta cct cac act ttc ggc caa ggg acc aag gtg gag atc aaa cgt acg      384
   Leu Pro His Thr Phe Gly Gln Gly Thr Lys Val Glu Ile Lys Arg Thr
           115                 120                 125
<210> 4
   <211> 128
   <212> PRT
   <213> Artificial Sequence
<220>
   <221> Source
   <223> /note= "Description of Artificial Sequence: Synthetic Construct"
<400> 4
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Asp Ser Leu Ser Val
               20                  25                  30
   Thr Pro Gly Glu Arg Val Thr Ile Thr Cys Arg Ala Ser Gln Ser Ile
           35                  40                  45
   Gly Ser Ser Leu His Trp Tyr Gln Gln Lys Pro Gly Gln Ser Pro Lys
       50                  55                  60
   Leu Leu Ile Lys Tyr Ala Ser Gln Ser Leu Ser Gly Val Pro Ser Arg
   65                  70                  75                  80
   Phe Ser Gly Ser Gly Ser Gly Thr Asp Phe Thr Leu Thr Ile Ser Ser
                   85                  90                  95
   Leu Glu Ala Glu Asp Phe Ala Val Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 5
   <211> 384
   <212> DNA
   <213> Artificial Sequence
<220>
   <221> Source
   <223> /note= "Description of Artificial Sequence: light chain E"
<220>
   <221> CDS
   <222> (1)..(384)
<400> 5
   atg tcg cca tca caa ctc att ggg ttt ctg ctg ctc tgg gtt cca gcc          48
   Met Ser Pro Ser Gln Leu Ile Gly Phe Leu Leu Leu Trp Val Pro Ala
   1               5                   10                  15
   tcc agg ggt gaa att gtg ctg act cag agc cca ggt acc ctg tct gtg          96
   Ser Arg Gly Glu Ile Val Leu Thr Gln Ser Pro Gly Thr Leu Ser Val
               20                  25                  30
   tct cca ggc gag aga gcc acc ctc tcc tgc cgg gcc agt cag agc att         144
   Ser Pro Gly Glu Arg Ala Thr Leu Ser Cys Arg Ala Ser Gln Ser Ile
           35                  40                  45
   ggt agt agc tta cac tgg tac cag cag aaa cca ggt cag gct cca agg         192
   Gly Ser Ser Leu His Trp Tyr Gln Gln Lys Pro Gly Gln Ala Pro Arg
       50                  55                  60
   ctt ctc atc aag tat gca tcc cag tcc ctc tca ggg atc ccc gat agg         240
   Leu Leu Ile Lys Tyr Ala Ser Gln Ser Leu Ser Gly Ile Pro Asp Arg
   65                  70                  75                  80
   ttc agt ggc agt gga tct ggg aca gat ttc acc ctc acc atc agt aga         288
   Phe Ser Gly Ser Gly Ser Gly Thr Asp Phe Thr Leu Thr Ile Ser Arg
                   85                  90                  95
   ctg gag cct gaa gat gct gca gcg tat tac tgt cat cag agt agt cgt         336
   Leu Glu Pro Glu Asp Ala Ala Ala Tyr Tyr Cys His Gln Ser Ser Arg
               100                 105                 110
   tta cct cac act ttc ggc caa ggg acc aag gtg gag atc aaa cgt aca         384
   Leu Pro His Thr Phe Gly Gln Gly Thr Lys Val Glu Ile Lys Arg Thr
           115                 120                 125
<210> 6
   <211> 128
   <212> PRT
   <213> Artificial Sequence
<220>
   <221> Source
   <223> /note= "Description of Artificial Sequence: Synthetic Construct"
<400> 6
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Gly Thr Leu Ser Val
               20                  25                  30
   Ser Pro Gly Glu Arg Ala Thr Leu Ser Cys Arg Ala Ser Gln Ser Ile
           35                  40                  45
   Gly Ser Ser Leu His Trp Tyr Gln Gln Lys Pro Gly Gln Ala Pro Arg
       50                  55                  60
   Leu Leu Ile Lys Tyr Ala Ser Gln Ser Leu Ser Gly Ile Pro Asp Arg
   65                  70                  75                  80
   Phe Ser Gly Ser Gly Ser Gly Thr Asp Phe Thr Leu Thr Ile Ser Arg
                   85                  90                  95
   Leu Glu Pro Glu Asp Ala Ala Ala Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 7
   <211> 384
   <212> DNA
   <213> Artificial Sequence
<220>
   <221> Source
   <223> /note= "Description of Artificial Sequence: light chain F"
<220>
   <221> CDS
   <222> (1)..(384)
<400> 7
   atg tcg cca tca caa ctc att ggg ttt ctg ctg ctc tgg gtt cca gcc       48
   Met Ser Pro Ser Gln Leu Ile Gly Phe Leu Leu Leu Trp Val Pro Ala
   1               5                   10                  15
   tcc agg ggt gaa att gtg ctg act cag agc cca ggt acc ctg tct gtg       96
   Ser Arg Gly Glu Ile Val Leu Thr Gln Ser Pro Gly Thr Leu Ser Val
               20                  25                  30
   tct cca ggc gag aga gcc acc ctc tcc tgc cgg gcc agt cag agc att      144
   Ser Pro Gly Glu Arg Ala Thr Leu Ser Cys Arg Ala Ser Gln Ser Ile
           35                  40                  45
   ggt agt agc tta cac tgg tac cag cag aaa cca ggt cag gct cca agg      192
   Gly Ser Ser Leu His Trp Tyr Gln Gln Lys Pro Gly Gln Ala Pro Arg
       50                  55                  60
   ctt ctc atc aag tat gca tcc cag tcc ctc tca ggg atc ccc gat agg      240
   Leu Leu Ile Lys Tyr Ala Ser Gln Ser Leu Ser Gly Ile Pro Asp Arg
   65                  70                  75                  80
   ttc agt ggc agt gga tct ggg aca gat ttc acc ctc acc atc agt aga      288
   Phe Ser Gly Ser Gly Ser Gly Thr Asp Phe Thr Leu Thr Ile Ser Arg
                   85                  90                  95
   ctg gag cct gaa gat ttc gca gtg tat tac tgt cat cag agt agt cgt      336
   Leu Glu Pro Glu Asp Phe Ala Val Tyr Tyr Cys His Gln Ser Ser Arg
               100                 105                 110
   tta cct cac act ttc ggc caa ggg acc aag gtg gag atc aaa cgt aca      384
   Leu Pro His Thr Phe Gly Gln Gly Thr Lys Val Glu Ile Lys Arg Thr
           115                 120                 125
<210> 8
   <211> 128
   <212> PRT
   <213> Artificial Sequence
<220>
   <221> Source
   <223> /note= "Description of Artificial Sequence: Synthetic Construct"
<400> 8
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Gly Thr Leu Ser Val
               20                  25                  30
   Ser Pro Gly Glu Arg Ala Thr Leu Ser Cys Arg Ala Ser Gln Ser Ile
           35                  40                  45
   Gly Ser Ser Leu His Trp Tyr Gln Gln Lys Pro Gly Gln Ala Pro Arg
       50                  55                  60
   Leu Leu Ile Lys Tyr Ala Ser Gln Ser Leu Ser Gly Ile Pro Asp Arg
   65                  70                  75                  80
   Phe Ser Gly Ser Gly Ser Gly Thr Asp Phe Thr Leu Thr Ile Ser Arg
                   85                  90                  95
   Leu Glu Pro Glu Asp Phe Ala Val Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 9
   <211> 411
   <212> DNA
   <213> Artificial Sequence
<220>
   <221> Source
   <223> /note= "Description of Artificial Sequence: heavy chain A"
<220>
   <221> CDS
   <222> (1)..(411)
<400> 9
   atg gag ttt ggg ctg agc tgg gtt ttc ctt gtt gct ata tta aaa ggt      48
   Met Glu Phe Gly Leu Ser Trp Val Phe Leu Val Ala Ile Leu Lys Gly
   1               5                   10                  15
   gtc cag tgt gag gtt cag ctg gtg cag tct ggg gga ggc ttg gta aag      96
   Val Gln Cys Glu Val Gln Leu Val Gln Ser Gly Gly Gly Leu Val Lys
               20                  25                  30
   cct ggg ggg tcc ctg aga ctc tcc tgt gca gcc tct gga ttc acc ttc     144
   Pro Gly Gly Ser Leu Arg Leu Ser Cys Ala Ala Ser Gly Phe Thr Phe
           35                  40                  45
   agt agc ttt gct atg cac tgg gtt cgc cag gct cca gga aaa ggt ctg     192
   Ser Ser Phe Ala Met His Trp Val Arg Gln Ala Pro Gly Lys Gly Leu
       50                  55                  60
   gag tgg ata tca gtt att gat act cgt ggt gcc aca tac tat gca gac     240
   Glu Trp Ile Ser Val Ile Asp Thr Arg Gly Ala Thr Tyr Tyr Ala Asp
   65                  70                  75                  80
   tcc gtg aag ggc cga ttc acc atc tcc aga gac aat gcc aag aac tcc     288
   Ser Val Lys Gly Arg Phe Thr Ile Ser Arg Asp Asn Ala Lys Asn Ser
                   85                  90                  95
   ttg tat ctt caa atg aac agc ctg aga gcc gag gac act gct gtg tat     336
   Leu Tyr Leu Gln Met Asn Ser Leu Arg Ala Glu Asp Thr Ala Val Tyr
               100                 105                 110
   tac tgt gca aga ctg ggg aac ttc tac tac ggt atg gac gtc tgg ggc     384
   Tyr Cys Ala Arg Leu Gly Asn Phe Tyr Tyr Gly Met Asp Val Trp Gly
           115                 120                 125
   caa ggg acc acg gtc acc gtc tcc tca                                 411
   Gln Gly Thr Thr Val Thr Val Ser Ser
       130                 135
<210> 10
   <211> 137
   <212> PRT
   <213> Artificial Sequence
<220>
   <221> Source
   <223> /note= "Description of Artificial Sequence: Synthetic Construct"
<400> 10
   Met Glu Phe Gly Leu Ser Trp Val Phe Leu Val Ala Ile Leu Lys Gly
   1               5                   10                  15
   Val Gln Cys Glu Val Gln Leu Val Gln Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Ser Phe Ala Met His Trp Val Arg Gln Ala Pro Gly Lys Gly Leu
       50                  55                  60
   Glu Trp Ile Ser Val Ile Asp Thr Arg Gly Ala Thr Tyr Tyr Ala Asp
   65                  70                  75                  80
   Ser Val Lys Gly Arg Phe Thr Ile Ser Arg Asp Asn Ala Lys Asn Ser
                   85                  90                  95
   Leu Tyr Leu Gln Met Asn Ser Leu Arg Ala Glu Asp Thr Ala Val Tyr
               100                 105                 110
   Tyr Cys Ala Arg Leu Gly Asn Phe Tyr Tyr Gly Met Asp Val Trp Gly
           115                 120                 125
   Gln Gly Thr Thr Val Thr Val Ser Ser
       130                 135
<210> 11
   <211> 411
   <212> DNA
   <213> Artificial Sequence
<220>
   <221> Source
   <223> /note= "Descriprion of Artificial Sequence: heavy chain B"
<220>
   <221> CDS
   <222> (1)..(411)
<400> 11
   atg gag ttt ggg ctg agc tgg gtt ttc ctt gtt gct ata tta aaa ggt      48
   Met Glu Phe Gly Leu Ser Trp Val Phe Leu Val Ala Ile Leu Lys Gly
   1               5                   10                  15
   gtc cag tgt gag gtt cag ctg gtg cag tct ggg gga ggc ttg gta cag      96
   Val Gln Cys Glu Val Gln Leu Val Gln Ser Gly Gly Gly Leu Val Gln
               20                  25                  30
   ccc ggg ggg tcc ctg aga ctc tcc tgt gca gcc tct gga ttc acc ttc      144
   Pro Gly Gly Ser Leu Arg Leu Ser Cys Ala Ala Ser Gly Phe Thr Phe
           35                  40                  45
   agt agc ttt gct atg cac tgg gtt cgc cag gct cca gga aaa ggt ctg      192
   Ser Ser Phe Ala Met His Trp Val Arg Gln Ala Pro Gly Lys Gly Leu
       50                  55                  60
   gag tgg ata tca gtt att gat act cgt ggt gcc aca tac tat gca gac      240
   Glu Trp Ile Ser Val Ile Asp Thr Arg Gly Ala Thr Tyr Tyr Ala Asp
   65                  70                  75                  80
   tcc gtg aag ggc cga ttc acc atc tcc aga gac aat gcc aag aac tcc      288
   Ser Val Lys Gly Arg Phe Thr Ile Ser Arg Asp Asn Ala Lys Asn Ser
                   85                  90                  95
   ttg tat ctt caa atg aac agc ctg aga gcc gag gac act gct gtg tat      336
   Leu Tyr Leu Gln Met Asn Ser Leu Arg Ala Glu Asp Thr Ala Val Tyr
               100                 105                 110
   tac tgt gca aga ctg ggg aac ttc tac tac ggt atg gac gtc tgg ggc      384
   Tyr Cys Ala Arg Leu Gly Asn Phe Tyr Tyr Gly Met Asp Val Trp Gly
           115                 120                 125
   caa ggg acc acg gtc acc gtc tcc tca                                  411
   Gln Gly Thr Thr Val Thr Val Ser Ser
       130                 135
<210> 12
   <211> 137
   <212> PRT
   <213> Artificial Sequence
<220>
   <221> Source
   <223> /note= "Description of Artificial Sequence: Synthetic Construct"
<400> 12
   Met Glu Phe Gly Leu Ser Trp Val Phe Leu Val Ala Ile Leu Lys Gly
   1 5 10 15
   Val Gln Cys Glu Val Gln Leu Val Gln Ser Gly Gly Gly Leu Val Gln
   20 25 30
   Pro Gly Gly Ser Leu Arg Leu Ser Cys Ala Ala Ser Gly Phe Thr Phe
   35 40 45
   Ser Ser Phe Ala Met His Trp Val Arg Gln Ala Pro Gly Lys Gly Leu
   50 55 60
   Glu Trp Ile Ser Val Ile Asp Thr Arg Gly Ala Thr Tyr Tyr Ala Asp
   65 70 75 80
   Ser Val Lys Gly Arg Phe Thr Ile Ser Arg Asp Asn Ala Lys Asn Ser
   85 90 95
   Leu Tyr Leu Gln Met Asn Ser Leu Arg Ala Glu Asp Thr Ala Val Tyr
   100 105 110
   Tyr Cys Ala Arg Leu Gly Asn Phe Tyr Tyr Gly Met Asp Val Trp Gly
   115 120 125
   Gln Gly Thr Thr Val Thr Val Ser Ser
   130 135
<210> 13
   <211> 174
   <212> PRT
   <213> Artificial Sequence
<220>
   <221> Source
   <223> /note= "Description of Artificial Sequence: immunoglobulin heavy chain variable region"
<400> 13
   Gly Arg Leu Gly Gln Ala Trp Arg Ser Leu Arg Leu Ser Cys Ala Ala
   1               5                   10                  15
   Ser Gly Phe Thr Phe Ser Asp Tyr Tyr Met Ser Trp Ile Arg Gln Ala
               20                  25                  30
   Pro Gly Lys Gly Leu Glu Trp Val Ser Tyr Ile Ser Ser Ser Gly Ser
           35                  40                  45
   Thr Arg Asp Tyr Ala Asp Ser Val Lys Gly Arg Phe Thr Ile Ser Arg
       50                  55                  60
   Asp Asn Ala Lys Asn Ser Leu Tyr Leu Gln Met Asn Ser Leu Arg Ala
   65                  70                  75                  80
   Glu Asp Thr Ala Val Tyr Tyr Cys Val Arg Asp Gly Val Glu Thr Thr
                   85                  90                  95
   Phe Tyr Tyr Tyr Tyr Tyr Gly Met Asp Val Trp Gly Gln Gly Thr Thr
               100                 105                 110
   Val Thr Val Ser Ser Ala Ser Thr Lys Gly Pro Ser Val Phe Pro Leu
           115                 120                 125
   Ala Pro Cys Ser Arg Ser Thr Ser Glu Ser Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ser Cys Ala
                   165                 170
<210> 14
   <211> 124
   <212> PRT
   <213> Artificial Sequence
<220>
   <221> Source
   <223> /note= "Description of Artificial Sequence: immunoglobulin heavy chain variable region"
<400> 14
   Val Gln Leu Leu Glu Ser Gly Gly Gly Leu Val Gln Pro Gly Gly Ser
   1               5                   10                  15
   Leu Arg Leu Ser Cys Thr Ala Ser Gly Phe Thr Phe Ser Ser Tyr Ala
               20                  25                  30
   Met Asn Trp Val Arg Gln Ala Pro Gly Lys Gly Leu Glu Trp Val Ser
           35                  40                  45
   Ala Ile Ser Gly Ser Gly Gly Thr Thr Phe Tyr Ala Asp Ser Val Lys
       50                  55                  60
   Gly Arg Phe Thr Ile Ser Arg Asp Asn Ser Arg Thr Thr Leu Tyr Leu
   65                  70                  75                  80
   Gln Met Asn Ser Leu Arg Ala Glu Asp Thr Ala Val Tyr Tyr Cys Ala
                   85                  90                  95
   Lys Asp Leu Gly Trp Ser Asp Ser Tyr Tyr Tyr Tyr Tyr Gly Met Asp
               100                 105                 110
   Val Trp Gly Gln Gly Thr Thr Val Thr Val Ser Ser
           115                 120
<210> 15
   <211> 112
   <212> PRT
   <213> Artificial Sequence
<220>
   <221> Source
   <223> /note= "Description of Artificial Sequence: immunoglobulin heavy chain variable region"
<400> 15
   Gly Pro Gly Leu Val Lys Pro Ser Glu Thr Leu Ser Leu Thr Cys Thr
   1               5                   10                  15
   Val Ser Gly Gly Ser Ile Ser Asn Tyr Tyr Trp Ser Trp Ile Arg Gln
   20 25 30
   Pro Ala Gly Lys Gly Leu Glu Trp Ile Gly Arg Ile Tyr Thr Ser Gly
   35 40 45
   Ser Pro Asn Tyr Asn Pro Ser Leu Lys Ser Arg Val Thr Met Ser Val
   50 55 60
   Asp Thr Ser Lys Asn Gln Phe Ser Leu Lys Leu Asn Ser Val Thr Ala
   65 70 75 80
   Ala Asp Thr Ala Val Tyr Tyr Cys Ala Val Thr Ile Phe Gly Val Val
   85 90 95
   Ile Ile Phe Asp Tyr Trp Gly Gln Gly Thr Leu Val Thr Val Ser Ser
   100 105 110
<210> 16
   <211> 125
   <212> PRT
   <213> Artificial Sequence
<220>
   <221> Source
   <223> /note= "Description of Artificial Sequence: immunoglobulin heavy chain variable region"
<400> 16
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ile Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Leu Gly Tyr Gly Asp Phe Tyr Tyr Tyr Tyr Tyr Gly Met
   100 105 110
   Asp Val Trp Gly Gln Gly Thr Thr Val Thr Val Ser Ser
   115 120 125
<210> 17
   <211> 113
   <212> PRT
   <213> Artificial Sequence
<220>
   <221> Source
   <223> /note= "Description of Artificial Sequence: immunoglobulin heavy chain variable region"
<400> 17
   Pro Gly Leu Val Lys Pro Ser Glu Thr Leu Ser Leu Thr Cys Thr Val
   1 5 10 15
   Ser Gly Gly Ser Ile Ser Ser Tyr Tyr Trp Ser Trp Ile Arg Gln Pro
   20 25 30
   Pro Gly Lys Gly Leu Glu Trp Ile Gly Tyr Ile Tyr Tyr Ser Gly Ser
   35 40 45
   Thr Asn Tyr Asn Pro Ser Leu Lys Ser Arg Val Thr Ile Ser Val Asp
   50 55 60
   Thr Ser Lys Asn Gln Phe Ser Leu Lys Leu Ser Ser Val Thr Ala Ala
   65 70 75 80
   Asp Thr Ala Val Tyr Tyr Cys Ala Arg Thr Tyr Ser Ser Ser Phe Tyr
   85 90 95
   Tyr Tyr Gly Met Asp Val Trp Gly Gln Gly Thr Thr Val Thr Val Ser
   100 105 110
   Ser
<210> 18
   <211> 122
   <212> PRT
   <213> Artificial Sequence
<220>
   <221> Source
   <223> /note= "Description of Artificial Sequence: immunoglobulin heavy chain variable region"
<400> 18
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Gly Ile Thr Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Pro Gly Thr Thr Val Ile Met Ser Trp Phe Asp Pro Trp
   100 105 110
   Gly Gln Gly Thr Leu Val Thr Val Ser Ser
   115 120
<210> 19
   <211> 136
   <212> PRT
   <213> Artificial Sequence
<220>
   <221> Source
   <223> /note= "Description of Artificial Sequence: immunoglobulin light chain variable region"
<400> 19
   Ala Ser Val Gly Asp Arg Val Thr Phe Thr Cys Arg Ala Ser Gln Asp
   1 5 10 15
   Ile Arg Arg Asp Leu Gly Trp Tyr Gln Gln Lys Pro Gly Lys Ala Pro
   20 25 30
   Lys Arg Leu Ile Tyr Ala Ala Ser Arg Leu Gln Ser Gly Val Pro Ser
   35 40 45
   Arg Phe Ser Gly Ser Gly Ser Gly Thr Glu Phe Thr Leu Thr Ile Ser
   50 55 60
   Ser Leu Gln Pro Glu Asp Phe Ala Thr Tyr Tyr Cys Leu Gln His Asn
   65                  70                  75                  80
   Asn Tyr Pro Arg Thr Phe Gly Gln Gly Thr Glu Val Glu Ile Ile Arg
                   85                  90                  95
   Thr Val Ala Ala Pro Ser Val Phe Ile Phe Pro Pro Ser Asp Glu Gln
               100                 105                 110
   Leu Lys Ser Gly Thr Ala Ser Val Val Cys Leu Leu Asn Asn Phe Tyr
           115                 120                 125
   Pro Arg Glu Ala Lys Val Gln Trp
       130                 135
<210> 20
   <211> 107
   <212> PRT
   <213> Artificial Sequence
<220>
   <221> Source
   <223> /note= "Description of Artificial Sequence: immunoglobulin light chain variable region"
<400> 20
<210> 21
   <211> 100
   <212> PRT
   <213> Artificial Sequence
<220>
   <221> Source
   <223> /note= "Description of Artificial Sequence: immunoglobulin light chain variable region"
<400> 21
   Ser Ser Leu Ser Ala Ser Val Gly Asp Arg Val Thr Phe Thr Cys Arg
   1               5                   10                  15
   Ala Ser Gln Asp Ile Arg Arg Asp Leu Gly Trp Tyr Gln Gln Lys Pro
               20                  25                  30
   Gly Lys Ala Pro Lys Arg Leu Ile Tyr Ala Ala Ser Arg Leu Gln Ser
           35                  40                  45
   Gly Val Pro Ser Arg Phe Ser Gly Ser Gly Ser Gly Thr Glu Phe Thr
       50                  55                  60
   Leu Thr Ile Ser Ser Leu Gln Pro Glu Asp Phe Ala Thr Tyr Tyr Cys
   65                  70                  75                  80
   Leu Gln His Asn Asn Tyr Pro Arg Thr Phe Gly Gln Gly Thr Glu Val
                   85                  90                  95
   Glu Ile Ile Arg
               100
<210> 22
   <211> 107
   <212> PRT
   <213> Artificial Sequence
<220>
   <221> Source
   <223> /note= "Description of Artificial Sequence: immunoglobulin light chain variable region"
<400> 22
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Ser Asp
               20                  25                  30
   Leu Gly Trp Phe Gln Gln Lys Pro Gly Lys Ala Pro Lys Arg Leu Ile
           35                  40                  45
   Tyr Ala Ala Ser Lys Leu His Arg Gly Val Pro Ser Arg Phe Ser Gly
       50                  55                  60
   Ser Gly Ser Gly Thr Glu Phe Thr Leu Thr Ile Ser Arg Leu Gln Pro
   65                  70                  75                  80
   Glu Asp Phe Ala Thr Tyr Tyr Cys Leu Gln His Asn Ser Tyr Pro Leu
                   85                  90                  95
   Thr Phe Gly Gly Gly Thr Lys Val Glu Ile Lys
               100                 105
<210> 23
   <211> 92
   <212> PRT
   <213> Artificial Sequence
<220>
   <221> Source
   <223> /note= "Description of Artificial Sequence: immunoglobulin light chain variable region"
<400> 23
   Gly Asp Arg Val Thr Ile Thr Cys Arg Ala Ser Gln Ser Ile Ser Thr
   1               5                   10                  15
   Phe Leu Asn Trp Tyr Gln Gln Lys Pro Gly Lys Ala Pro Lys Leu Leu
               20                  25                  30
   Ile His Val Ala Ser Ser Leu Gln Gly Gly Val Pro Ser Arg Phe Ser
           35                  40                  45
   Gly Ser Gly Ser Gly Thr Asp Phe Thr Leu Thr Ile Ser Ser Leu Gln
       50                  55                  60
   Pro Glu Asp Phe Ala Thr Tyr Tyr Cys Gln Gln Ser Tyr Asn Ala Pro
   65                  70                  75                  80
   Leu Thr Phe Gly Gly Gly Thr Lys Val Glu Ile Lys
                   85                  90
<210> 24
   <211> 91
   <212> PRT
   <213> Artificial Sequence
<220>
   <221> Source
   <223> /note= "Description of Artificial Sequence: immunoglobulin light chain variable region"
<400> 24
   Arg Ala Thr Leu Ser Cys Arg Ala Ser Gln Ser Val Arg Gly Arg Tyr
   1               5                   10                  15
   Leu Ala Trp Tyr Gln Gln Lys Pro Gly Gln Ala Pro Arg Leu Leu Ile
               20                  25                  30
   Tyr Gly Ala Ser Ser Arg Ala Thr Gly Ile Pro Asp Arg Phe Ser Gly
           35                  40                  45
   Ser Gly Ser Gly Thr Asp Phe Thr Leu Thr Ile Ser Arg Leu Glu Pro
       50                  55                  60
   Glu Asp Phe Ala Val Phe Tyr Cys Gln Gln Tyr Gly Ser Ser Pro Arg
   65                  70                  75                  80
   Thr Phe Gly Gln Gly Thr Lys Val Glu Ile Lys
                   85                  90
<210> 25
   <211> 236
   <212> PRT
   <213> Artificial Sequence
<220>
   <221> Source
   <223> /note= "Description of Artificial Sequence: light chain immunoglobulin"
<400> 25
   Met Asp Met Arg Val Pro Ala Gln Leu Leu Gly Leu Leu Leu Leu Trp
   1               5                   10                  15
   Phe Pro Gly Ala Arg Cys Asp Ile Gln Met Thr Gln Ser Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Asp Leu Gly Trp Tyr Gln Gln Lys Pro Gly Lys
       50                  55                  60
   Ala Pro Lys Arg Leu Ile Tyr Ala Ala Ser Ser Leu Gln Ser Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Leu Gln
               100                 105                 110
   His Asn Ser Tyr Pro Trp Thr Phe Gly Gln Gly Thr Lys Val Glu Ile
   115 120 125
   Lys Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 26
   <211> 236
   <212> PRT
   <213> Artificial Sequence
<220>
   <221> Source
   <223> /note= "Description of Artificial Sequence: light chain immunoglobulin"
<400> 26
   Met Asp Met Arg Val Pro Ala Gln Leu Leu Gly Leu Leu Leu Leu Trp
   1 5 10 15
   Phe Pro Gly Ala Arg Cys Asp Ile Gln Met Thr Gln Ser Pro Ser Ser
   20 25 30
   Leu Ser Ala Ser Val Gly Asp Arg Val Thr Phe Thr Cys Arg Ala Ser
   35 40 45
   Gln Asp Ile Arg Arg Asp Leu Gly Trp Tyr Gln Gln Lys Pro Gly Lys
   50 55 60
   Ala Pro Lys Arg Leu Ile Tyr Ala Ala Ser Arg Leu Gln Ser Gly Val
   65 70 75 80
   Pro Ser Arg Phe Ser Gly Ser Gly Ser Gly Thr Glu Phe Thr Leu Thr
   85 90 95
   Ile Ser Ser Leu Gln Pro Glu Asp Phe Ala Thr Tyr Tyr Cys Leu Gln
   100 105 110
   His Asn Asn Tyr Pro Arg Thr Phe Gly Gln Gly Thr Glu Val Glu Ile
   115 120 125
   Ile Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 27
   <211> 236
   <212> PRT
   <213> Artificial Sequence
<220>
   <221> Source
   <223> /note= "Description of Artificial Sequence: light chain immunoglobulin"
<400> 27
   Met Asp Met Arg Val Pro Ala Gln Leu Leu Gly Leu Leu Leu Leu Trp
   1               5                   10                  15
   Phe Pro Gly Ala Arg Cys Asp Ile Gln Met Thr Gln Ser Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Asp Leu Gly Trp Tyr Gln Gln Lys Pro Gly Lys
       50                  55                  60
   Ala Pro Lys Arg Leu Ile Tyr Ala Ala Ser Ser Leu Gln Ser Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Leu Gln
               100                 105                 110
   His Asn Ser Tyr Pro Tyr Thr Phe Gly Gln Gly Thr Lys Leu Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 28
   <211> 236
   <212> PRT
   <213> Artificial Sequence
<220>
   <221> Source
   <223> /note= "Description of Artificial Sequence: light chain immunoglobulin"
<400> 28
   Met Asp Met Arg Val Pro Ala Gln Leu Leu Gly Leu Leu Leu Leu Trp
   1               5                   10                  15
   Phe Pro Gly Ala Arg Cys Asp Ile Gln Met Thr Gln Phe Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Asp Leu Gly Trp Tyr Gln Gln Lys Pro Gly Lys
       50                  55                  60
   Ala Pro Lys Arg Leu Ile Tyr Ala Ala Ser Arg Leu His Arg Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Leu Gln
               100                 105                 110
   His Asn Ser Tyr Pro Cys Ser Phe Gly Gln Gly Thr Lys Leu Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
       210                 215                 220
   Ser Pro Val Thr Lys Ser Phe Asn Arg Gly Glu Cys
   225                 230                 235
<210> 29
   <211> 473
   <212> PRT
   <213> Artificial Sequence
<220>
   <221> Source
   <223> /note= "Description of Artificial Sequence: heavy chain immunoglobulin"
<400> 29
   Met Glu Phe Gly Leu Ser Trp Val Phe Leu Val Ala Ile Ile Lys Gly
   1               5                   10                  15
   Val Gln Cys Gln Val Gln Leu Val Glu Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Asp Tyr Tyr Met Ser Trp Ile Arg Gln Ala Pro Gly Lys Gly Leu
       50                  55                  60
   Glu Trp Val Ser Tyr Ile Ser Ser Ser Gly Ser Thr Ile Tyr Tyr Ala
   65                  70                  75                  80
   Asp Ser Val Lys Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
               100                 105                 110
   Tyr Tyr Cys Ala Arg Val Leu Arg Phe Leu Glu Trp Leu Leu Tyr Tyr
           115                 120                 125
   Tyr Tyr Tyr Tyr Gly Met Asp Val Trp Gly Gln Gly Thr Thr Val Thr
       130                 135                 140
   Val Ser Ser Ala Ser Thr Lys Gly Pro Ser Val Phe Pro Leu Ala Pro
   145                 150                 155                 160
   Cys Ser Arg Ser Thr Ser Glu Ser Thr Ala Ala Leu Gly Cys Leu Val
                   165                 170                 175
   Lys Asp Tyr Phe Pro Glu Pro Val Thr Val Ser Trp Asn Ser Gly Ala
               180                 185                 190
   Leu Thr Ser Gly Val His Thr Phe Pro Ala Val Leu Gln Ser Ser Gly
           195                 200                 205
   Leu Tyr Ser Leu Ser Ser Val Val Thr Val Pro Ser Ser Asn Phe Gly
       210                 215                 220
   Thr Gln Thr Tyr Thr Cys Asn Val Asp His Lys Pro Ser Asn Thr Lys
   225                 230                 235                     240
   Val Asp Lys Thr Val Glu Arg Lys Cys Cys Val Glu Cys Pro Pro Cys
                   245                 250                 255
   Pro Ala Pro Pro Val Ala Gly Pro Ser Val Phe Leu Phe Pro Pro Lys
               260                 265                 270
   Pro Lys Asp Thr Leu Met Ile Ser Arg Thr Pro Glu Val Thr Cys Val
           275                 280                 285
   Val Val Asp Val Ser His Glu Asp Pro Glu Val Gln Phe Asn Trp Tyr
       290                 295                 300
   Val Asp Gly Val Glu Val His Asn Ala Lys Thr Lys Pro Arg Glu Glu
   305                 310                 315                     320
   Gln Phe Asn Ser Thr Phe Arg Val Val Ser Val Leu Thr Val Val His
                   325                 330                 335
   Gln Asp Trp Leu Asn Gly Lys Glu Tyr Lys Cys Lys Val Ser Asn Lys
               340                 345                 350
   Gly Leu Pro Ala Pro Ile Glu Lys Thr Ile Ser Lys Thr Lys Gly Gln
           355                 360                 365
   Pro Arg Glu Pro Gln Val Tyr Thr Leu Pro Pro Ser Arg Glu Glu Met
       370                 375                 380
   Thr Lys Asn Gln Val Ser Leu Thr Cys Leu Val Lys Gly Phe Tyr Pro
   385                 390                 395                     400
   Ser Asp Ile Ala Val Glu Trp Glu Ser Asn Gly Gln Pro Glu Asn Asn
                   405                 410                 415
   Tyr Lys Thr Thr Pro Pro Met Leu Asp Ser Asp Gly Ser Phe Phe Leu
               420                 425                 430
   Tyr Ser Lys Leu Thr Val Asp Lys Ser Arg Trp Gln Gln Gly Asn Val
           435                 440                 445
   Phe Ser Cys Ser Val Met His Glu Ala Leu His Asn His Tyr Thr Gln
       450                 455                 460
   Lys Ser Leu Ser Leu Ser Pro Gly Lys
   465 470
<210> 30
   <211> 470
   <212> PRT
   <213> Artificial Sequence
<220>
   <221> Source
   <223> /note= "Description of Artificial Sequence: heavy chain immunoglobulin"
<400> 30
   Met Glu Phe Gly Leu Ser Trp Val Phe Leu Val Ala Ile Ile Lys Gly
   1               5                   10                  15
   Val Gln Cys Gln Ala Gln Leu Val Glu Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Asp Tyr Tyr Met Ser Trp Ile Arg Gln Ala Pro Gly Lys Gly Leu
       50                  55                  60
   Glu Trp Val Ser Tyr Ile Ser Ser Ser Gly Ser Thr Arg Asp Tyr Ala
   65                  70                  75                  80
   Asp Ser Val Lys Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
               100                 105                 110
   Tyr Tyr Cys Val Arg Asp Gly Val Glu Thr Thr Phe Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Cys Ser Arg
   145                 150                 155                 160
   Ser Thr Ser Glu Ser Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
   180 185 190
   Gly Val His Thr Phe Pro Ala Val Leu Gln Ser Ser Gly Leu Tyr Ser
   195 200 205
   Leu Ser Ser Val Val Thr Val Pro Ser Ser Asn Phe Gly Thr Gln Thr
   210 215 220
   Tyr Thr Cys Asn Val Asp His Lys Pro Ser Asn Thr Lys Val Asp Lys
   225 230 235 240
   Thr Val Glu Arg Lys Cys Cys Val Glu Cys Pro Pro Cys Pro Ala Pro
   245 250 255
   Pro Val Ala Gly Pro Ser Val Phe Leu Phe Pro Pro Lys Pro Lys Asp
   260 265 270
   Thr Leu Met Ile Ser Arg Thr Pro Glu Val Thr Cys Val Val Val Asp
   275 280 285
   Val Ser His Glu Asp Pro Glu Val Gln Phe Asn Trp Tyr Val Asp Gly
   290 295 300
   Val Glu Val His Asn Ala Lys Thr Lys Pro Arg Glu Glu Gln Phe Asn
   305 310 315 320
   Ser Thr Phe Arg Val Val Ser Val Leu Thr Val Val His Gln Asp Trp
   325 330 335
   Leu Asn Gly Lys Glu Tyr Lys Cys Lys Val Ser Asn Lys Gly Leu Pro
   340 345 350
   Ala Pro Ile Glu Lys Thr Ile Ser Lys Thr Lys Gly Gln Pro Arg Glu
   355 360 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 31
   <211> 470
   <212> PRT
   <213> Artificial Sequence
<220>
   <221> Source
   <223> /note= "Description of Artificial Sequence: heavy chain immunoglobulin"
<400> 31
   Met Glu Phe Gly Leu Ser Trp Leu Phe Leu Val Ala Ile Leu Lys Gly
   1 5 10 15
   Val Gln Cys Glu Val Gln Leu Leu Glu Ser Gly Gly Gly Leu Val Gln
   20 25 30
   Pro Gly Gly Ser Leu Arg Leu Ser Cys Ala Ala Ser Gly Phe Thr Phe
   35 40 45
   Ser Ser Tyr Ala Met Ser Trp Val Arg Gln Ala Pro Gly Lys Gly Leu
   50 55 60
   Glu Trp Val Ser Ala Ile Ser Gly Ser Gly Gly Ser Thr Tyr Tyr Ala
   65 70 75 80
   Asp Ser Val Lys Gly Arg Phe Thr Ile Ser Arg Asp Asn Ser Lys Asn
   85 90 95
   Thr Leu Tyr Leu Gln Met Asn Ser Leu Arg Ala Glu Asp Thr Ala Val
   100 105 110
   Tyr Tyr Cys Ala Lys Gly Tyr Ser Ser Gly Trp Tyr Tyr Tyr Tyr Tyr
   115 120 125
   Tyr Gly Met Asp Val Trp Gly Gln Gly Thr Thr Val Thr Val Ser Ser
   130 135 140
   Ala Ser Thr Lys Gly Pro Ser Val Phe Pro Leu Ala Pro Cys Ser Arg
   145 150 155 160
   Ser Thr Ser Glu Ser Thr Ala Ala Leu Gly Cys Leu Val Lys Asp Tyr
   165 170 175
   Phe Pro Glu Pro Val Thr Val Ser Trp Asn Ser Gly Ala Leu Thr Ser
   180 185 190
   Gly Val His Thr Phe Pro Ala Val Leu Gln Ser Ser Gly Leu Tyr Ser
   195 200 205
   Leu Ser Ser Val Val Thr Val Pro Ser Ser Asn Phe Gly Thr Gln Thr
   210 215 220
   Tyr Thr Cys Asn Val Asp His Lys Pro Ser Asn Thr Lys Val Asp Lys
   225 230 235 240
   Thr Val Glu Arg Lys Cys Cys Val Glu Cys Pro Pro Cys Pro Ala Pro
   245 250 255
   Pro Val Ala Gly Pro Ser Val Phe Leu Phe Pro Pro Lys Pro Lys Asp
   260 265 270
   Thr Leu Met Ile Ser Arg Thr Pro Glu Val Thr Cys Val Val Val Asp
   275 280 285
   Val Ser His Glu Asp Pro Glu Val Gln Phe Asn Trp Tyr Val Asp Gly
   290 295 300
   Val Glu Val His Asn Ala Lys Thr Lys Pro Arg Glu Glu Gln Phe Asn
   305 310 315 320
   Ser Thr Phe Arg Val Val Ser Val Leu Thr Val Val His Gln Asp Trp
   325 330 335
   Leu Asn Gly Lys Glu Tyr Lys Cys Lys Val Ser Asn Lys Gly Leu Pro
   340 345 350
   Ala Pro Ile Glu Lys Thr Ile Ser Lys Thr Lys Gly Gln Pro Arg Glu
   355 360 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
                   405                 410                 415
   Thr Pro Pro Met Leu Asp Ser Asp Gly Ser Phe Phe Leu Tyr Ser Lys
               420                 425                 430
   Leu Thr Val Asp Lys Ser Arg Trp Gln Gln Gly Asn Val Phe Ser Cys
           435                 440                 445
   Ser Val Met His Glu Ala Leu His Asn His Tyr Thr Gln Lys Ser Leu
       450                 455                 460
   Ser Leu Ser Pro Gly Lys
   465                 470
<210> 32
   <211> 470
   <212> PRT
   <213> Artificial Sequence
<220>
   <221> Source
   <223> /note= "Description of Artificial Sequence: heavy chain immunoglobulin"
<400> 32
   Met Glu Phe Gly Leu Ser Trp Leu Phe Leu Val Ala Ile Leu Lys Gly
   1               5                   10                  15
   Val Gln Cys Glu Val Gln Leu Leu Glu Ser Gly Gly Gly Leu Val Gln
               20                  25                  30
   Pro Gly Gly Ser Leu Arg Leu Ser Cys Thr Ala Ser Gly Phe Thr Phe
           35                  40                  45
   Ser Ser Tyr Ala Met Asn Trp Val Arg Gln Ala Pro Gly Lys Gly Leu
       50                  55                  60
   Glu Trp Val Ser Ala Ile Ser Gly Ser Gly Gly Thr Thr Phe Tyr Ala
   65                  70                  75                  80
   Asp Ser Val Lys Gly Arg Phe Thr Ile Ser Arg Asp Asn Ser Arg Thr
                   85                  90                  95
   Thr Leu Tyr Leu Gln Met Asn Ser Leu Arg Ala Glu Asp Thr Ala Val
               100                 105                 110
   Tyr Tyr Cys Ala Lys Asp Leu Gly Trp Ser Asp Ser Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Cys Ser Arg
   145                 150                 155                 160
   Ser Thr Ser Glu Ser Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Asn Phe Gly Thr Gln Thr
       210                 215                 220
   Tyr Thr Cys Asn Val Asp His Lys Pro Ser Asn Thr Lys Val Asp Lys
   225                 230                 235                 240
   Thr Val Glu Arg Lys Cys Cys Val Glu Cys Pro Pro Cys Pro Ala Pro
                   245                 250                 255
   Pro Val Ala Gly Pro Ser Val Phe Leu Phe Pro Pro Lys Pro Lys Asp
               260                 265                 270
   Thr Leu Met Ile Ser Arg Thr Pro Glu Val Thr Cys Val Val Val Asp
           275                 280                 285
   Val Ser His Glu Asp Pro Glu Val Gln Phe Asn Trp Tyr Val Asp Gly
       290                 295                 300
   Val Glu Val His Asn Ala Lys Thr Lys Pro Arg Glu Glu Gln Phe Asn
   305                 310                 315                 320
   Ser Thr Phe Arg Val Val Ser Val Leu Thr Val Val His Gln Asp Trp
                   325                 330                 335
   Leu Asn Gly Lys Glu Tyr Lys Cys Lys Val Ser Asn Lys Gly Leu Pro
               340                 345                 350
   Ala Pro Ile Glu Lys Thr Ile Ser Lys Thr Lys Gly Gln Pro Arg Glu
           355                 360                 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 33
   <211> 470
   <212> PRT
   <213> Artificial Sequence
<220>
   <221> Source
   <223> /note= "Description of Artificial Sequence: immunoglobulin heavy chain of 2.12.1 fx"
<400> 33
   Met Glu Phe Gly Leu Ser Trp Val Phe Leu Val Ala Ile Ile Lys Gly
   1               5                   10                  15
   Val Gln Cys Gln Val Gln Leu Val Glu Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Asp Tyr Tyr Met Ser Trp Ile Arg Gln Ala Pro Gly Lys Gly Leu
       50                  55                  60
   Glu Trp Val Ser Tyr Ile Ser Ser Ser Gly Ser Thr Arg Asp Tyr Ala
   65                  70                  75                  80
   Asp Ser Val Lys Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
   100 105 110
   Tyr Tyr Cys Ala Arg Asp Gly Val Glu Thr Thr Phe Tyr Tyr Tyr Tyr
   115 120 125
   Tyr Gly Met Asp Val Trp Gly Gln Gly Thr Thr Val Thr Val Ser Ser
   130 135 140
   Ala Ser Thr Lys Gly Pro Ser Val Phe Pro Leu Ala Pro Cys Ser Arg
   145 150 155 160
   Ser Thr Ser Glu Ser Thr Ala Ala Leu Gly Cys Leu Val Lys Asp Tyr
   165 170 175
   Phe Pro Glu Pro Val Thr Val Ser Trp Asn Ser Gly Ala Leu Thr Ser
   180 185 190
   Gly Val His Thr Phe Pro Ala Val Leu Gln Ser Ser Gly Leu Tyr Ser
   195 200 205
   Leu Ser Ser Val Val Thr Val Pro Ser Ser Asn Phe Gly Thr Gln Thr
   210 215 220
   Tyr Thr Cys Asn Val Asp His Lys Pro Ser Asn Thr Lys Val Asp Lys
   225 230 235 240
   Thr Val Glu Arg Lys Cys Cys Val Glu Cys Pro Pro Cys Pro Ala Pro
   245 250 255
   Pro Val Ala Gly Pro Ser Val Phe Leu Phe Pro Pro Lys Pro Lys Asp
   260 265 270
   Thr Leu Met Ile Ser Arg Thr Pro Glu Val Thr Cys Val Val Val Asp
   275 280 285
   Val Ser His Glu Asp Pro Glu Val Gln Phe Asn Trp Tyr Val Asp Gly
   290 295 300
   Val Glu Val His Asn Ala Lys Thr Lys Pro Arg Glu Glu Gln Phe Asn
   305 310 315 320
   Ser Thr Phe Arg Val Val Ser Val Leu Thr Val Val His Gln Asp Trp
   325 330 335
   Leu Asn Gly Lys Glu Tyr Lys Cys Lys Val Ser Asn Lys Gly Leu Pro
   340 345 350
   Ala Pro Ile Glu Lys Thr Ile Ser Lys Thr Lys Gly Gln Pro Arg Glu
   355 360 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 34
   <211> 125
   <212> PRT
   <213> Artificial Sequence
<220>
   <221> Source
   <223> /note= "Description of Artificial Sequence: mature immunoglobulin heavy chain variable region of 2.12.1 fx"
<400> 34
   Gln Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asp Tyr
   20 25 30
   Tyr Met Ser Trp Ile Arg Gln Ala Pro Gly Lys Gly Leu Glu Trp Val
   35 40 45
   Ser Tyr Ile Ser Ser Ser Gly Ser Thr Arg Asp Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Gly Val Glu Thr Thr Phe Tyr Tyr Tyr Tyr Tyr Gly Met
   100 105 110
   Asp Val Trp Gly Gln Gly Thr Thr Val Thr Val Ser Ser
   115 120 125
<210> 35
   <211> 236
   <212> PRT
   <213> Artificial Sequence
<220>
   <221> Source
   <223> /note= "Description of Artificial Sequence: immunoglobulin light chain of 2.12.1 fx"
<400> 35
   Met Asp Met Arg Val Pro Ala Gln Leu Leu Gly Leu Leu Leu Leu Trp
   1 5 10 15
   Phe Pro Gly Ala Arg Cys Asp Ile Gln Met Thr Gln Ser Pro Ser Ser
   20 25 30
   Leu Ser Ala Ser Val Gly Asp Arg Val Thr Ile Thr Cys Arg Ala Ser
   35 40 45
   Gln Asp Ile Arg Arg Asp Leu Gly Trp Tyr Gln Gln Lys Pro Gly Lys
   50 55 60
   Ala Pro Lys Arg Leu Ile Tyr Ala Ala Ser Arg Leu Gln Ser Gly Val
   65 70 75 80
   Pro Ser Arg Phe Ser Gly Ser Gly Ser Gly Thr Glu Phe Thr Leu Thr
   85 90 95
   Ile Ser Ser Leu Gln Pro Glu Asp Phe Ala Thr Tyr Tyr Cys Leu Gln
   100 105 110
   His Asn Asn Tyr Pro Arg Thr Phe Gly Gln Gly Thr Lys Val Glu Ile
   115 120 125
   Lys Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 36
   <211> 108
   <212> PRT
   <213> Artificial Sequence
<220>
   <221> Source
   <223> /note= "Description of Artificial Sequence: mature immunoglobulin light chain variable region of 2.12.1 fx"
<400> 36
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Arg Asp
               20                  25                  30
   Leu Gly Trp Tyr Gln Gln Lys Pro Gly Lys Ala Pro Lys Arg Leu Ile
           35                  40                  45
   Tyr Ala Ala Ser Arg Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Asn Tyr Pro Arg
                   85                  90                  95
   Thr Phe Gly Gln Gly Thr Lys Val Glu Ile Lys Arg
               100                 105
<210> 37
   <211> 112
   <212> PRT
   <213> Artificial Sequence
<220>
   <221> Source
   <223> /note= "Description of Artificial Sequence: humanized 7C10 immunoglobulin light chain variable region; version 1"
<400> 37
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Gln Trp Tyr Leu Gln Lys Pro Gly Gln Ser
           35                  40                  45
   Pro Gln Leu Leu Ile Tyr Lys Val Ser Asn Arg Leu Tyr Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Trp Thr Phe Gly Gln Gly Thr Lys Val Glu Ile Lys
               100                 105                 110
<210> 38
   <211> 112
   <212> PRT
   <213> Artificial Sequence
<220>
   <221> Source
   <223> /note= "Description of Artificial Sequence: humanized 7C10 immunoglobulin light chain variable region; version 2"
<400> 38
<210> 39
   <211> 117
   <212> PRT
   <213> Artificial Sequence
<220>
   <221> Source
   <223> /note= "Description of Artificial Sequence: humanized 7C10 immunoglobulin heavy chain variable region; version 1"
<400> 39
   Gln Val Gln Leu Gln Glu Ser Gly Pro Gly Leu Val Lys Pro Ser Glu
   1 5 10 15
   Thr Leu Ser Leu Thr Cys Thr Val Ser Gly Tyr Ser Ile Thr Gly Gly
   20 25 30
   Tyr Leu Trp Asn Trp Ile Arg Gln Pro Pro Gly Lys Gly Leu Glu Trp
   35 40 45
   Met Gly Tyr Ile Ser Tyr Asp Gly Thr Asn Asn Tyr Lys Pro Ser Leu
   50 55 60
   Lys Asp Arg Ile Thr Ile Ser Arg Asp Thr Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Tyr Gly Arg Val Phe Phe Asp Tyr Trp Gly Gln Gly Thr Leu
   100 105 110
   Val Thr Val Ser Ser
   115
<210> 40
   <211> 117
   <212> PRT
   <213> Artificial Sequence
<220>
   <221> Source
   <223> /note= "Description of Artificial Sequence: humanized 7C10 immunoglobulin heavy chain variable region; version 2"
<400> 40
<210> 41
   <211> 117
   <212> PRT
   <213> Artificial Sequence
<220>
   <221> Source
   <223> /note= "Description of Artificial Sequence: humanized 7C10 immunoglobulin heavy chain variable region; version 3"
<400> 41
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Tyr Ser Ile Ser Gly Gly
               20                  25                  30
   Tyr Leu Trp Asn Trp Ile Arg Gln Pro Pro Gly Lys Gly Leu Glu Trp
           35                  40                  45
   Ile Gly Tyr Ile Ser Tyr Asp Gly Thr Asn Asn Tyr Lys Pro Ser Leu
       50                  55                  60
   Lys Asp Arg Val Thr Ile Ser Val Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Tyr Gly Arg Val Phe Phe Asp Tyr Trp Gly Gln Gly Thr Leu
               100                 105                 110
   Val Thr Val Ser Ser
           115
<210> 42
   <211> 130
   <212> PRT
   <213> Artificial Sequence
<220>
   <221> Source
   <223> /note= "Description of Artificial Sequence: A12 immunoglobulin heavy chain variable region"
<400> 42
   Glu Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Ser Tyr
   20 25 30
   Ala Ile Ser Trp Val Arg Gln Ala Pro Gly Gln Gly Leu Glu Trp Met
   35 40 45
   Gly Gly Ile Ile Pro Ile Phe Gly Thr Ala Asn Tyr Ala Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Ala Pro Leu Arg Phe Leu Glu Trp Ser Thr Gln Asp His Tyr
   100 105 110
   Tyr Tyr Tyr Tyr Met Asp Val Trp Gly Lys Gly Thr Thr Val Thr Val
   115 120 125
   Ser Ser
   130
<210> 43
   <211> 109
   <212> PRT
   <213> Artificial Sequence
<220>
   <221> Source
   <223> /note= "Description of Artificial Sequence: A12 immunoglobulin light chain variable region"
<400> 43
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Asn Ser Asp Asn Arg
                   85                  90                  95
   Leu Ile Phe Gly Gly Gly Thr Lys Leu Thr Val Leu Ser
               100                 105
<210> 44
   <212> 119
   <212> PRT
   <213> Artificial Sequence
<220>
   <221> Source
   <223> /note= "Description of Artificial Sequence: 1A immunoglobulin heavy chain variable region"
<220>
   <221> MISC_FEATURE
   <222> (1)..(119)
   <223> Possible mutations: R30, S30, N31, S31, Y94, H94, D104, E104.
<400> 44
<210> 45
   <211> 107
   <212> PRT
   <213> Artificial Sequence
<220>
   <221> Source
   <223> /note= "Description of Artificial Sequence: 1A immunoglobulin light chain variable region"
<220>
   <221> MISC_FEATURE
   <222> (1)..(107)
   <223> possible mutations: P96, 196, P100, Q100, R103, K103, V104, L104, D105, E105
<400> 45
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Pro
                   85                  90                  95
   Thr Phe Gly Pro Gly Thr Lys Val Asp Ile Lys
               100                 105
<210> 46
   <211> 251
   <212> PRT
   <213> Artificial Sequence
<220>
   <221> Source
   <223> /note= "Description of Artificial Sequence: single chain fv 8A1"
<400> 46
   Glu Val Gln Leu Val Gln Ser Gly Ala Glu Val Lys Lys Pro Gly Glu
   1               5                   10                  15
   Ser Leu Thr Ile Ser Cys Lys Gly Pro Gly Tyr Asn Phe Phe Asn Tyr
               20                  25                  30
   Trp Ile Gly Trp Val Arg Gln Met Pro Gly Lys Gly Leu Glu Trp Met
           35                  40                  45
   Gly Ile Ile Tyr Pro Thr Asp Ser Asp Thr Arg Tyr Ser Pro Ser Phe
       50                  55                  60
   Gln Gly Gln Val Thr Ile Ser Val Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Ser Ile Arg Tyr Cys Pro Gly Gly Arg Cys Tyr Ser Gly Tyr
                   100                 105                 110
   Tyr Gly Met Asp Val Trp Gly Gln Gly Thr Met Val Thr Val Ser Ser
               115                 120                 125
   Gly Gly Gly Gly Ser Gly Gly Gly Gly Ser Gly Gly Gly Gly Ser Ser
       130                 135                 140
   Glu Leu Thr Gln Asp Pro Ala Val Ser Val Ala Leu Gly Gln Thr Val
   145                 150                 155                 160
   Arg Ile Thr Cys Gln Gly Asp Ser Leu Arg Ser Tyr Tyr Ala Ser Trp
                   165                 170                 175
   Tyr Gln Gln Lys Pro Gly Gln Ala Pro Val Leu Val Ile Tyr Gly Lys
               180                 185                 190
   Asn Asn Arg Pro Ser Gly Ile Pro Asp Arg Phe Ser Gly Ser Ser Ser
           195                 200                 205
   Gly Asn Thr Ala Ser Leu Thr Ile Thr Gly Ala Gln Ala Glu Asp Glu
       210                 215                 220
   Ala Asp Tyr Tyr Cys Asn Ser Arg Asp Ser Ser Gly Asn His Val Val
   225                 230                 235                 240
   Phe Gly Gly Gly Thr Lys Leu Thr Val Leu Gly
                   245                 250
<210> 47
   <211> 245
   <212> PRT
   <213> Artificial Sequence
<220>
   <221> Source
   <223> /note= "Description of Artificial Sequence: single chain fv 9A2"
<400> 47
   Gln Val Gln Leu Val Gln Ser Gly Ala Glu Val Arg Lys Pro Gly Ala
   1               5                   10                  15
   Ser Val Lys Val Ser Cys Lys Thr Ser Gly Tyr Thr Phe Arg Asn Tyr
               20                  25                  30
   Asp Ile Asn Trp Val Arg Gln Ala Pro Gly Gln Gly Leu Glu Trp Met
           35                  40                  45
   Gly Arg Ile Ser Gly His Tyr Gly Asn Thr Asp His Ala Gln Lys Phe
       50                  55                  60
   Gln Gly Arg Phe Thr Met Thr Lys Asp Thr Ser Thr Ser Thr Ala Tyr
   65                  70                  75                  80
   Met Glu Leu Arg Ser Leu Thr Phe Asp Asp Thr Ala Val Tyr Tyr Cys
                   85                  90                  95
   Ala Arg Ser Gln Trp Asn Val Asp Tyr Trp Gly Arg Gly Thr Leu Val
               100                 105                 110
   Thr Val Ser Ser Gly Gly Gly Gly Ser Gly Gly Gly Gly Ser Gly Gly
           115                 120                 125
   Gly Gly Ser Ala Leu Asn Phe Met Leu Thr Gln Pro His Ser Val Ser
       130                 135                 140
   Glu Ser Pro Gly Lys Thr Val Thr Ile Ser Cys Thr Arg Ser Ser Gly
   145                 150                 155                 160
   Ser Ile Ala Ser Asn Tyr Val Gln Trp Tyr Gln Gln Arg Pro Gly Ser
                   165                 170                 175
   Ser Pro Thr Thr Val Ile Phe Glu Asp Asn Arg Arg Pro Ser Gly Val
               180                 185                 190
   Pro Asp Arg Phe Ser Gly Ser Ile Asp Thr Ser Ser Asn Ser Ala Ser
           195                 200                 205
   Leu Thr Ile Ser Gly Leu Lys Thr Glu Asp Glu Ala Asp Tyr Tyr Cys
       210                 215                 220
   Gln Ser Phe Asp Ser Thr Asn Leu Val Val Phe Gly Gly Gly Thr Lys
   225                 230                 235                 240
   Val Thr Val Leu Gly
                   245
<210> 48
   <211> 245
   <212> PRT
   <213> Artificial Sequence
<220>
   <221> Source
   <223> /note= "Description of Artificial Sequence: single chain fv 11A4"
<400> 48
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Ser Pro Tyr Ser Ser Arg Trp Tyr Ser Phe Asp Pro Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Ala Leu Ser Tyr Glu Leu Thr Gln
       130                 135                 140
   Pro Pro Ser Val Ser Val Ser Pro Gly Gln Thr Ala Thr Ile Thr Cys
   145                 150                 155                 160
   Ser Gly Asp Asp Leu Gly Asn Lys Tyr Val Ser Trp Tyr Gln Gln Lys
                   165                 170                 175
   Pro Gly Gln Ser Pro Val Leu Val Ile Tyr Gln Asp Thr Lys Arg Pro
               180                 185                 190
   Ser Gly Ile Pro Glu Arg Phe Ser Gly Ser Asn Ser Gly Asn Ile Ala
           195                 200                 205
   Thr Leu Thr Ile Ser Gly Thr Gln Ala Val Asp Glu Ala Asp Tyr Tyr
       210                 215                 220
   Cys Gln Val Trp Asp Thr Gly Thr Val Val Phe Gly Gly Gly Thr Lys
   225                 230                 235                 240
   Leu Thr Val Leu Gly
                   245
<210> 49
   <211> 251
   <212> PRT
   <213> Artificial Sequence
<220>
   <221> Source
   <223> /note= "Description of Artificial Sequence: single chain fv 7A4"
<400> 49
   Glu Val Gln Leu Val Gln Ser Gly Ala Glu Val Lys Lys Pro Gly Glu
   1               5                   10                  15
   Ser Leu Thr Ile Ser Cys Lys Gly Ser Gly Tyr Asn Phe Phe Asn Tyr
               20                  25                  30
   Trp Ile Gly Trp Val Arg Gln Met Pro Gly Lys Asp Leu Glu Trp Met
           35                  40                  45
   Gly Ile Ile Tyr Pro Thr Asp Ser Asp Thr Arg Tyr Ser Pro Ser Phe
       50                  55                  60
   Gln Gly Gln Val Thr Ile Ser Val Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Ser Ile Arg Tyr Cys Pro Gly Gly Arg Cys Tyr Ser Gly Tyr
               100                 105                 110
   Tyr Gly Met Asp Val Trp Gly Gln Gly Thr Met Val Thr Val Ser Ser
           115                 120                 125
   Gly Gly Gly Ser Ser Gly Gly Gly Gly Ser Gly Gly Gly Gly Ser Ser
       130                 135                 140
   Glu Leu Thr Gln Asp Pro Ala Val Ser Val Ala Leu Gly Gln Thr Val
   145                 150                 155                 160
   Arg Ile Thr Cys Arg Gly Asp Ser Leu Arg Asn Tyr Tyr Ala Ser Trp
                   165                 170                 175
   Tyr Gln Gln Lys Pro Gly Gln Ala Pro Val Leu Val Ile Tyr Gly Lys
               180                 185                 190
   Asn Asn Arg Pro Ser Gly Ile Pro Asp Arg Phe Ser Gly Ser Ser Ser
   195 200 205
   Gly Asn Thr Ala Ser Leu Thr Ile Thr Gly Ala Gln Ala Glu Asp Glu
   210 215 220
   Ala Asp Tyr Tyr Cys Asn Ser Arg Asp Ser Ser Gly Asn His Met Val
   225 230 235 240
   Phe Gly Gly Gly Thr Lys Leu Thr Val Leu Gly
   245 250
<210> 50
   <211> 249
   <212> PRT
   <213> Artificial Sequence
<220>
   <221> Source
   <223> /note= "Description of Artificial Sequence: single chain fv 11A1"
<400> 50
   Glu Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Asp Phe
               20                  25                  30
   Ala Met His Trp Val Arg Gln Ile Pro Gly Lys Gly Leu Glu Trp Leu
           35                  40                  45
   Ser Gly Leu Arg His Asp Gly Ser Thr Ala Tyr Tyr Ala Gly Ser Val
       50                  55                  60
   Lys Gly Arg Phe Thr Ile Ser Arg Asp Asn Ser Arg Asn Thr Val Tyr
   65                  70                  75                  80
   Leu Gln Met Asn Ser Leu Arg Ala Glu Asp Thr Ala Thr Tyr Tyr Cys
                   85                  90                  95
   Val Thr Gly Ser Gly Ser Ser Gly Pro His Ala Phe Pro Val Trp Gly
               100                 105                 110
   Lys Gly Thr Leu Val Thr Val Ser Ser Gly Gly Gly Gly Ser Gly Gly
           115                 120                 125
   Gly Gly Ser Gly Gly Gly Gly Ser Ala Leu Ser Tyr Val Leu Thr Gln
       130                 135                 140
   Pro Pro Ser Ala Ser Gly Thr Pro Gly Gln Arg Val Thr Ile Ser Cys
   145                 150                 155                 160
   Ser Gly Ser Asn Ser Asn Ile Gly Thr Tyr Thr Val Asn Trp Phe Gln
                   165                 170                 175
   Gln Leu Pro Gly Thr Ala Pro Lys Leu Leu Ile Tyr Ser Asn Asn Gln
               180                 185                 190
   Arg Pro Ser Gly Val Pro Asp Arg Phe Ser Gly Ser Lys Ser Gly Thr
           195                 200                 205
   Ser Ala Ser Leu Ala Ile Ser Gly Leu Gln Ser Glu Asp Glu Ala Asp
       210                 215                 220
   Tyr Tyr Cys Ala Ala Trp Asp Asp Ser Leu Asn Gly Pro Val Phe Gly
   225                 230                 235                 240
   Gly Gly Thr Lys Val Thr Val Leu Gly
                   245
<210> 51
   <211> 251
   <212> PRT
   <213> Artificial Sequence
<220>
   <221> Source
   <223> /note= "Description of Artificial Sequence: single chain fv 7A6"
<400> 51
   Glu Val Gln Leu Val Gln Ser Gly Ala Glu Val Lys Lys Pro Gly Glu
   1               5                   10                  15
   Ser Leu Thr Ile Ser Cys Lys Gly Ser Gly Tyr Asn Phe Phe Asn Tyr
               20                  25                  30
   Trp Ile Gly Trp Val Arg Gln Met Pro Gly Lys Gly Leu Glu Trp Met
           35                  40                  45
   Gly Ile Ile Tyr Pro Thr Asp Ser Asp Thr Arg Tyr Ser Pro Ser Phe
       50                  55                  60
   Gln Gly Gln Val Thr Ile Ser Val Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Ser Ile Arg Tyr Cys Pro Gly Gly Arg Cys Tyr Ser Gly Tyr
   100 105 110
   Tyr Gly Met Asp Val Trp Gly Gln Gly Thr Leu Val Thr Val Ser Ser
   115 120 125
   Gly Gly Gly Gly Ser Gly Gly Gly Gly Ser Gly Gly Gly Gly Ser Ser
   130 135 140
   Glu Leu Thr Gln Asp Pro Ala Val Ser Val Ala Leu Gly Gln Thr Val
   145 150 155 160
   Arg Ile Thr Cys Gln Gly Asp Ser Leu Arg Ser Tyr Tyr Thr Asn Trp
   165 170 175
   Phe Gln Gln Lys Pro Gly Gln Ala Pro Leu Leu Val Val Tyr Ala Lys
   180 185 190
   Asn Lys Arg Pro Ser Gly Ile Pro Asp Arg Phe Ser Gly Ser Ser Ser
   195 200 205
   Gly Asn Thr Ala Ser Leu Thr Ile Thr Gly Ala Gln Ala Glu Asp Glu
   210 215 220
   Ala Asp Tyr Tyr Cys Asn Ser Arg Asp Ser Ser Gly Asn His Val Val
   225 230 235 240
   Phe Gly Gly Gly Thr Lys Leu Thr Val Leu Gly
   245 250
<210> 52
   <211> 5
   <212> PRT
   <213> Artificial Sequence
<220>
   <221> Source
   <223> /note= "Description of Artificial Sequence: CDR"
<400> 52
   Ser Tyr Trp Met His
   1 5
<210> 53
   <211> 17
   <212> PRT
   <213> Artificial Sequence
<220>
   <221> Source
   <223> /note= "Description of Artificial Sequence: CDR"
<400> 53
   Glu Ile Asn Pro Ser Asn Gly Arg Thr Asn Tyr Asn Glu Lys Phe Lys
   1 5 10 15
   Arg
<210> 54
   <211> 15
   <212> PRT
   <213> Artificial Sequence
<220>
   <221> Source
   <223> /note= "Description of Artificial Sequence: CDR"
<400> 54
   Gly Arg Pro Asp Tyr Tyr Gly Ser Ser Lys Trp Tyr Phe Asp Val
   1 5 10 15
<210> 55
   <211> 16
   <212> PRT
   <213> Artificial Sequence
<220>
   <221> Source
   <223> /note= "Description of Artificial Sequence: CDR"
<400> 55
   Arg Ser Ser Gln Ser Ile Val His Ser Asn Val Asn Thr Tyr Leu Glu
   1 5 10 15
<210> 56
   <211> 7
   <212> PRT
   <213> Artificial Sequence
<220>
   <221> Source
   <223> /note= "Description of Artificial Sequence: CDR"
<400> 56
   Lys Val Ser Asn Arg Phe Ser
   1 5
<210> 57
   <211> 9
   <212> PRT
   <213> Artificial Sequence
<220>
   <221> Source
   <223> /note= "Description of Artificial Sequence: CDR"
<400> 57
   Phe Gln Gly Ser His Val Pro Pro Thr
   1               5
<210> 58
   <211> 123
   <212> PRT
   <213> Artificial Sequence
<220>
   <221> Source
   <223> /note= "Description of Artificial Sequence: heavy chain immunoglobulin variable region"
<400> 58
   Gln Val Gln Leu Val Gln Ser Gly Ala Glu Val Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Gln Lys Phe
       50                  55                  60
   Gln Gly Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Gln Gly Thr Thr Val Thr Val Ser
           115                 120
<210> 59
   <211> 118
   <212> PRT
   <213> Artificial Sequence
<220>
   <221> Source
   <223> /note= "Description of Artificial Sequence: easy chain immunoglobulin variable region"
<400> 59
   Gln Val Gln Phe Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Leu Met His Trp Ile Lys Gln Arg Pro Gly Arg Gly Leu Glu Trp Ile
           35                  40                  45
   Gly Arg Ile Asp Pro Asn Asn Val Val Thr Lys Phe Asn Glu Lys Phe
       50                  55                  60
   Lys Ser Lys Ala Thr Leu Thr Val Asp Lys Pro Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Tyr Ala Tyr Cys Arg Pro Met Asp Tyr Trp Gly Gln Gly Thr
               100                 105                 110
   Thr Val Thr Val Ser Ser
           115
<210> 60
   <211> 123
   <212> PRT
   <213> Artificial Sequence
<220>
   <221> Source
   <223> /note= "Description of Artificial Sequence: heavy chain immunoglobulin variable region"
<400> 60
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Glu Lys Phe
       50                  55                  60
   Lys Arg Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Ala Gly Thr Thr Val Thr Val Ser
           115                 120
<210> 61
   <211> 120
   <212> PRT
   <213> Artificial Sequence
<220>
   <221> Source
   <223> /note= "Description of Artificial Sequence: heavy chain immunoglobulin variable region"
<400> 61
   Gln Val Gln Leu Gln Gln Ser Gly Ala Glu Leu Met Lys Pro Gly Ala
   1               5                   10                  15
   Ser Val Lys Ile Ser Cys Lys Ala Thr Gly Tyr Thr Phe Ser Ser Phe
               20                  25                  30
   Trp Ile Glu Trp Val Lys Gln Arg Pro Gly His Gly Leu Glu Trp Ile
           35                  40                  45
   Gly Glu Ile Leu Pro Gly Ser Gly Gly Thr His Tyr Asn Glu Lys Phe
       50                  55                  60
   Lys Gly Lys Ala Thr Phe Thr Ala Asp Lys Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Gly His Ser Tyr Tyr Phe Tyr Asp Gly Asp Tyr Trp Gly Gln
               100                 105                 110
   Gly Thr Ser Val Thr Val Ser Ser
           115                 120
<210> 62
   <211> 120
   <212> PRT
   <213> Artificial Sequence
<220>
   <221> Source
   <223> /note= "Description of Artificial Sequence: heavy chain immunoglobulin variable region"
<400> 62
   Gln Val Gln Leu Gln Gln Pro Gly Ser Val Leu Val Arg Pro Gly Ala
   1 5 10 15
   Ser Val Lys Leu Ser Cys Lys Ala Ser Gly Tyr Thr Phe Thr Ser Ser
   20 25 30
   Trp Ile His Trp Ala Lys Gln Arg Pro Gly Gln Gly Leu Glu Trp Ile
   35 40 45
   Gly Glu Ile His Pro Asn Ser Gly Asn Thr Asn Tyr Asn Glu Lys Phe
   50 55 60
   Lys Gly Lys Ala Thr Leu Thr Val Asp Thr Ser Ser Ser Thr Ala Tyr
   65 70 75 80
   Val Asp Leu Ser Ser Leu Thr Ser Glu Asp Ser Ala Val Tyr Tyr Cys
   85 90 95
   Ala Arg Trp Arg Tyr Gly Ser Pro Tyr Tyr Phe Asp Tyr Trp Gly Gln
   100 105 110
   Gly Thr Thr Leu Thr Val Ser Ser
   115 120
<210> 63
   <211> 120
   <212> PRT
   <213> Artificial Sequence
<220>
   <221> Source
   <223> /note= "Description of Artificial Sequence: heavy chain immunoglobulin variable region"
<400> 63
   Gln Val Gln Leu Gln Gln Pro Gly Ala Glu Leu Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Arg Gly Leu Glu Trp Ile
   35 40 45
   Gly Arg Ile Asp Pro Asn Ser Gly Gly Thr Lys Tyr Asn Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Asp Tyr Tyr Gly Ser Ser Tyr Phe Asp Tyr Trp Gly Gln
               100                 105                 110
   Gly Thr Thr Leu Thr Val Ser Ser
           115                 120
<210> 64
   <211> 123
   <212> PRT
   <213> Artificial Sequence
<220>
   <221> Source
   <223> /note= "Description of Artificial Sequence: heavy chain immunoglobulin variable region"
<400> 64
   Gln Val Gln Leu Val Gln Ser Gly Ala Glu Val Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Gln Lys Phe
       50                  55                  60
   Gln Gly Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Gln Gly Thr Thr Val Thr Val Ser
   115                 120
<210> 65
   <211> 124
   <212> PRT
   <213> Artificial Sequence
<220>
   <221> Source
   <223> /note= "Description of Artificial Sequence: heavy chain immunoglobulin variable region"
<400> 65
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Glu Lys Phe
       50                  55                  60
   Lys Arg Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Ala Gly Thr Thr Val Thr Val Ser Ser
           115                 120
<210> 66
   <211> 124
   <212> PRT
   <213> Artificial Sequence
<220>
   <221> Source
   <223> /note= "Description of Artificial Sequence: heavy chain immunoglobulin variable region"
<400> 66
   Gln Val Gln Leu Val Gln Ser Gly Ala Glu Val Val Lys Pro Gly Ala
   1               5                   10                  15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Gln Lys Phe
   50 55 60
   Gln Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Gln Gly Thr Thr Val Thr Val Ser Ser
   115 120
<210> 67
   <211> 120
   <212> PRT
   <213> Artificial Sequence
<220>
   <221> Source
   <223> /note= "Description of Artificial Sequence: heavy chain immunoglobulin variable region"
<400> 67
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Arg Gly Leu Glu Trp Ile
           35                  40                  45
   Gly Arg Ile Asp Pro Asn Ser Gly Gly Thr Lys Tyr Asn Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Asp Tyr Tyr Gly Ser Ser Tyr Phe Asp Tyr Trp Gly Gln
               100                 105                 110
   Gly Thr Thr Val Thr Val Ser Ser
           115                 120
<210> 68
   <211> 117
   <212> PRT
   <213> Artificial Sequence
<220>
   <221> Source
   <223> /note= "Description of Artificial Sequence: heavy chain immunoglobulin variable region"
<400> 68
   Gln Ile Gln Leu Gln Gln Ser Gly Pro Glu Leu Val Arg Pro Gly Ala
   1               5                   10                  15
   Ser Val Lys Ile Ser Cys Lys Ala Ser Gly Tyr Thr Phe Thr Asp Tyr
               20                  25                  30
   Tyr Ile His Trp Val Lys Gln Arg Pro Gly Glu Gly Leu Glu Trp Ile
           35                  40                  45
   Gly Trp Ile Tyr Pro Gly Ser Gly Asn Thr Lys Tyr Asn Glu Lys Phe
       50                  55                  60
   Lys Gly Lys Ala Thr Leu Thr Val Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Gly Gly Lys Phe Ala Met Asp Tyr Trp Gly Gln Gly Thr Ser
               100                 105                 110
   Val Thr Val Ser Ser
           115
<210> 69
   <211> 124
   <212> PRT
   <213> Artificial Sequence
<220>
   <221> Source
   <223> /note= "Description of Artificial Sequence: heavy chain immunoglobulin variable region"
<400> 69
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Glu Lys Phe
       50                  55                  60
   Lys Arg Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Ala Gly Thr Thr Val Thr Val Ser Ser
           115                 120
<210> 70
   <211> 120
   <212> PRT
   <213> Artificial Sequence
<220>
   <221> Source
   <223> /note= "Description of Artificial Sequence: heavy chain immunoglobulin variable region"
<400> 70
   Gln Ile Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Ile Asn Trp Met Lys Gln Lys Pro Gly Gln Gly Leu Glu Trp Ile
           35                  40                  45
   Gly Trp Ile Asp Pro Gly Ser Gly Asn Thr Lys Tyr Asn Glu Lys Phe
       50                  55                  60
   Lys Gly Lys Ala Thr Leu Thr Val Asp Thr Ser Ser Ser Thr Ala Tyr
   65 70 75 80
   Met Gln Leu Ser Ser Leu Thr Ser Glu Asp Thr Ala Val Tyr Phe Cys
   85 90 95
   Ala Arg Glu Lys Thr Thr Tyr Tyr Tyr Ala Met Asp Tyr Trp Gly Gln
   100 105 110
   Gly Thr Ser Val Thr Val Ser Ala
   115 120
<210> 71
   <211> 115
   <212> PRT
   <213> Artificial Sequence
<220>
   <221> Source
   <223> /note= "Description of Artificial Sequence: heavy chain immunoglobulin variable region"
<400> 71
   Val Gln Leu Gln Gln Ser Gly Ala Glu Leu Met Lys Pro Gly Ala Ser
   1 5 10 15
   Val Lys Ile Ser Cys Lys Ala Ser Gly Tyr Thr Phe Ser Asp Tyr Trp
   20 25 30
   Ile Glu Trp Val Lys Gln Arg Pro Gly His Gly Leu Glu Trp Ile Gly
   35 40 45
   Glu Ile Leu Pro Gly Ser Gly Ser Thr Asn Tyr His Glu Arg Phe Lys
   50 55 60
   Gly Lys Ala Thr Phe Thr Ala Asp Thr Ser Ser Ser Thr Ala Tyr Met
   65 70 75 80
   Gln Leu Asn Ser Leu Thr Ser Glu Asp Ser Gly Val Tyr Tyr Cys Leu
   85 90 95
   His Gly Asn Tyr Asp Phe Asp Gly Trp Gly Gln Gly Thr Thr Leu Thr
   100 105 110
   Val Ser Ser
   115
<210> 72
   <211> 120
   <212> PRT
   <213> Artificial Sequence
<220>
   <221> Source
   <223> /note= "Description of Artificial Sequence: heavy chain immunoglobulin variable region"
<400> 72
   Gln Val Gln Leu Leu Glu Ser Gly Ala Glu Leu Met Lys Pro Gly Ala
   1               5                   10                  15
   Ser Val Lys Ile Ser Cys Lys Ala Thr Gly Tyr Thr Phe Ser Ser Phe
               20                  25                  30
   Trp Ile Glu Trp Val Lys Gln Arg Pro Gly His Gly Leu Glu Trp Ile
           35                  40                  45
   Gly Glu Ile Leu Pro Gly Ser Gly Gly Thr His Tyr Asn Glu Lys Phe
       50                  55                  60
   Lys Gly Lys Ala Thr Phe Thr Ala Asp Lys Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Gly His Ser Tyr Tyr Phe Tyr Asp Gly Asp Tyr Trp Gly Gln
               100                 105                 110
   Gly Thr Ser Val Thr Val Ser Ser
           115                 120
<210> 73
   <211> 113
   <212> PRT
   <213> Artificial Sequence
<220>
   <221> Source
   <223> /note= "Description of Artificial Sequence: light chain immunoglobulin variable region"
<400> 73
   Asp Val Leu Met Thr Gln Ile Pro Val Ser Leu Pro Val Ser Leu Gly
   1               5                   10                  15
   Asp Gln Ala Ser Ile Ser Cys Arg Ser Ser Gln Ile Ile Val His Asn
               20                  25                  30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Phe Thr Phe Gly Ser Gly Thr Lys Leu Glu Ile Lys
   100 105 110
   Arg
<210> 74
   <211> 113
   <212> PRT
   <213> Artificial Sequence
<220>
   <221> Source
   <223> /note= "Description of Artificial Sequence: light chain immunoglobulin variable region"
<400> 74
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
                   100                 105                 110
   Arg
<210> 75
   <211> 113
   <212> PRT
   <213> Artificial Sequence
<220>
   <221> Source
   <223> /note= "Description of Artificial Sequence: light chain immunoglobulin variable region"
<400> 75
   Asp Val Leu Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 76
   <211> 113
   <212> PRT
   <213> Artificial Sequence
<220>
   <221> Source
   <223> /note= "Description of Artificial Sequence: light chain immunoglobulin variable region"
<400> 76
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                      80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 77
   <211> 113
   <212> PRT
   <213> Artificial Sequence
<220>
   <221> Source
   <223> /note= "Description of Artificial Sequence: light chain immunoglobulin variable region"
<400> 77
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 78
   <211> 113
   <212> PRT
   <213> Artificial Sequence
<220>
   <221> Source
   <223> /note= "Description of Artificial Sequence: light chain immunoglobulin variable region"
<220>
   <221> misc_feature
   <222> (28)..(28)
   <223> Xaa can be any naturally occurring amino acid
<220>
   <221> misc_feature
   <222> (101)..(101)
   <223> Xaa can be any naturally occurring amino acid
<400> 78
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Xaa Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Xaa Thr Phe Gly Gly Gly Thr Lys Leu Glu Ile Lys
               100                 105                 110
   Arg
<210> 79
   <211> 113
   <212> PRT
<210> Artificial Sequence
<220>
   <221> Source
   <223> /note= "Description of Artificial Sequence: light chain immunoglobulin variable region"
<400> 79
<210> 80
   <211> 113
   <212> FRT
   <213> Artificial Sequence
<220>
   <221> Source
   <223> /note= "Description of Artificial Sequence: light chain immunoglobulin variable region"
<400> 80
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 81
   <211> 113
   <212> PRT
   <213> Artificial Sequence
<220>
   <221> Source
   <223> /note= "Description of Artificial Sequence: light chain immunoglobulin variable region"
<400> 81
   Asp Val Leu Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 82
   <211> 113
   <212> PRT
   <213> Artificial Sequence
<220>
   <221> Source
   <223> /note= "Description of Artificial Sequence: light chain immunoglobulin variable region"
<400> 82
   Asp Val Leu Met Thr Gln Ile Pro Val Ser Leu Pro Val Ser Leu Gly
   1 5 10 15
   Asp Gln Ala Ser Ile Ser Cys Arg Ser Ser Gln Ile Ile Val His Asn
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Phe Thr Phe Gly Ser Gly Thr Lys Leu Glu Ile Lys
   100 105 110
   Arg
<210> 83
   <211> 113
   <212> PRT
   <213> Artificial Sequence
<220>
   <221> Source
   <223> /note= "Description of Artificial Sequence: light chain immunoglobulin variable region"
<400> 83
   Asp Val Leu Met Thr Gln Thr Pro Leu Ser Leu Pro Val Ser Leu Gly
   1               5                   10                  15
   Asp Gln Ala Ser Ile Ser Cys Arg Phe Ser Gln Ser Ile Val His Ser
               20                  25                  30
   Asn Gly Asn Thr Tyr Leu Glu Trp Tyr Leu Gln Lys Ser Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Arg Thr Phe Gly Gly Gly Thr Lys Leu Glu Ile Lys
               100                 105                 110
   Arg
<210> 84
   <211> 113
   <212> PRT
   <213> Artificial Sequence
<220>
   <221> Source
   <223> /note= "Description of Artificial Sequence: light chain immunoglobulin variable region"
<400> 84
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 85
   <211> 113
   <212> PRT
   <213> Artificial Sequence
<220>
   <221> Source
   <223> /note= "Description of Artificial Sequence: light chain immunoglobulin variable regions"
<400> 85
   Asp Val Val Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 86
   <211> 113
   <212> PRT
   <213> Artificial Sequence
<220>
   <221> Source
   <223> /note= "Description of Artificial Sequence: light chain immunoglobulin variable region"
<400> 86
   Glu Leu Val Met Thr Gln Thr Pro Leu Ser Leu Pro Val Ser Leu Gly
   1               5                   10                  15
   Asp Gln Ala Ser Ile Ser Cys Arg Ser Ser Gln Thr Ile Val His Ser
               20                  25                  30
   Asn Gly Asp Thr Tyr Leu Asp Trp Phe Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 87
   <211> 113
   <212> PRT
   <213> Artificial Sequence
<220>
   <221> Source
   <223> /note= "Description of Artificial Sequence: light chain immunoglobulin variable region"
<400> 87
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 88
   <211> 113
   <212> PRT
   <213> Artificial Sequence
<220>
   <221> Source
   <223> /note= "Description or Artificial Sequence: light chain immunoglobulin variable region"
<400> 88
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 89
   <211> 113
   <212> PRT
   <213> Artificial Sequence
<220>
   <221> Source
   <223> /note= "Description of Artificial Sequence: light chain immunoglobulin variable region"
<400> 89
   Asp Val Leu Met Thr Gln Thr Pro Val Ser Leu Ser Val Ser Leu Gly
   1               5                   10                  15
   Asp Gln Ala Ser Ile Ser Cys Arg Ser Ser Gln Ser Ile Val His Ser
               20                  25                  30
   Thr Gly Asn Thr Tyr Leu Glu Trp Tyr Leu Gln Lys Pro Gly Gln Ser
           35                  40                  45
   Pro Lys Leu Leu Ile Tyr Lys Ile Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Ala
                   85                  90                  95
   Ser His Ala Pro Arg Thr Phe Gly Gly Gly Thr Lys Leu Glu Ile Lys
               100                 105                 110
   Arg
<210> 90
   <211> 113
   <212> PRT
   <213> Artificial Sequence
<220>
   <221> Source
   <223> /note= "Description of Artificial Sequence: light chain immunoglobulin variable region"
<400> 90
   Asp Val Leu Met Thr Gln Thr Pro Leu Ser Leu Pro Val Ser Leu Gly
   1 5 10 15
   Asp Gln Ala Ser Ile Ser Cys Lys Ser Ser Gln Ser Ile Val His Ser
   20 25 30
   Ser Gly Asn Thr Tyr Phe Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Ile Pro Phe Thr Phe Gly Ser Gly Thr Lys Leu Glu Ile Lys
   100 105 110
   Arg
<210> 91
   <211> 113
   <212> PRT
   <213> Artificial Sequence
<220>
   <221> Source
   <223> /note= "Description of Artificial Sequence: light chain immunoglobulin variable region"
<400> 91
   Asp Ile Glu Leu Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg
<210> 92
   <211> 113
   <212> PRT
   <213> Artificial Sequence
<220>
   <221> Source
   <223> /note= "Description of Artificial Sequence: light chain immunoglobulin variable regions"
<400> 92
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 93
   <211> 113
   <212> PRT
   <213> Artificial Sequence
<220>
   <221> Source
   <223> /note= "Description of Artificial Sequence: light chain immunoglobulin variable region"
<400> 93
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 94
   <211> 113
   <212> PRT
   <213> Artificial Sequence
<220>
   <221> Source
   <223> /note= "Description of Artificial Sequence: light chain immunoglobulin variable region"
<400> 94
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 95
   <211> 113
   <212> PRT
   <213> Artificial Sequence
<220>
   <221> Source
   <223> /note= "Description of Artificial Sequence: light chain immunoglobulin variable region"
<400> 95
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 96
   <211> 113
   <212> PRT
   <213> Artificial Sequence
<220>
   <221> Source
   <223> /note= "Description of Artificial Sequence: light chain immunoglobulin variable region"
<400> 96
   Asp Val Leu Met Thr Gln Thr Pro Leu Ser Leu Pro Val Ser Leu Gly
   1 5 10 15
   Asp Gln Ala Ser Ile Ser Cys Arg Ser Asn Gln Thr Ile Leu Leu Ser
   20 25 30
   Asp Gly Asp Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 97
   <211> 113
   <212> PRT
   <213> Artificial Sequence
<220>
   <221> Source
   <223> /note= "Description of Artificial Sequence: light chain immunoglobulin variable region"
<400> 97
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Thr Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Thr Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Thr His Ala Pro Tyr Thr Phe Gly Gly Gly Thr Lys Leu Glu Ile Lys
               100                 105                 110
   Arg
<210> 98
   <211> 113
   <212> PRT
   <213> Artificial Sequence
<220>
   <221> Source
   <223> /note= "Description of Artificial Sequence: light chain immunoglobulin variable region"
<400> 98
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Ser Ile Ser Ser Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Gln Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg
<210> 99
   <211> 11
   <212> PRT
   <213> Artificial Sequence:
<220>
   <221> Source
   <223> /note= "Description of Artificial Sequence: CDR"
<400> 99
   Arg Ala Ser Gln Ser Ile Gly Ser Ser Leu His
   1 5 10
<210> 100
   <211> 7
   <212> PRT
   <213> Artificial Sequence
<220>
   <221> Source
   <223> /note= "Description of Artificial Sequence: CDR"
<400> 100
   Tyr Ala Ser Gln Ser Leu Ser
   1 5
<210> 101
   <211> 9
   <212> PRT
   <213> Artificial Sequence
<220>
   <221> Source
   <223> /note= "Description of Artificial Sequence: CDR"
<400> 101
   His Gln Ser Ser Arg Leu Pro His Thr
   1 5
<210> 102
   <211> 5
   <212> PRT
<210> Artificial Sequence
<220>
   <221> Source
   <223> /note= "Description of Artificial Sequence:: CDR"
<400> 102
<210> 103
   <211> 16
   <212> PRT
   <213> Artificial Sequence
<220>
   <221> Source
   <223> /note= "Description of Artificial Sequence: CDR"
<400> 103
   Val Ile Asp Thr Arg Gly Ala Thr Tyr Tyr Ala Asp Ser Val Lys Gly
   1 5 10 15
<210> 104
   <211> 10
   <212> PRT
   <213> Artificial Sequence
<220>
   <221> Source
   <223> /note= "Description of Artificial Sequence: CDR"
<400> 104
   Leu Gly Asn Phe Tyr Tyr Gly Met Asp Val
   1               5                   10
<210> 105
   <211> 328
   <212> PRT
   <213> Homo Sapiens
<400> 105
   Met Leu Pro Arg Val Gly Cys Pro Ala Leu Pro Leu Pro Pro Pro Pro
   1               5                   10                  15
   Leu Leu Pro Leu Leu Pro Leu Leu Leu Leu Leu Leu Gly Ala Ser Gly
               20                  25                  30
   Gly Gly Gly Gly Ala Arg Ala Glu Val Leu Phe Arg Cys Pro Pro Cys
           35                  40                  45
   Thr Pro Glu Arg Leu Ala Ala Cys Gly Pro Pro Pro Val Ala Pro Pro
       50                  55                  60
   Ala Ala Val Ala Ala Val Ala Gly Gly Ala Arg Met Pro Cys Ala Glu
   65                  70                  75                  80
   Leu Val Arg Glu Pro Gly Cys Gly Cys Cys Ser Val Cys Ala Arg Leu
                   85                  90                  95
   Glu Gly Glu Ala Cys Gly Val Tyr Thr Pro Arg Cys Gly Gln Gly Leu
   100 105 110
   Arg Cys Tyr Pro His Pro Gly Ser Glu Leu Pro Leu Gln Ala Leu Val
   115 120 125
   Met Gly Glu Gly Thr Cys Glu Lys Arg Arg Asp Ala Glu Tyr Gly Ala
   130 135 140
   Ser Pro Glu Gln Val Ala Asp Asn Gly Asp Asp His Ser Glu Gly Gly
   145 150 155 160
   Leu Val Glu Asn His Val Asp Ser Thr Met Asn Met Leu Gly Gly Gly
   165 170 175
   Gly Ser Ala Gly Arg Lys Pro Leu Lys Ser Gly Met Lys Glu Leu Ala
   180 185 190
   Val Phe Arg Glu Lys Val Thr Glu Gln His Arg Gln Met Gly Lys Gly
   195 200 205
   Gly Lys His His Leu Gly Leu Glu Glu Pro Lys Lys Leu Arg Pro Pro
   210 215 220
   Pro Ala Arg Thr Pro Cys Gln Gln Glu Leu Asp Gln Val Leu Glu Arg
   225 230 235 240
   Ile Ser Thr Met Arg Leu Pro Asp Glu Arg Gly Pro Leu Glu His Leu
   245 250 255
   Tyr Ser Leu His Ile Pro Asn Cys Asp Lys His Gly Leu Tyr Asn Leu
   260 265 270
   Lys Gln Cys Lys Met Ser Leu Asn Gly Gln Arg Gly Glu Cys Trp Cys
   275 280 285
   Val Asn Pro Asn Thr Gly Lys Leu Ile Gln Gly Ala Pro Thr Ile Arg
   290 295 300
   Gly Asp Pro Glu Cys His Leu Phe Tyr Asn Glu Gln Gln Glu Ala Arg
   305 310 315 320
   Gly Val His Thr Gln Arg Met Gln
   325
<210> 106
   <211> 109
   <212> PRT
   <213> Artificial Sequence
<220>
   <221> Source
   <223> /note= "Description of Artificial Sequence: A12 immunoglobulin light chain variable region"
<400> 106
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Thr Trp Tyr Gln Gln Lys Pro Gly Gln Ala Pro Ile Leu Val Ile Tyr
           35                  40                  45
   Gly Glu Asn Lys Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Lys Ser Arg Asp Gly Ser Gly Gln His
                   85                  90                  95
   Leu Val Phe Gly Gly Gly Thr Lys Leu Thr Val Leu Gly
   100 105
<160> 107
<170> PatentIn version 3.4
<210> 1
   <211> 10
   <212> PRT
   <<213> Artificial Sequence
<220>
   <221> Source
   <223> /note= "Description of Artificial Sequence: heavy chain immunoglobulin variable region"
<400> 107